(19) Europäisches Patentamt
European Patent Office
Office européen des brevets

(11) Veröffentlichungsnummer: **0 429 968 B1**

# (12) EUROPÄISCHE PATENTSCHRIFT

(45) Veröffentlichungstag der Patentschrift: **27.07.94**

(51) Int. Cl.5: **C07D 417/04**, A01N 43/90

(21) Anmeldenummer: **90121845.3**

(22) Anmeldetag: **15.11.90**

Die Akte enthält technische Angaben, die nach dem Eingang der Anmeldung eingereicht wurden und die nicht in dieser Patentschrift enthalten sind.

## (54) Heterocyclisch sustituierte Acrylsäureester.

(30) Priorität: **28.11.89 DE 3939238**

(43) Veröffentlichungstag der Anmeldung: **05.06.91 Patentblatt 91/23**

(45) Bekanntmachung des Hinweises auf die Patenterteilung: **27.07.94 Patentblatt 94/30**

(84) Benannte Vertragsstaaten: **BE CH DE DK FR GB IT LI NL**

(56) Entgegenhaltungen:
**EP-A- 0 015 502**
**EP-A- 0 274 825**
**EP-A- 0 383 117**

(73) Patentinhaber: **BAYER AG**
**D-51368 Leverkusen(DE)**

(72) Erfinder: **Klausener, Alexander, Dr.**
**Dahlerdyk 173**
**W-4150 Krefeld 1(DE)**
Erfinder: **Berg, Dieter, Dr.**
**Gellertweg 27**
**W-5600 Wuppertal 1(DE)**
Erfinder: **Seitz, Thomas, Dr.**
**Mozartstrasse 32**
**W-4019 Monheim(DE)**
Erfinder: **Brandes, Wilhelm, Dr.**
**Eichendorffstrasse 3**
**W-5653 Leichlingen 1(DE)**
Erfinder: **Dutzmann, Stefan, Dr.**
**Leinenweberweg 33**
**W-4000 Düsseldorf 13(DE)**
Erfinder: **Hänssler, Gerd, Dr.**
**Am Arenzberg 58a**
**W-5090 Leverkusen 3(DE)**
Erfinder: **Wachendorff-Neumann, Ulrike, Dr.**
**Krischerstrasse 81**
**W-4019 Monheim(DE)**

EP 0 429 968 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue heterocyclisch substituierte Acrylsäureester, mehrere Verfahren zu ihrer Herstellung, ihre Verwendung zur Bekämpfung von Schädlingen und neue Zwischenprodukte.

Es ist bekannt, daß bestimmte substituierte Acrylsäureester, wie beispielsweise die Verbindung 3-Methoxy-2-(2-methylphenyl)acrylsäuremethylester fungizide Eigenschaften besitzen (vgl. z.B. EP 178826).

Weiterhin ist bekannt, daß bestimmte in 2-Stellung durch einen 1-Indolylrest substituierte Alkoxyacrylsäureester fungizid wirksam sind (vgl. EP 274 825).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue heterocyclisch substituierte Acrylsäureester der allgemeinen Formel (I) gefunden,

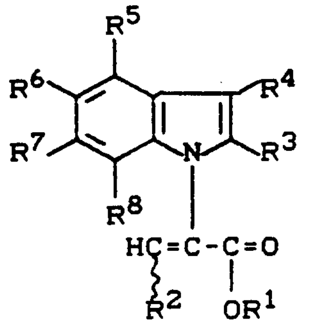

(I)

in welcher

| | |
|---|---|
| $R^1$ | für Alkyl oder für unsubstituiertes oder substituiertes Aralkyl steht, |
| $R^2$ | für Dialkylamino, Alkoxy, Alkylthio oder für jeweils unsubstituiertes oder substituiertes Aralkyloxy oder Arylalkylthio steht, |
| $R^3$ und $R^4$ | jeweils unabhängig voneinander für Wasserstoff, Cyano, Halogen oder Alkyl stehen, |
| $R^5$, $R^6$ und $R^8$ | unabhängig voneinander jeweils für Wasserstoff, Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils unsubstituiertes oder im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil stehen und |
| $R^7$ | für eine der folgenden Gruppierungen steht |

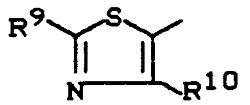 ; 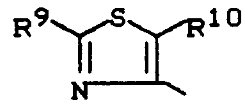 ; 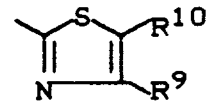

oder 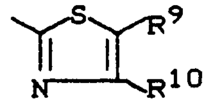

wobei

$R^9$ und $R^{10}$ jeweils unabhängig voneinander für Wasserstoff, Halogen, Cyano, Nitro, Alkyl, Alkoxy, Alkylthio, Halogenalkyl, Alkoxycarbonyl, Dialkylaminocarbonyl oder für jeweils unsubstituiertes oder substituiertes Aryl, Aralkyl, Aryloxy, Arylthio, Aralkyloxy, Aralkylthio, Hetaryl, Hetaryloxy oder Hetarylthio stehen.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäße beansprucht.

Weiterhin wurde gefunden, daß man die neuen heterocyclisch substituierten Acrylsäureester der allgemeinen Formel (I),

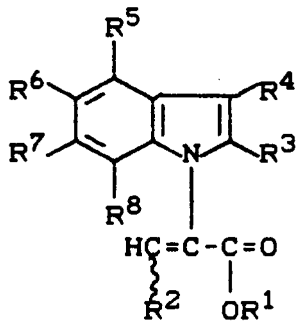

(I)

in welcher

$R^1$ bis $R^8$ die oben angegebenen Bedeutungen haben,

nach einem der im folgenden beschriebenen Verfahren erhält:

a) Man erhält substituierte Acrylsäureester der allgemeinen Formel (Ia)

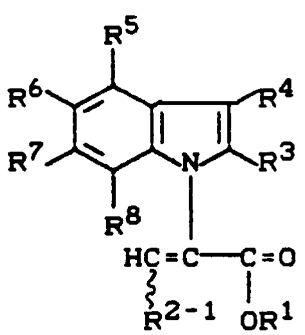

(Ia)

in welcher

$R^1$, $R^3$, $R^4$, $R^5$, $R^6$, $R^7$ und $R^8$ die oben angegebene Bedeutung haben und

$R^{2-1}$ für Alkoxy oder unsubstituiertes oder substituiertes Aralkyloxy steht,

wenn man Hydroxyacrylsäureester oder deren Alkalimetallsalze der Formel (II)

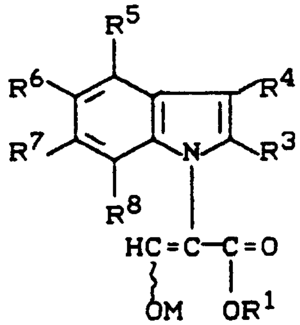

(II)

in welcher

M für Wasserstoff oder für ein Alkalimetallkation steht und

$R^1$, $R^3$, $R^4$, $R^5$, $R^6$, $R^7$ und $R^8$ die oben angegebene Bedeutung haben,

mit Alkylierungsmitteln der Formel (III)

$R^{11}$-$E^1$ (III)

in welcher

$R^{11}$ für Alkyl oder unsubstituiertes oder substituiertes Aralkyl steht und

$E^1$ für eine elektronenanziehende Abgangsgruppe steht,

gegebenenfalls in Gegenwart eines Verdünnungsmittels und gegebenenfalls in Gegenwart eines Reaktionshilfsmittels umsetzt;

b) man erhält substituierte Acrylsäureester der allgemeinen Formel (Ib)

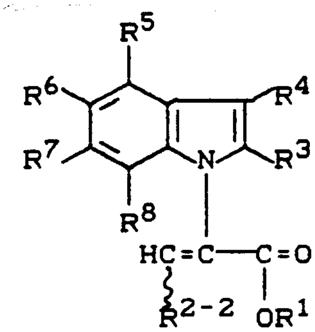

(Ib)

in welcher

$R^1$, $R^3$, $R^4$, $R^5$, $R^6$, $R^7$ und $R^8$ die oben angegebene Bedeutung haben und

$R^{2-2}$ für Dialkylamino steht,

wenn man substituierte Essigsäureester der Formel (IV)

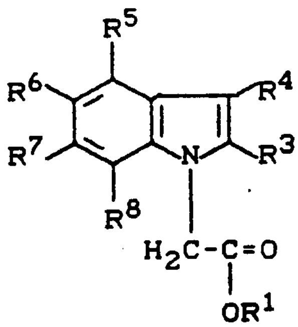

(IV)

in welcher

$R^1$, $R^3$, $R^4$, $R^5$, $R^6$, $R^7$ und $R^8$ die oben angegebene Bedeutung haben,
mit Formamiden der Formel (Va)

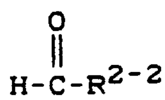

(Va)

in welcher
$R^{2-2}$ die oben angegebene Bedeutung hat,
oder mit Formamid-Derivaten der Formel (Vb)

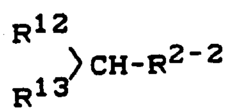

(Vb)

in welcher
$R^{12}$ und $R^{13}$ unabhängig voneinander für Alkoxy oder Dialkylamino stehen und
$R^{2-2}$ die oben angegebene Bedeutung hat,
gegebenenfalls in Gegenwart eines Verdünnungsmittels umsetzt;
c) man erhält substituierte Acrylsäureester der Formel (Ic)

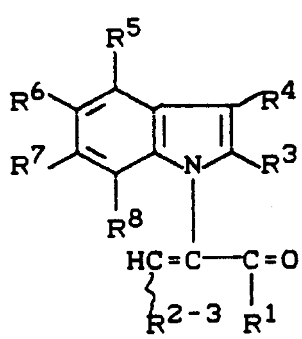

(Ic)

in welcher
$R^1$, $R^3$, $R^4$, $R^5$, $R^6$, $R^7$ und $R^8$ die oben angegebene Bedeutung haben und
$R^{2-3}$ für Alkylthio oder unsubstituiertes oder substituiertes Aralkylthio steht,
wenn man Ketocarbonsäurederivate der Formel (VI)

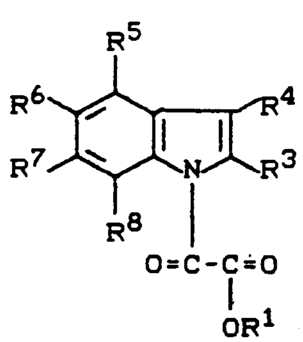

(VI)

in welcher
$R^1$, $R^3$, $R^4$, $R^5$, $R^6$, $R^7$ und $R^8$ die oben angegebene Bedeutung haben,
mit metallorganischen Verbindungen der Formel (VII)

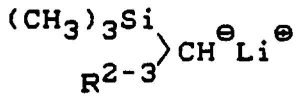

(VII)

in welcher
$R^{2-3}$ die oben angegebene Bedeutung hat,
gegebenenfalls in Gegenwart eines Verdünnungsmittels umsetzt;
d) man erhält substituierte Acrylsäureester der Formel (Ic) weiterhin, wenn man substituierte Acrylsäureester der Formel (VIII)

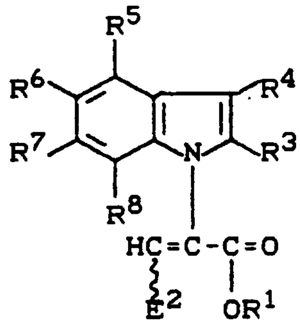

(VIII)

in welcher
$R^1$, $R^3$, $R^4$, $R^5$, $R^6$, $R^7$ und $R^8$ die oben angegebene Bedeutung haben und
$E^2$ für eine elektronenanziehende Abgangsgruppe steht,
mit Thiolen der Formel (IX)

$R^{2-3}$-H (IX)

in welcher
$R^{2-3}$ die oben angegebene Bedeutung hat,
gegebenenfalls in Gegenwart eines Verdünnungsmittels und gegebenenfalls in Gegenwart eines Reaktionshilfsmittels umsetzt.

Schließlich wurde gefunden, daß die neuen heterocyclisch substituierten Acrylsäureester und auch die Essigsäureester der allgemeinen Formel (I) bzw. der Formel (IV) eine gute Wirkung gegen Schädlinge besitzen.

Überraschenderweise zeigen die erfindungsgemäßen substituierten Acrylsäureester der allgemeinen Formel (I) eine insektizide Wirkung sowie eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche strukturell und wirkungsmäßig naheliegende Verbindungen sind.

Die erfindungsgemäßen neuen heterocyclisch substituierten Acrylsäureester sind durch die Formel (I) allgemein definiert. Falls nicht anders definiert, werden bevorzugte Substituenten bzw. Bereiche der in dieser und den nachstehend erwähnten Formeln aufgeführten Reste im folgenden erläutert:
Für Alkyl in den Definitionen von $R^1$, $R^3$, $R^4$, $R^5$, $R^6$, $R^8$, $R^9$ und $R^{10}$ in den allgemeinen Formeln, steht geradkettiges oder verzweigtes Alkyl mit vorzugsweise 1 bis 8, besonders bevorzugt 1 bis 6 und insbesondere 1 bis 4, Kohlenstoffatomen. Beispielhaft und vorzugsweise seien Methyl, Ethyl, n-Propyl, i-Propyl, n-Butyl, sec-Butyl, i-Butyl, t-Butyl, n-Pentyl, i-Pentyl, t-Pentyl und n-Hexyl genannt.

Dialkylamino in der Definiton von $R^2$ oder in Zusammensetzungen wie Dialkylaminocarbonyl in der Definition von $R^9$ und $R^{10}$ steht für eine Aminogruppe mit 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien.

Beispielhaft und vorzugsweise seien Dimethylamino, Diethylamino, Di-n-propylamino und Di-i-propylamino aufgeführt.

Unter dem Begriff unsubstituiertes oder substituiertes Aryl in der Definition von $R^5$, $R^6$, $R^8$, $R^9$ und $R^{10}$ in den allgemeinen Formeln ist Aryl mit vorzugsweise 6 bis 10 Kohlenstoffatomen im Arylteil zu verstehen. Beispielhaft und vorzugsweise seien unsubstituiertes oder substituiertes Phenyl oder Naphthyl, insbesondere Phenyl genannt.

Unsubstituiertes oder substituiertes Aralkyl in den Definitionen von $R^1$, $R^5$, $R^6$, $R^8$, $R^9$ und $R^{10}$ enthält vorzugsweise 1 bis 6, insbesondere 1 bis 4 Kohlenstoffatome im geradkettigen oder verzweigten Alkylteil und vorzugsweise Phenyl als Arylteil. Als Aralkylgruppen seien beispielhaft und vorzugsweise Benzyl und Phenethyl genannt.

Heteroaryl in der Definition von $R^5$, $R^6$, $R^8$, $R^9$ und $R^{10}$ steht im allgemeinen für einen 5- bis 6-gliedrigen Ring, der ein bis 4, bevorzugt 1 bis 3 gleiche oder verschiedene Heteroatome, enthält. Als Heteroatome seien vorzugsweise Sauerstoff, Schwefel und Stickstoff genannt; beispielhaft und vorzugsweise seien genannt: Pyrimidinyl, Pyrrolyl, Isothiazolyl, Oxazolyl, Pyridyl, Thienyl, Furyl, Pyridazinyl, Pyrazinyl, Isoxazolyl, Thiazolyl und Pyrazolyl.

Unter dem Begriff Alkoxy in der Definition von $R^2$, $R^5$, $R^6$, $R^8$, $R^9$ und $R^{10}$ in den allgemeinen Formeln ist geradkettiges oder verzweigtes Alkoxy mit vorzugsweise 1 bis 6, insbesondere 1 bis 4 Kohlenstoffatomen zu verstehen. Beispielhaft und vorzugsweise seien Methoxy, Ethoxy, Propoxy, Butoxy sowie ihre Isomeren, i-Propoxy, i-, s- und t-Butoxy genannt.

Halogen steht in den Definitionen $R^3$, $R^4$, $R^5$, $R^6$, $R^8$, $R^9$ und $R^{10}$ für Fluor, Chlor, Brom und Iod, bevorzugt für Fluor, Chlor und Brom, besonders bevorzugt für Fluor und Chlor.

Alkyl steht in Zusammensetzungen wie Alkoximinoalkyl in den Definitionen $R^5$, $R^6$ und $R^8$ für geradkettiges oder verzweigtes Alkyl, bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkylthio steht in den Definitionen $R^2$, $R^5$, $R^6$, $R^8$, $R^9$ und $R^{10}$ für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen in den Definitionen von $R^5$, $R^6$, $R^8$, $R^9$ und $R^{10}$ für geradkettiges oder verzweigtes Halogenalkyl oder Halogenalkoxy mit je 1 bis 6, insbesondere 1 bis 4 Kohlenstoffatomen, besonders bevorzugt mit 1 oder 2 Kohlenstoffatomen und jeweils 1 bis 13, insbesondere 1 bis 9, vorzugsweise 1 bis 5, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht in den Definitionen von $R^5$, $R^6$ und $R^8$ für geradkettige oder verzweigtes Halogenalkylthio mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft und vorzugsweise seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Alkoxycarbonyl steht in den Definitionen $R^5$, $R^6$, $R^8$, $R^9$ und $R^{10}$ für geradkettiges oder verzweigtes Alkoxycarbonyl mit 1 bis 6, insbesondere 1 bis 4, vorzugsweise 1 oder 2 Kohlenstoffatomen im Alkoxyrest; beispielhaft und vorzugsweise seien genannt: Methoxycarbonyl, Ethoxycarbonyl, n-Propoxycarbonyl, i-Propoxycarbonyl, n-, i-, s- und t-Butoxycarbonyl.

Cycloalkyl steht in den Definitionen $R^5$, $R^6$ und $R^8$ für Cycloalkyl mit vorzugsweise 3 bis 7, insbesondere 3, 5 oder 6 Kohlenstoffatomen. Beispielhaft und vorzugsweise seien unsubstituiertes oder substituiertes Cyclopropyl, Cyclopentyl und Cyclohexyl genannt.

Unsubstituiertes oder substituiertes Aryloxy und Arylthio stehen in den Definitionen von $R^5$, $R^6$, $R^8$, $R^9$ und $R^{10}$ in den allgemeinen Formeln für Aryl mit vorzugsweise 6 bis 10 Kohlenstoffatomen im Arylteil.

Vorzugsweise seien unsubstituiertes oder substituiertes Phenoxy oder Phenylthio, insbesondere Phenoxy, genannt.

Unsubstituiertes oder substituiertes Aralkyloxy oder Aralkylthio enthalten in den Definitionen $R^2$, $R^5$, $R^6$, $R^8$, $R^9$ und $R^{10}$ vorzugsweise 1 bis 6 Kohlenstoffatome im geradkettigen oder verzweigten Alkylteil und vorzugsweise Phenyl als Arylteil. Als Aralkylgruppen seien vorzugsweies Benzyl und Phenethyl genannt.

Heteroarylalkyl, Heteroaryloxy und Heteroarylthio in der Definition von $R^5$, $R^6$, $R^8$, $R^9$ und $R^{10}$ stehen im allgemeinen für einen 5- bis 6-gliedrigen Ring, der ein oder mehrere Heteroatome, bevorzugt 1 bis 3, insbesondere 1 oder 2, gleiche oder verschiedene Heteroatome, enthält. Als Heteroatome seien vorzugsweise Sauerstoff, Schwefel und Stickstoff genannt; beispielhaft und vorzugsweise seien genannt: Pyridyl, Thienyl, Furyl, Pyridazinyl, Pyrazinyl, Isoxazolyl, Thiazolyl, Pyridylmethyl, Thienylmethyl, Furylmethyl, Pyridyloxy, Thienyloxy, Furyloxy, Pyridazinyloxy, Pyrazinyloxy, Isoxazolyloxy, Thiazolyloxy, Pyridylmethyloxy, Thienylmethyloxy, Furylmethyloxy, Pyridylthio, Thienylthio, Furylthio, Pyridazinylthio, Pyrazinylthio, Isoxazolylthio, Thiazolylthio, Pyridylmethylthio, Thienylmethylthio und Furylmethylthio.

Die Substituenten für die Arylreste als solche oder in Zusammensetzungen wie Arylalkyl, Aryloxy, Arylthio, Aralkyloxy, Aralkylthio und für die heterocyclischen Ringe wie Heteroarylalkyl und Heteroaryl haben die im folgenden angegebenen Bedeutungen.

Halogen steht im allgemeinen als Substituent für Fluor, Chlor, Brom und Iod, bevorzugt für Fluor, Chlor und Brom, besonders bevorzugt für Fluor und Chlor.

Alkyl steht im allgemeinen als Substituent oder in Zusammensetzungen wie Alkoximinoalkyl für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl, i-Propyl und t-Butyl. Die beispielhafte Aufzählung entspricht der weiter oben gegebenen.

Alkoxy steht im allgemeinen als Substituent oder in Zusammensetzungen wie Alkoximinoalkyl für geradkettiges oder verzweigtes Alkoxy mit 1 bis 6, besonders bevorzugt 1 bis 3 Kohlenstoffatomen je Alkylrest; beispielhaft und vorzugsweise seien genannt: Methoxy, Ethoxy, n-und i-Propoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio, Ethylthio, Propylthio, Butylthio, Pentylthio sowie ihre Isomeren, wie z.B. i-Propylthio, i-, s- und t-Butylthio, 1-Methyl-butylthio, 2-Methyl-butylthio und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen im allgemeinen als Substituenten in den Resten für geradkettiges oder verzweigtes Halogenalkyl oder Halogenalkoxy mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettige oder verzweigtes Halogenalkylthio mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Die hier aufgeführten Definitionen gelten in entsprechender Weise auch für die im folgenden aufgeführten bevorzugten Kombinationen von Resten.

Bevorzugt sind Verbindungen der Formel (I), bei welchen

$R^1$ für geradkettiges oder verzweigtes Alkyl mit 1 bis 6 Kohlenstoffatomen oder für Benzyl steht,

$R^2$ für Dialkylamino mit jeweils 1 bis 6 Kohlenstoffatomen in den einzelnen geradkettigen oder verzweigten Alkylteilen, jeweils geradkettiges oder verzweigtes Alkoxy oder Alkylthio mit 1 bis 6 Kohlenstoffatomen oder für jeweils unsubstituiertes oder einfach oder mehrfach, gleich oder verschieden substituiertes Benzyloxy oder Benzylthio

steht, wobei als Phenylsubstituenten infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Cycloalkyl mit 3 bis 7 Kohlenstoffatomen,

$R^3$ und $R^4$ jeweils unabhängig voneinander für Wasserstoff, Cyano, Fluor, Chlor, Brom oder geradkettiges oder verzweigtes Alkyl mit 1 bis 6 Kohlenstoffatomen stehen,

$R^5$, $R^6$ und $R^8$ unabhängig voneinander für Wasserstoff, Fluor, Chlor, Brom, Methyl, Ethyl, n- oder i-Propyl, n-, i-, s- oder t-Butyl, Methoxy, Ethoxy, n- oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, zweifach verknüpftes 1,3-Propandiyl oder 1,4-Butandiyl oder für jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Benzyloxy, Phenylthio oder Benzylthio stehen und

$R^7$ für eine der folgenden Gruppierungen steht

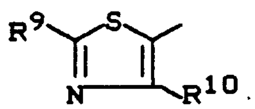 ; 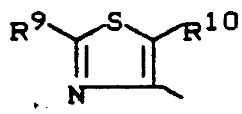 ; 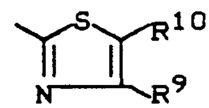

oder 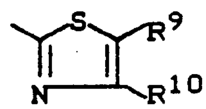

wobei

$R^9$ und $R^{10}$ jeweils unabhängig voneinander für Wasserstoff, Fluor, Chlor, Brom, Cyano, jeweils geradkettiges oder verzweigtes Alkyl, Alkoxy, Alkylthio oder Halogenalkyl mit 1 bis 6 Kohlenstoffatomen und gegebenenfalls 1 bis 13 gleichen oder verschiedenen Halogenatomen steht, für geradkettiges oder verzweigtes Alkoxycarbonyl mit 1 bis 6 Kohlenstoffatomen im Alkoxyteil, jeweils in den einzelnen Alkylteilen geradkettiges oder verzweigtes, gleich oder verschieden substituiertes Dialkylaminocarbonyl mit 1 bis 4 Kohlenstoffatomen in jedem Alkylteil oder für jeweils unsubstituiertes oder einfach bis dreifach, gleich oder verschieden substituiertes Phenyl, Benzyl, Phenyloxy, Phenylthio, Benzyloxy oder Benzylthio steht oder für unsubstituiertes oder einfach oder zweifach, gleich oder verschieden substituiertes 5- oder 6-gliedriges Heteroaryl steht, das 1 bis 3 Heteroatome aus der Reihe Stickstoff, Sauerstoff oder Schwefel enthält, wobei als Substituenten jeweils Halogen, Alkyl, Alkoxy, Alkylthio, Halogenalkyl, Halogenalkyloxy, Halogenalkylthio mit jeweils 1 bis 4 Kohlenstoffatomen und gegebenenfalls 1 bis 9 gleichen oder verschiedenen Halogenatomen, Dialkylamino oder Dialkylaminocarbonyl mit jeweils 1 bis 4 Kohlenstoffatomen in den jeweils geradkettigen oder verzweigten Alkylteilen, 1,3-Propandiyl, 1,4-Butandiyl oder unsubstituiertes oder einfach bis zweifach, gleich oder verschieden substituiertes Phenyl oder Benzyl genannt seien, wobei als Substituenten jeweils Halogen oder Phenyloxy infrage kommen.

Besonders bevorzugt sind Verbindungen der Formel (I), bei welchen

$R^1$ für geradkettiges oder verzweigtes Alkyl mit 1 bis 4 Kohlenstoffatomen steht,

$R^2$ für Dialkylamino mit jeweils 1 bis 4 Kohlenstoffatomen in den einzelnen geradkettigen oder verzweigten Alkylteilen, jeweils geradkettiges oder verzweigtes Alkoxy oder Alkylthio mit 1 bis 4 Kohlenstoffatomen oder für Benzyloxy oder Benzylthio steht,

$R^3$ und $R^4$ gleich oder verschieden sind und für Wasserstoff, Cyano, Fluor, Chlor, Brom, Methyl, Ethyl, n-oder i-Propyl, n-, i-, s- oder t-Butyl stehen,

$R^5$ und $R^6$ gleich oder verschieden sind und für Wasserstoff, Fluor, Chlor, Brom, Cyano, Nitro, Methyl, Ethyl, n- oder i-Propyl, n-, i-, s-oder t-Butyl, Methoxy, Ethoxy, n- oder i-Propoxy,

Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl oder Cyclohexyl stehen oder gemeinsam für eine Methylendioxy-, 1,3-Propandiyl- oder 1,4-Butandiylgruppierung stehen,

$R^7$ für eine der folgenden Gruppierungen steht,

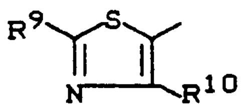 ; 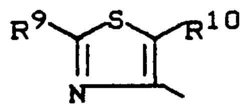 ; 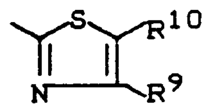

oder 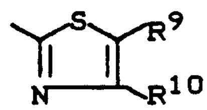

wobei

$R^9$ für Wasserstoff, geradkettiges oder verzweigtes Alkyl mit 1 bis 6 Kohlenstoffatomen, jeweils unsubstituiertes oder einfach bis dreifach, gleich oder verschieden substituiertes Phenyl, Benzyl oder für einfach oder zweifach, gleich oder verschieden substituiertes 5- oder 6-gliedriges Heteroaryl steht, das 1 bis 3 Heteroatome aus der Reihe Stickstoff, Sauerstoff oder Schwefel enthält, wobei als Substituenten jeweils Halogen, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, n-, i-, s- oder t-Butylthio, Halogenalkyl, Halogenalkoxy oder Halogenalkylthio mit jeweils 1 oder 2 Kohlenstoffatomen und 1 bis 5 gleichen oder verschiedenen Fluor oder Chloratomen, Dialkylamino oder Dialkylaminocarbonyl mit 1 oder 2 Kohlenstoffatomen in den einzelnen Alkylteilen, unsubstituiertes oder einfach bis zweifach, gleich oder verschieden substituiertes Phenyl, Phenoxy oder Benzyl genannt seien, wobei als Substituenten jeweils Fluor, Chlor oder Phenoxy infrage kommen und

$R^{10}$ für Wasserstoff, geradkettiges oder verzweigtes Alkyl mit 1 bis 4 Kohlenstoffatomen, Chlor, Brom, Cyano, Methoxycarbonyl, Ethoxycarbonyl, Dimethylaminocarbonyl, Diethylaminocarbonyl, Methoxy, Ethoxy, Methylthio oder Ethylthio steht und

$R^8$ für Wasserstoff, Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl oder Ethoxycarbonyl steht.

Ganz besonders bevorzugt sind Verbindungen der Formel (I), bei welchen

$R^1$ für Methyl oder Ethyl steht,

$R^2$ für Dimethylamino, Diethylamino, Methoxy, Ethoxy, Methylthio, Ethylthio, Benzyloxy oder Benzylthio steht,

$R^3$ für Wasserstoff, Chlor oder Methyl steht,

$R^4$ für Wasserstoff, Chlor oder Methyl steht,

$R^5$ für Wasserstoff, Fluor, Chlor, Brom, Methyl, Ethyl, Trifluormethyl, Methoxycarbonyl oder Ethoxycarbonyl steht,

$R^6$ für Wasserstoff, Fluor, Chlor, Brom, Methyl, Ethyl, Methoxy, Ethoxy, Methylthio, Trifluormethyl, Methoxycarbonyl oder Ethoxycarbonyl steht oder

$R^5$ und $R^6$ gemeinsam für eine Methylendioxy-, 1,3-Propandiyl- oder 1,4-Butandiylgruppiurung stehen,

$R^7$ für eine der folgenden Gruppierungen steht

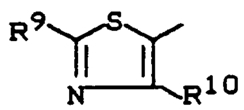 ; 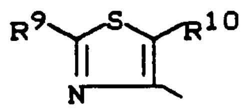 ; 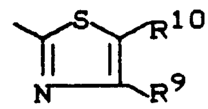

oder 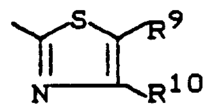

wobei

$R^9$ für Wasserstoff, Methyl, Ethyl, n- oder i-Propyl, n-, s-, i-Butyl, n- oder i-Pentyl, n-Hexyl, Benzyl, o-, m- oder p-Chlorbenzyl, o-, m- oder p-Methylbenzyl, Phenyl oder jeweils einfach bis dreifach, gleich oder verschieden substituiertes Phenyl oder für einfach oder zweifach, gleich oder verschiedes substituiertes 5- oder 6-gliedriges Heteroaryl steht, das 1 oder 2 Heteroatome aus der Reihe Stickstoff, Sauerstoff oder Schwefel enthält, wobei als Substituenten jeweils Fluor, Chlor, Methyl, Ethyl, t-Butyl, Methoxy, Ethoxy, Methylthio, Trifluormethyl, Trifluormethoxy, Trifluormethylthio, Phenyl, p-Chlorphenyl, m- oder p-Phenoxyphenyl oder Benzyl infrage kommen und

$R^{10}$ für Wasserstoff, Methyl, Ethyl, Chlor, Brom, Methoxycarbonyl oder Ethoxycarbonyl steht, und

$R^8$ für Wasserstoff, Methyl oder Ethyl steht.

Im einzelnen seien außer den bei den Herstellungsbeispielen genannten Verbindungen die folgenden heterocyclisch substituierten Acrylsäureester der allgemeinen Formel (I) genannt:

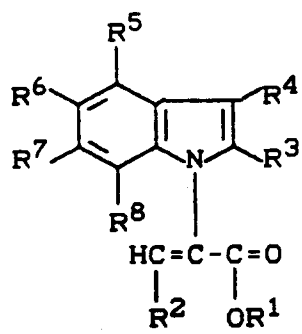 (I)

**Tabelle 1:**

| $R^1$ | $R^2$ | $R^3$ | $R^4$ | $R^5$ | $R^6$ | $R^7$ | $R^8$ |
|---|---|---|---|---|---|---|---|
| $C_2H_5$ | $OCH_3$ | H | H | H | H | 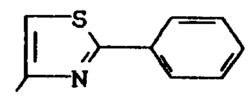 | H |
| $C_2H_5$ | $OCH_3$ | H | H | H | H | 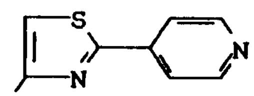 | H |
| $C_2H_5$ | $OCH_3$ | H | H | H | H | 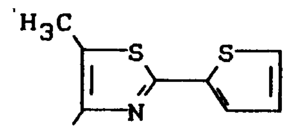 | H |
| $CH_3$ | $SCH_3$ | H | H | H | H | 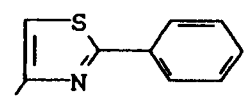 | H |
| $CH_3$ | $SCH_3$ | H | H | H | H | 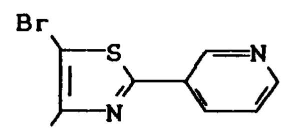 | H |
| $CH_3$ | $SCH_3$ | H | H | H | H | 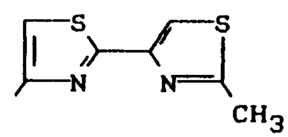 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $N(CH_3)_2$ | H | H | H | H | 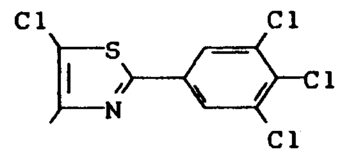 | H |
| $CH_3$ | $OCH_3$ | H | H | $CH_3$ | H | 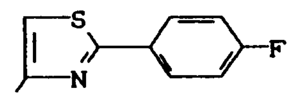 | H |
| $CH_3$ | $OCH_3$ | H | H | $CH_3$ | H | 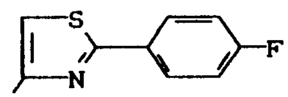 | H |
| $CH_3$ | $OCH_3$ | H | H | Cl | H | 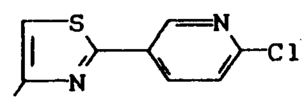 | H |
| $CH_3$ | $OC_2H_5$ | H | H | Cl | H | 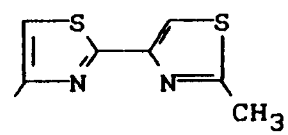 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | $CH_3$ | H | H | H | 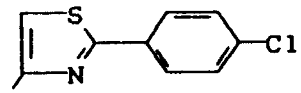 | H |
| $CH_3$ | $OCH_3$ | $CH_3$ | H | H | H | 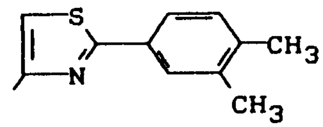 | H |
| $CH_3$ | $OCH_3$ | Cl | H | H | H | 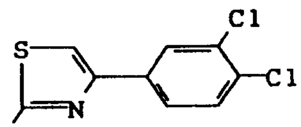 | H |
| $CH_3$ | $OCH_3$ | H | $CH_3$ | H | H | 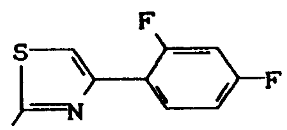 | H |
| $CH_3$ | $OCH_3$ | H | $CH_3$ | H | H | 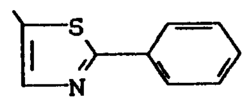 | H |
| $CH_3$ | $OCH_3$ | H | H | H | H | 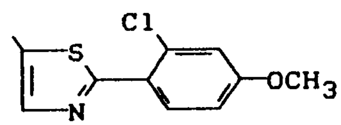 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | $CH_3$ | H | H | H | 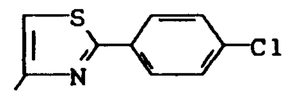 | H |
| $CH_3$ | $OCH_3$ | $CH_3$ | H | H | H | 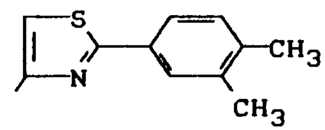 | H |
| $CH_3$ | $OCH_3$ | Cl | H | H | H | 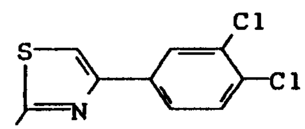 | H |
| $CH_3$ | $OCH_3$ | H | $CH_3$ | H | H | 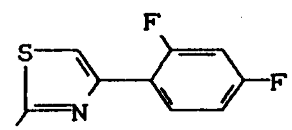 | H |
| $CH_3$ | $OCH_3$ | H | $CH_3$ | H | H | 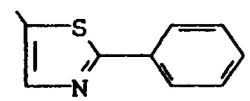 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 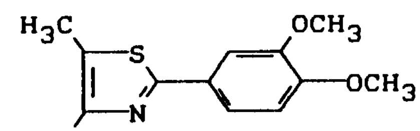 | H |
| $CH_3$ | $OCH_3$ | H | H | H | H | 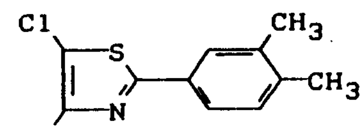 | H |
| $CH_3$ | $OCH_3$ | H | H | H | H | 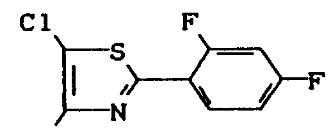 | H |
| $CH_3$ | $OCH_3$ | H | H | H | H | 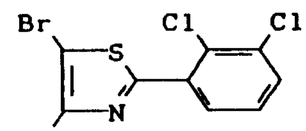 | H |
| $CH_3$ | $OCH_3$ | H | H | H | H | 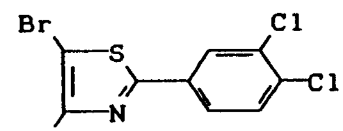 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 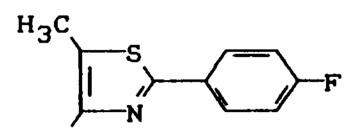 | H |
| $CH_3$ | $OCH_3$ | H | H | H | H | 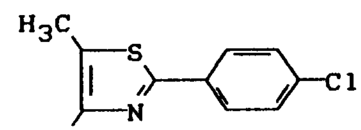 | H |
| $CH_3$ | $OCH_3$ | H | H | H | H | 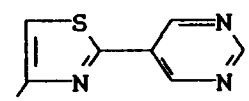 | H |
| $CH_3$ | $OCH_3$ | H | H | H | H | 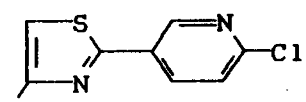 | H |
| $CH_3$ | $OCH_3$ | H | H | H | H | 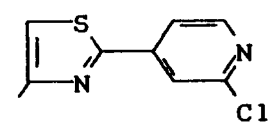 | H |

<u>Tabelle 1</u> - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 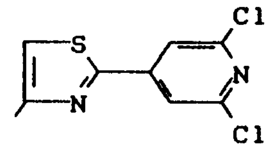 | H |
| $CH_3$ | $OCH_3$ | H | H | H | H | 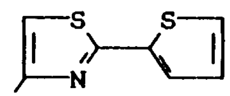 | H |
| $CH_3$ | $OCH_3$ | H | H | H | H | 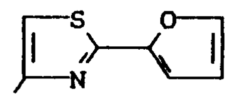 | H |
| $CH_3$ | $OCH_3$ | H | H | H | H | 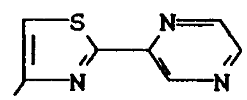 | H |
| $CH_3$ | $OCH_3$ | H | H | H | H | 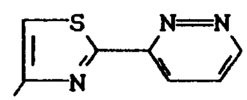 | H |
| $CH_3$ | $OCH_3$ | H | H | H | H | 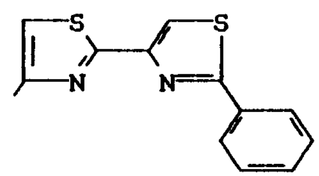 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 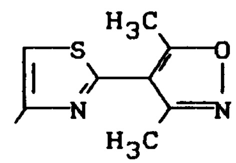 | H |
| $CH_3$ | $OCH_3$ | H | H | H | H | 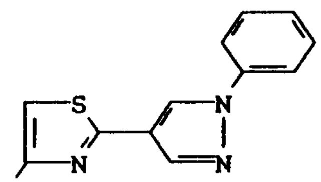 | H |
| $CH_3$ | $OCH_3$ | H | H | H | H | 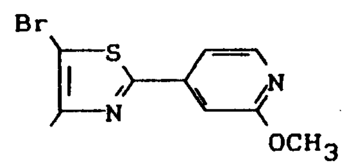 | H |
| $CH_3$ | $OCH_3$ | H | H | H | H | 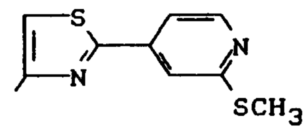 | H |
| $CH_3$ | $OCH_3$ | H | H | H | H | 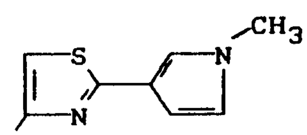 | H |

<u>Tabelle 1</u> - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 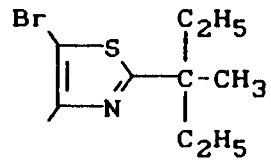 | H |
| $CH_3$ | $OCH_3$ | H | H | H | H | 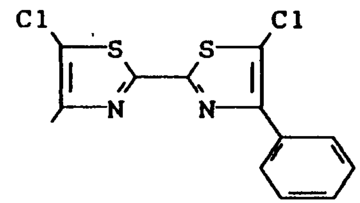 | H |
| $CH_3$ | $OCH_3$ | H | H | H | H | 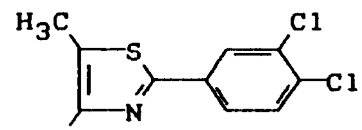 | H |
| $CH_3$ | $OCH_3$ | H | H | H | H | 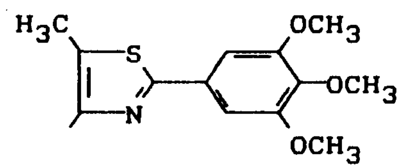 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 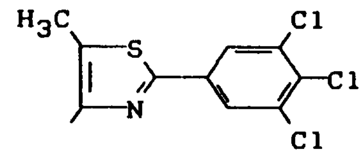 | H |
| $CH_3$ | $OCH_3$ | H | H | H | H | 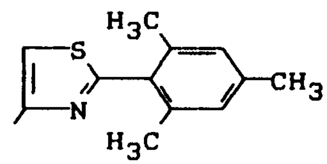 | H |
| $CH_3$ | $OCH_3$ | H | H | H | H | 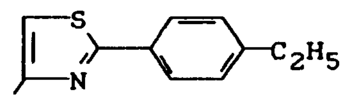 | H |
| $CH_3$ | $OCH_3$ | H | H | H | H | 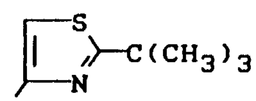 | H |
| $CH_3$ | $OCH_3$ | H | H | H | H | 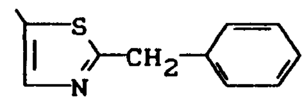 | H |
| $CH_3$ | $OCH_3$ | H | H | H | H | 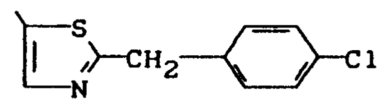 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 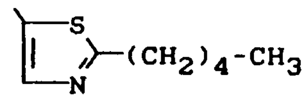 | H |
| $CH_3$ | $OCH_3$ | H | H | H | H | 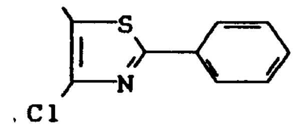 | H |
| $CH_3$ | $OCH_3$ | H | H | H | H | 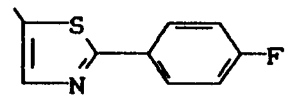 | H |
| $CH_3$ | $OCH_3$ | H | H | H | H | 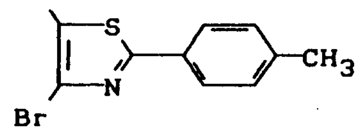 | H |
| $CH_3$ | $OCH_3$ | H | H | H | H | 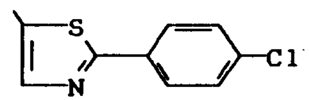 | H |
| $CH_3$ | $OCH_3$ | H | H | H | H | 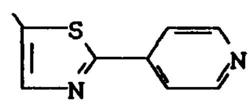 | H |

**Tabelle 1** - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 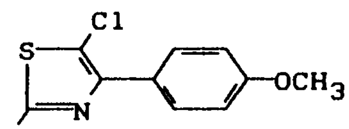 | H |
| $CH_3$ | $OCH_3$ | H | H | H | H | 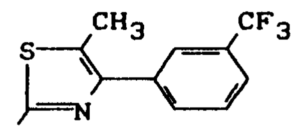 | H |
| $CH_3$ | $OCH_3$ | H | H | H | H | 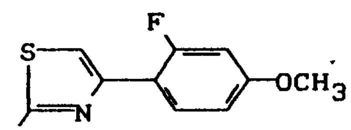 | H |
| $CH_3$ | $OCH_3$ | H | H | H | H | 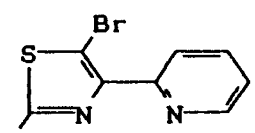 | H |
| $CH_3$ | $OCH_3$ | H | H | H | H | 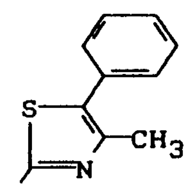 | H |

**Tabelle 1** - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 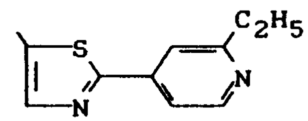 | H |
| $CH_3$ | $OCH_3$ | H | H | H | H | 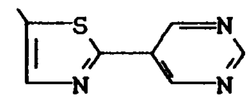 | H |
| $CH_3$ | $OCH_3$ | H | H | H | H | 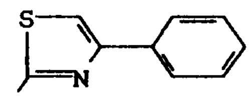 | H |
| $CH_3$ | $OCH_3$ | H | H | H | H | 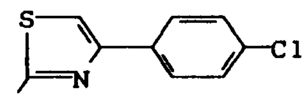 | H |
| $CH_3$ | $OCH_3$ | H | H | H | H | 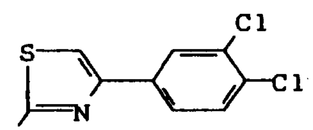 | H |
| $CH_3$ | $OCH_3$ | H | H | H | H | 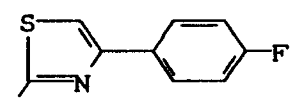 | H |

Tabelle 1 - Fortsetzung

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 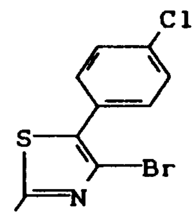 | H |
| $CH_3$ | $OCH_3$ | H | H | H | H | 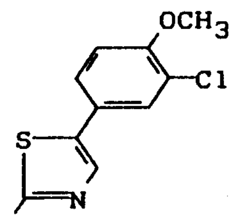 | H |
| $CH_3$ | $OCH_3$ | H | H | H | H | 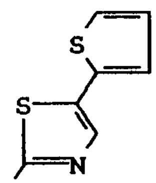 | H |
| $CH_3$ | $OCH_3$ | H | H | H | H | 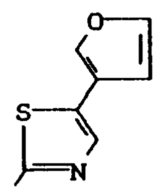 | H |

**Tabelle 1 - Fortsetzung**

| $R^1$ | $R^2$ | $R^3$ | $R^4$ | $R^5$ | $R^6$ | $R^7$ | $R^8$ |
|---|---|---|---|---|---|---|---|
| $CH_3$ | $OCH_3$ | H | H | H | H | 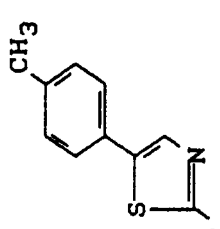 | H |
| $CH_3$ | $OCH_3$ | H | H | H | H | 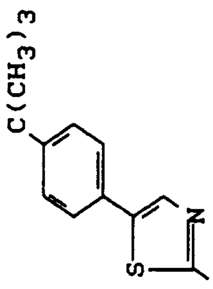 | H |
| $CH_3$ | $OCH_3$ | H | H | H | H | 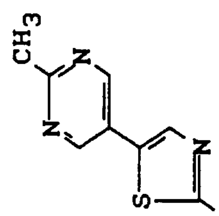 | H |

Verwendet man beispielsweise 3-Hydroxy-2-[6-[2-(2,4-Difluor-phenyl)thiazol-4-yl]-indol-1-yl]-acrylsäuremethylester und Dimethylsulfat als Ausgangsstoffe, so läßt sich der Reaktionsablauf des erfindungsgemäßen Verfahrens (a) durch das folgende Formelschema darstellen:

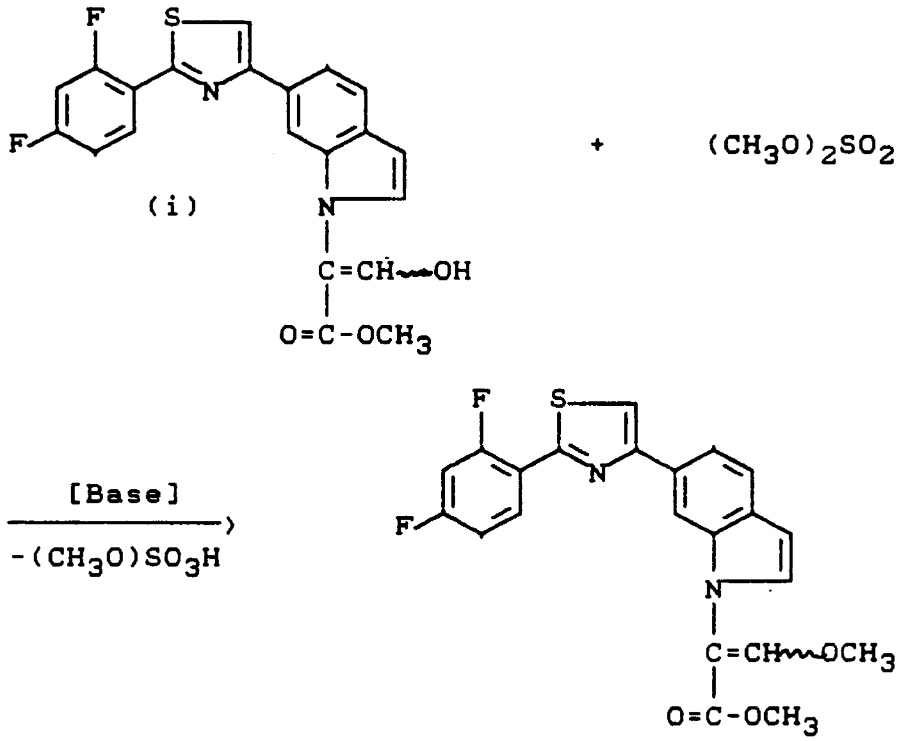


Verwendet man beispielsweise [6-[2-(phenyl)-thiazol-4-yl]-3-methyl-indol-1-yl]-essigsäuremethylester und Dimethylformamiddimethylacetal als Ausgangsstoffe, so läßt sich der Reaktionsablauf des erfindungsgemäßen Verfahrens (b) durch das folgende Reaktionsschema darstellen:

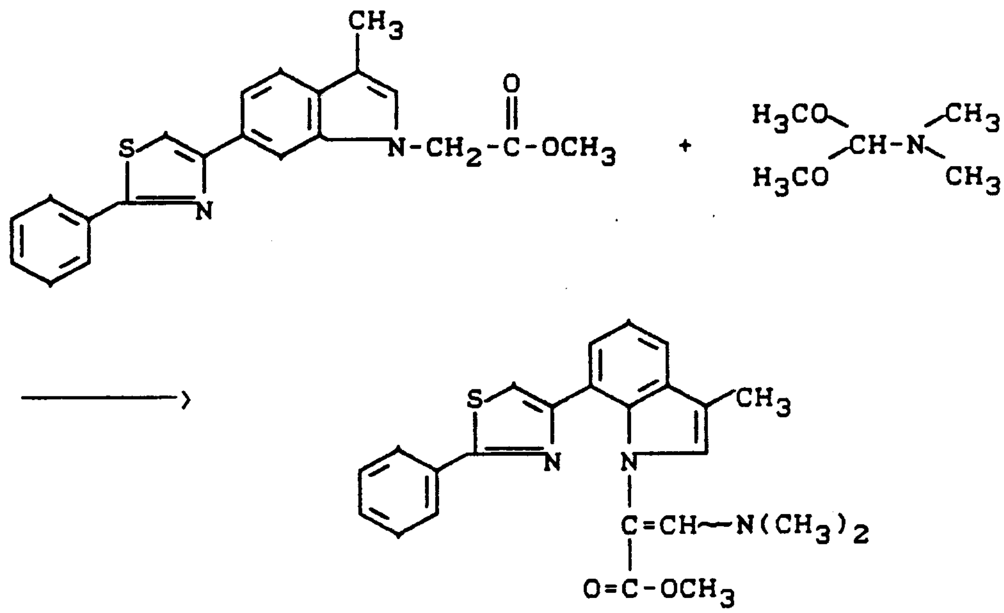


Verwendet man beispielsweise 2-Oxo-[6-[2-(pyridin-4-yl)-thiazol-4-yl]-3-chlor-indol-1-yl]-essigsäuremethylester und [(Methylthio)-(trimethylsilyl)]methylen-lithium als Ausgangsverbindungen, so läßt sich der Reaktionsverlauf des erfindungsgemäßen Verfahrens (c) durch das folgende Formelschema darstellen:

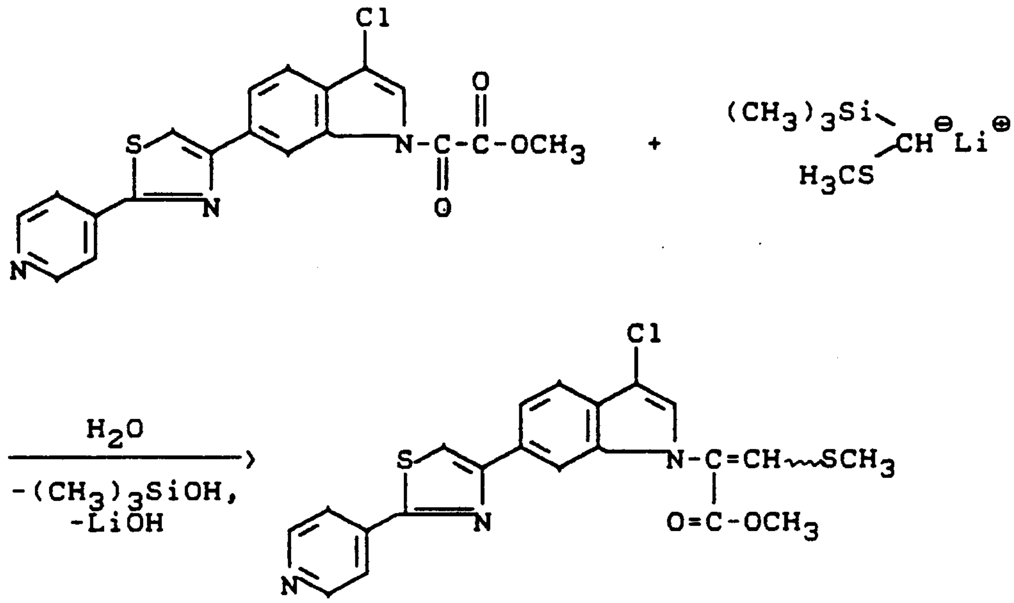


Verwendet man beispielsweise 2-[6-[5-Brom-2-(pyridin-2-yl)-thiazol-4-yl]-indol-1-yl]-3-methansulfonyloxy-acrylsäuremethylester und Methylmercaptan als Ausgangsstoffe, so läßt sich der Reaktionsablauf des erfindungsgemäßen Verfahrens (d) durch das folgende Reaktionsschema darstellen:

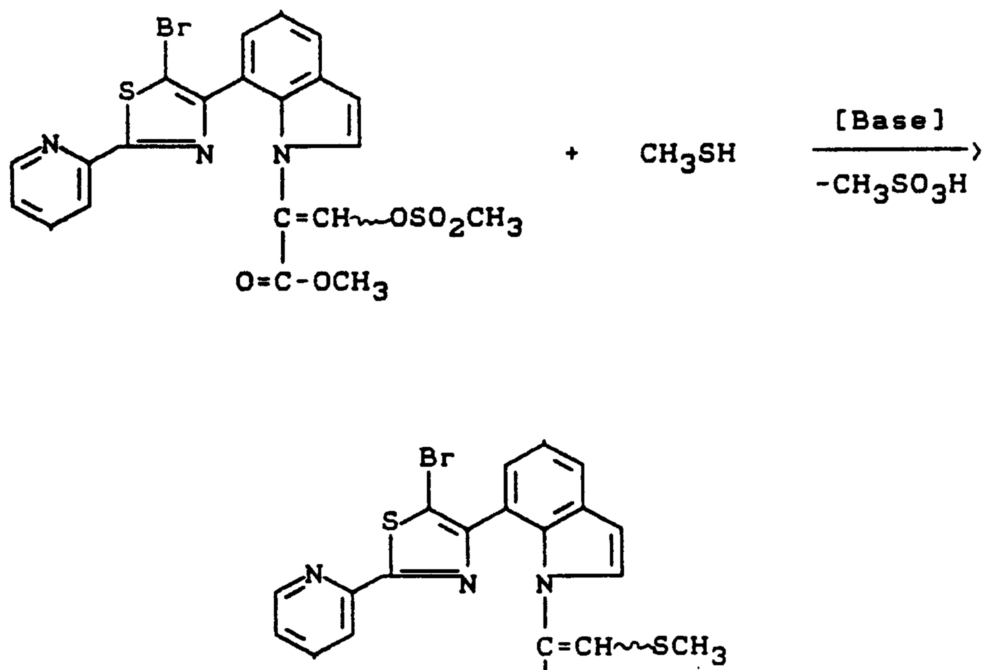


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$, $R^3$, $R^4$, $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Lithium-, Natrium- oder Kaliumkation.

Die zur Durchführung des erfindungsgemäßen Verfahrens (a) benötigten Hydroxyacrylsäureester der Formel (II) sind noch nicht bekannt und Gegenstand der Erfindung.

Man erhält sie, wenn man substituierte Essigsäureester der Formel (IV),

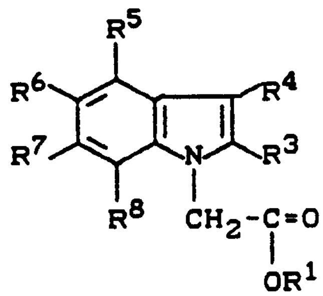

(IV)

in welcher

$R^1$, $R^3$, $R^4$, $R^5$, $R^6$, $R^7$ und $R^8$ die oben angegebene Bedeutung haben,

mit Ameisensäureestern der Formel (X),

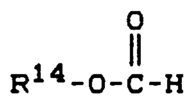

(X)

in welcher

$R^{14}$ für Alkyl, insbesondere für Methyl oder Ethyl steht,

gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen von -20 °C bis +50 °C umsetzt (vgl. z.B. EP 274 825).

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht $R^{11}$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester der allgemeinen Formel (IV) sind neu und Gegenstand der Erfindung. In dieser Formel (IV) stehen $R^1$, $R^3$, $R^4$, $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

Man erhält die Verbindungen der Formel (IV),

wenn man Indolderivate der allgemeinen Formel (XI)

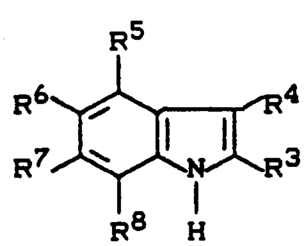

(XI)

in welcher
$R^3$, $R^4$, $R^5$, $R^6$, $R^7$ und $R^8$ die oben angegebene Bedeutung haben,
mit Essigsäurederivaten der allgemeinen Formel (XII)

$E^3$-$CH_2$-$COOR^1$ (XII)

in welcher
$R^1$ die oben angegebene Bedeutung hat und
$E^3$ für eine elektronenanziehende Abgangsgruppe, bevorzugt für Halogen, insbesondere für Chlor oder Brom, steht,
gegebenenfalls in Gegenwart eines Verdünnungsmittels, wie beispielsweise Acetonitril oder Aceton und gegebenenfalls in Gegenwart eines basischen Hilfsmittels, wie beispielsweise Kaliumcarbonat oder Kalium-tert.-butylat bei Temperaturen zwischen -20 °C und +100 °C umsetzt.

Die Essigsäurederivate der Formel (XII) sind allgemein bekannte Verbindungen der organischen Chemie.

Die Indolderivate der allgemeinen Formel (XI) sind noch nicht bekannt und Gegenstand der Erfindung. Man erhält sie jedoch nach bekannten Verfahren in analoger Weise, indem man beispielsweise Nitrobenzol-Derivate der Formel (XIII)

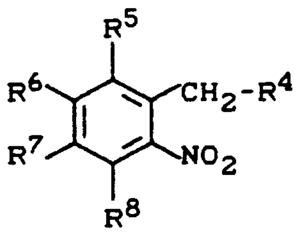

(XIII)

in welcher
$R^4$, $R^5$, $R^6$, $R^7$ und $R^8$ die oben angegebene Bedeutung haben,
mit Verbindungen der Formel (XIV)

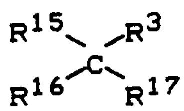

(XIV)

in welcher
$R^3$ die oben angegebene Bedeutung hat,
$R^{15}$ für Alkoxy oder Dialkylamino steht,
$R^{16}$ für Alkoxy oder Dialkylamino steht und
$R^{17}$ für Dialkylamino steht,
gegebenenfalls in Gegenwart eines Verdünnungsmittels wie beispielsweise Toluol oder Dimethylformamid bei Temperaturen zwischen 25 °C und 200 °C und gegebenenfalls unter einem Druck von 1 bis 100 bar zu den Verbindungen der allgemeinen Formel (XV)

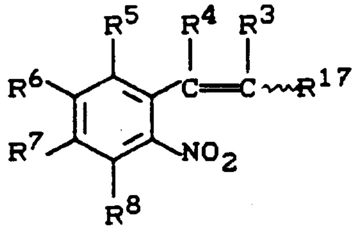

(XV)

in welcher

$R^3$, $R^4$, $R^5$, $R^6$, $R^7$, $R^8$ und $R^{17}$ die oben angegebene Bedeutung haben,

umsetzt und die so erhaltenen Verbindungen der Formel (XV), gegebenenfalls nach ihrer Isolierung und/oder Reinigung, gegebenenfalls in Gegenwart eines Verdünnungsmittels, wie beispielsweise Methanol, Ethanol, Tetrahydrofuran oder Dioxan gegebenenfalls in Gegenwart eines Inertgases, wie beispielsweise Stickstoff, mit üblichen Reduktionsmitteln, wie beispielsweise Wasserstoff, in Gegenwart eines geeigneten Katalysators, wie beispielsweise Raney-Nickel und einem Druck zwischen 1 und 200 bar, bei Temperaturen zwischen -20 °C und +200 °C cyclisiert.

Die Verbindungen der allgemeinen Formel (XIV) sind allgemein bekannte Verbindungen der organischen Chemie (vgl. Tetrahedron 35, 1675 (1979)).

Die Nitrobenzolderivate der Formel (XIII)

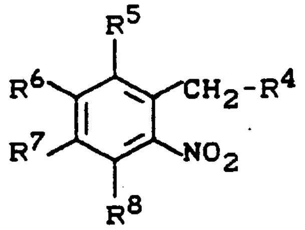

(XIII)

in welcher

$R^4$, $R^5$, $R^6$, $R^7$ und $R^8$ die oben angegebene Bedeutung haben,

sind neu, ausgenommen die Verbindungen 5-Chlor-4-(iodmethyl)-2-(4-methyl-3-nitrophenyl)-thiazol; 4-(Iodmethyl)-2-(4-methyl-3-nitrophenyl)-thiazol; 5-Chlor-4-(chlormethyl)-2-(4-methyl-3-nitrophenyl)-thiazol; 4-(Chlormethyl)-2-(4-methyl-3-nitrophenyl)-5-nitrothiazol; 5-Brom4-(chlormethyl)-2-(4-methyl-3-nitrophenyl)-thiazol; 4-(Chlormethyl)-2-(4-methyl-3-nitrophenyl)-thiazol; 5-Brom-N,N-dimethyl-4-(4-methyl-3-nitrophenyl)-2-thiazolamin und 5-Methyl-2-(p-nitrophenyl)-4-(3-nitro-p-tolyl)-thiazol (vgl. Rev. Roum. Chim. 28 (6), 645-51, 1983; Acta Chim. Acad. Sci. Hung. 83 (3-4), 381-9, 1974; DE-OS 2130981 und Rev. Roum. Chim. 12(7), 905-11).

Man erhält die Nitrobenzolderivate der Formel (XIII) nach einem der im folgenden beschriebenen Verfahren:

a) Man erhält Nitrobenzolderivate der allgemeinen Formel (XIIIa)

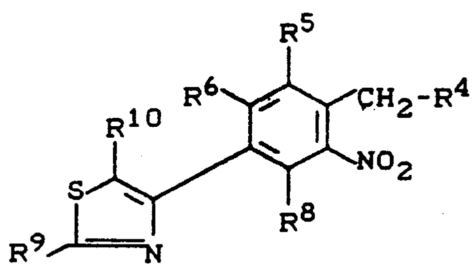

(XIIIa)

in welcher

$R^4$, $R^5$, $R^6$, $R^8$, $R^9$ und $R^{10}$ die oben angegebene Bedeutung haben,

wenn man Verbindungen der Formel (XVI)

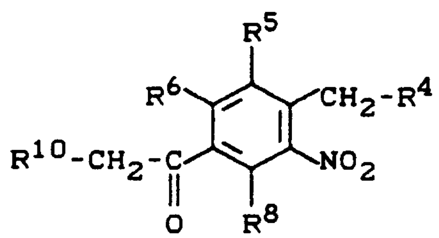

(XVI)

in welcher

$R^4$, $R^5$, $R^6$, $R^8$ und $R^{10}$ die oben angegebene Bedeutung haben,

mit Brom und gegebenenfalls in Gegenwart eines Verdünnungsmittels, wie beispielsweise Eisessig oder Dichlormethan, bei Temperaturen zwischen 0 °C und 100 °C zu den Verbindungen der Formel (XVII)

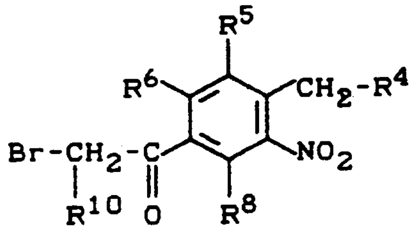

(XVII)

in welcher

$R^4$, $R^5$, $R^6$, $R^8$ und $R^{10}$ die oben angegebene Bedeutung haben,

bromiert und anschließend die Verbindungen der Formel (XVII), mit Thioamiden der Formel (XVIII)

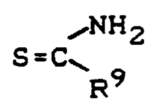

(XVIII)

in welcher

$R^9$ die oben angegebene Bedeutung hat,

gegebenenfalls in Gegenwart eines Verdünnungsmittels, wie beispielsweise Ethanol, bei Temperaturen zwischen -20 °C und +120 °C cyclisiert oder

b) man erhält Nitrobenzolderivate der allgemeinen Formel (XIIIb)

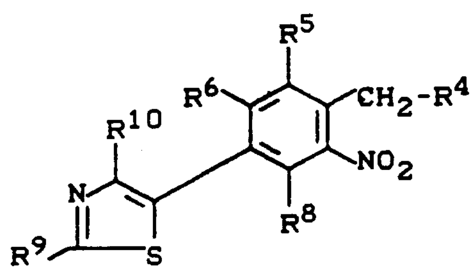

(XIIIb)

in welcher

$R^4$, $R^5$, $R^6$, $R^8$, $R^9$ und $R^{10}$ die oben angegebene Bedeutung haben,

wenn man Verbindungen der Formel (XIX)

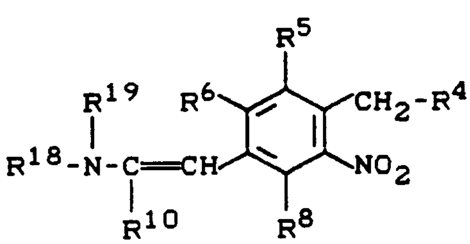

(XIX)

in welcher

$R^4$, $R^5$, $R^6$, $R^8$ und $R^{10}$ die oben angegebene Bedeutung haben und

$R^{18}$ und $R^{19}$ jeweils unabhängig voneinander für geradkettiges oder verzweigtes Alkyl mit 1 bis 4 Kohlenstoffatomen oder gemeinsam für eine geradkettige oder

verzweigte, gesättigte Alkylkette mit 4 bis 8 Kohlenstoffatomen steht, die gegebenenfalls durch ein oder 2 Heteroatome aus der Reihe Sauerstoff, Schwefel oder Stickstoff unterbrochen ist, vorzugsweise jedoch für Dimethylamino, Pyrrolidinyl, Piperidinyl oder Morpholinyl steht,

mit Brom und gegebenenfalls in Gegenwart eines Verdünnungsmittels, wie beispielsweise Dichlormethan, bei Temperaturen zwischen -80 °C und +100 °C zu den Verbindungen der Formel (XX)

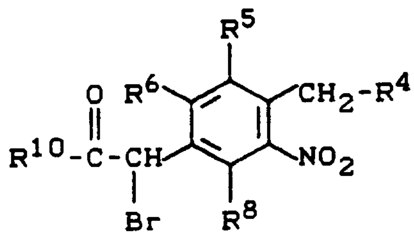

(XX)

in welcher

$R^4$, $R^5$, $R^6$, $R^8$ und $R^{10}$ die oben angegebene Bedeutung haben,

bromiert und anschließend die Verbindungen der Formel (XX) mit Thioamiden der Formel (XVIII)

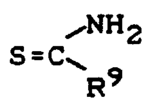

(XVIII)

in welcher

$R^9$ die oben angegebene Bedeutung hat,

gegebenenfalls in Gegenwart eines Verdünnungsmittels, wie beispielsweise Ethanol, bei Temperaturen zwischen -20 °C und +120 °C cyclisiert, oder

c) man erhält Nitrobenzolderivate der allgemeinen Formel (XIIIc-1)

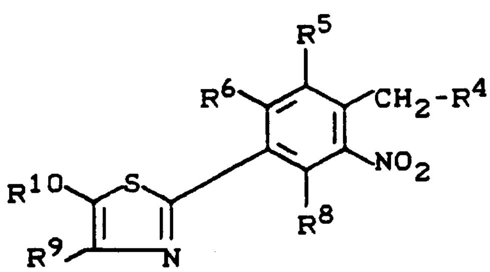

(XIIIc-1)

in welcher

$R^4$, $R^5$, $R^6$, $R^8$, $R^9$ und $R^{10}$ die oben angegebene Bedeutung haben,

oder

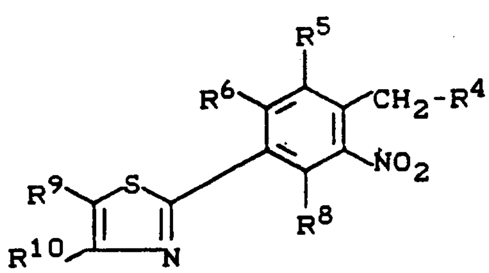

(XIIIc-2)

in welcher
$R^4$, $R^5$, $R^6$, $R^8$, $R^9$ und $R^{10}$ die oben angegebene Bedeutung haben,
wenn man Thioamide der Formel (XXI)

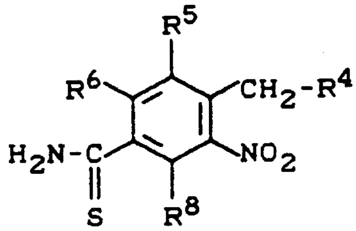

(XXI)

in welcher
$R^4$, $R^5$, $R^6$ und $R^8$ die oben angegebene Bedeutung haben,
mit Ketonen der Formel (XXIIa)

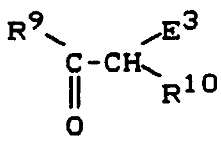

(XXIIa)

in welcher
$R^9$, $R^{10}$ und $E^3$ die oben angegebene Bedeutung haben,
oder
mit Ketonen der Formel (XXIIb)

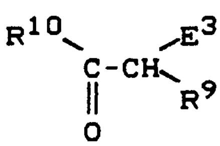

(XXIIb)

in welcher
$R^9$, $R^{10}$ und $E^3$ die oben angegebene Bedeutung haben,
gegebenenfalls in Gegenwart eines Verdünnungsmittels, wie beispielsweise Methanol, Ethanol oder n-Propanol und gegebenenfalls in Gegenwart eines basischen Reaktionshilfsmittels, wie beispielsweise Pyridin oder Kaliumcarbonat bei Temperaturen zwischen -25 °C und +120 °C umsetzt.

Die Thioamide der Formel (XXI) sind neu. Man erhält sie jedoch nach bekannten Verfahren in analoger Weise, indem man beispielsweise Nitrile der Formel (XXIII)

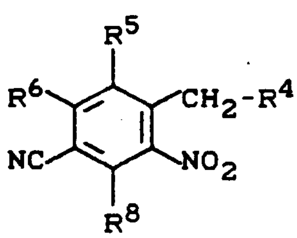

(XXIII)

in welcher
$R^4$, $R^5$, $R^6$ und $R^8$ die oben angegebene Bedeutung haben,

α) entweder in üblicher Art und Weise mit Schwefelkohlenstoff, gegebenenfalls in Gegenwart eines Verdünnungsmittels, wie beispielsweise Toluol, Pyridin oder Dimethylformamid und gegebenenfalls in Gegenwart einer Schutzgasatmosphäre, wie beispielsweise Wasserstoff, bei Temperaturen zwischen 25 °C und 100 °C umsetzt (vgl. Organikum, Organisch-Chemisches Grundpraktikum, VEB Deutscher Verlag der Wissenschaften 1986, 424), oder

β) mit Schwefelwasserstoff und gegebenenfalls in Gegenwart eines Verdünnungsmittels, wie beispielsweise Pyridin und/oder Triethylamin und gegebenenfalls unter Verwendung einer Schutzgasatmosphäre, wie beispielsweise Stickstoff oder Argon, bei Temperaturen zwischen 0 °C und 120 °C umsetzt, oder

γ) gegebenenfalls in Gegenwart von Verdünnungsmitteln, wie beispielsweise Dioxan, Methanol oder Ethanol oder aus Gemischen dieser Lösungsmittel mit Wasser bei Temperaturen zwischen 25 °C und 100 °C oder ohne Verdünnungsmittel mit konzentrierten anorganischen Säuren, wie beispielsweise Schwefelsäure, bei Raumtemperatur zu den Amiden der Formel (XXIV)

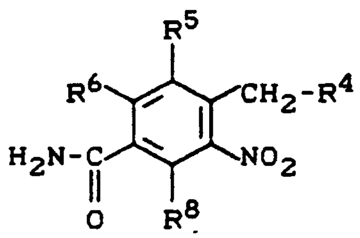

(XXIV)

in welcher

$R^4$, $R^5$, $R^6$ und $R^8$ die oben angegebene Bedeutung haben,

verseift (vgl. Organikum, Organisch-Chemisches Grundpraktikum, VEB Deutscher Verlag der Wissenschaften, 1986, 424) und die so erhaltenen Verbindungen der Formel (XXIV) mit "Lawessons-Reagenz" der Formel (XXV)

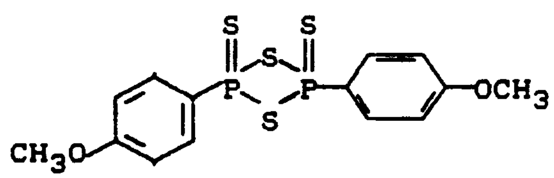

(XXV)

gegebenenfalls in Gegenwart eines Verdünnungsmittels, wie beispielsweise Toluol, Xylol oder Dioxan bei Temperaturen zwischen 20 °C und 150 °C umsetzt.

Die Verbindungen der Formel (XXIII) und (XXV) sind allgemein bekannte Verbindungen der organischen Chemie.

In manchen Fällen kann es vorteilhaft sein, die Nitrobenzolderivate der Formel (XIIIa), (XIIIb), (XIIIc-1) und (XIIIc-2), in welchen $R^{10}$ für Wasserstoff steht, mit geeigneten Reaktionspartnern, wie beispielsweise Halogenierungsmitteln oder Nitriermitteln nach allgemein bekannten, üblichen Methoden der organischen Chemie, zu den Verbindungen der allgemeinen Formel (XIII) umzusetzen und diese gegebenenfalls durch weitere geeignete Umsetzungen zu derivatisieren.

Die Verbindungen der Formel (XVI), (XVIII), (XIX), (XXIIa) und (XXIIb) sind allgemein bekannte Verbindungen der organischen Chemie.

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht $R^{2-2}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{2-2}$ steht ganz besonders bevorzugt für Dimethylamino oder Diethylamino.

$R^{12}$ und $R^{13}$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest, mit jeweils 1 bis 6 insbesondere mit 1 bis 4 Kohlenstoffatomen in den einzelnen geradkettigen oder verzweigten Alkylteilen.

Die Formamide der Formel (Va) und deren Derivate der Formel (Vb) sind allgemein bekannte Verbindungen der organischen Chemie.

Die zur Durchführung des erfindungsgemäßen Verfahrens (c) als Ausgangsstoffe benötigten Ketocarbonsäurederivate sind durch die Formel (VI) allgemein definiert.

In dieser Formel (VI) stehen $R^1$, $R^3$, $R^4$, $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

Die Ketocarbonsäurederivate der Formel (VI) sind neu und Gegenstand der Erfindung. Man erhält sie jedoch in Analogie zu bekannten Verfahren, indem man beispielsweise Oxalester der Formel (XXVI)

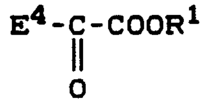

(XXVI)

in welcher

$R^1$ die oben angegebene Bedeutung hat und

$E^4$ für Alkoxy oder Halogen, insbesondere für Methoxy, Ethoxy oder Chlor steht,

mit Indolderivaten der Formel (XI)

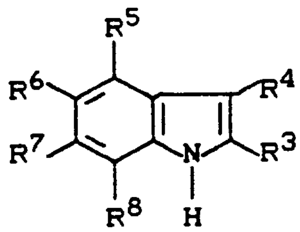

(XI)

in welcher

$R^3$, $R^4$, $R^5$, $R^6$, $R^7$ und $R^8$ die oben angegebene Bedeutung haben,

gegebenenfalls in Gegenwart eines Verdünnungsmittels, wie beispielsweise Dichlormethan oder Tetrahydrofuran und gegebenenfalls in Gegenwart einer Base, wie beispielsweise n-Butyllithium, Natriumhydrid, Kalium-t-butylat, Triethylamin oder Pyridin bei Temperaturen zwischen -80 °C und +80 °C umsetzt (vgl. DE-OS 3807232).

Die zur Durchführung des erfindungsgemäßen Verfahrens (c) weiterhin als Ausgangsstoffe benötigten metallorganischen Verbindungen sind durch die Formel (VII) allgemein definiert. In dieser Formel (VII) steht $R^{2-3}$ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J.Org.Chem. 33, 780 [1968]; J.Org.Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert. In dieser Formel (VIII) stehen $R^1$, $R^3$, $R^4$, $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy- oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (II),

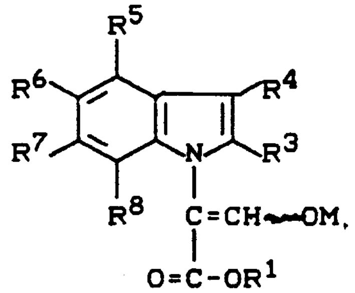

(II)

in welcher

M, $R^1$, $R^3$, $R^4$, $R^5$, $R^6$, $R^7$ und $R^8$ die oben angegebene Bedeutung haben,

mit Säurechloriden der Formel (XXVII)

$R^{20}$-Cl (XXVII)

in welcher

$R^{20}$ für einen Acyl- oder Sulfonylrest, insbesondere für einen Acetyl-, einen Methansulfonyl- oder einen p-Toluolsulfonylrest steht,

gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen von -20 °C bis +120 °C umsetzt.

Säurechloride der Formel (XXVII) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht $R^{2-3}$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen von - 30 °C bis + 120 °C, vorzugsweise bei Temperaturen von - 20 °C bis + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol,

vorzugsweise 1.0 bis 2.0 Mol an Reaktionshilfsmittel ein.

Dabei ist es auch möglich, die als Ausgangsverbindungen zur Durchführung des erfindungsgemäßen Verfahrens (a) benötigten 3-Hydroxyacrylsäureester oder deren Alkalimetallsalze der Formel (II) in einer vorgelagerten Reaktion direkt im Reaktionsgefäß herzustellen und direkt aus dem Reaktionsgemisch heraus ohne Isolierung mit dem Alkylierungsmittel der Formel (III) gemäß dem erfindungsgemäßen Verfahren (a) weiter umzusetzen ("Eintopfverfahren").

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen von - 20 °C bis + 200 °C, vorzugsweise bei Temperaturen von 0 °C bis 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein.

Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Toluol, Xylol, Petrolether, Hexan oder Cyclohexan oder Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen von -100 °C bis +100 °C, vorzugsweise bei Temperaturen von -80 °C bis +50 °C.

Zur Durchführung des erfindungsgemäßen Verfahrens (c) setzt man pro Mol an Ketocarbonsäurederivat der Formel (VI) im allgemeinen 1.0 bis 1.5 Mol, vorzugsweise 1.0 bis 1.2 Mol an metallorganischer Verbindung der Formel (VII) ein.

Die Reaktionsdurchführung, Aufarbeitung und Isolierung der Reaktionsprodukte erfolgt nach bekannten Verfahren (vgl. z.B. J.Org.Chem. 33, 780 [1968]; J.Org.Chem. 37, 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen von -20 °C bis 180 °C, vorzugsweise bei Temperaturen von 0 °C bis 150 °C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.

Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe der Formeln (I) und (IV) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide und Insektizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur protektiven Bekämpfung von Venturia-Arten an Äpfeln, Phytophthora-Arten an Tomaten und Cochliobolus sativus- und Pyrenophora teres-Arten an Gerste sowie zur protektiven Bekämpfung von Reiskrankheiten, wie beispielsweise gegen den Erreger der Reisfleckenkrankheit (Pyricularia oryzae), einsetzen.

Darüber hinaus zeigen die erfindungsgemäßen Wirkstoffe außerdem eine fungizide Wirkung gegen Plasmopara, Septoria nodorum, Cochliobulus sativus und Fusariosen in Getreide sowie gegen Uncinula necator in Reben, Venturia inaequalis, Erysiphe graminis, Botrytis und Pellicularia sowie eine breite und gute in vitro-Wirkung.

Desweiteren eignen sich die Wirkstoffe zur Bekämpfung von tierischen Schädlingen, vorzugsweise Arthropoden und Nematoden, ins besondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.

Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. und Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymanoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globedera ssp., Meloidogyne spp., Aphelenchoides spp., Longidorus spp., Xiphinema spp., Trichodorus spp..

Die erfindungsgsmäßen Wirkstoffe zeichnen sich durch eine hervorragende insektizide Wirksamkeit, insbesondere beim Einsatz gegen Käferlarven, wie z.B. Phaedon cochleariae, Plutella xylostella und Spodoptera frugiperda sowie gegen Blattläuse, wie z.B. Myzus persicae, aus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit

Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,1 bis 95 Gewichtsprozent Wirkstoff, vorzugsweise 0,5 bis 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1

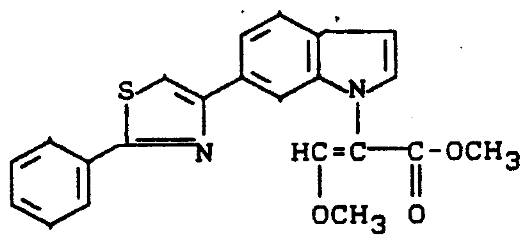

In eine Suspension von 1.20 g (40.00 mmol) Natriumhydrid (80%iges Gemisch mit Paraffin) in 10 ml Dimethylformamid läßt man unter Rühren und Kühlen bei einer Temperatur von 0 °C bis 5 °C eine Lösung von 6.10 g (17.51 mmol) [6-(2-Phenyl-thiazol-4-yl)-indol-1-yl]-essigsäuremethylester in ein Gemisch aus 10 ml Dimethylformamid und 20 ml Ameisensäuremethylester tropfen. Man rührt ca. 2 Stunden lang bei 0 °C nach und versetzt tropfenweise bei gleicher Temperatur und intensivem Rühren mit 6.00 g (47.57 mmol) Dimethylsulfat. Während 2 Stunden läßt man nun auf Raumtemperatur kommen, verrührt das Reaktionsgemisch mit überschüssiger wäßriger Natriumhydrogencarbonat-Lösung, extrahiert mit Essigsäureethylester, trocknet die vereinigten Extrakte über wasserfreiem Natriumsulfat, filtriert und engt ein. Der verbleibende Rückstand wird säulenchromatographisch an Silicagel gereinigt (Laufmittel: Dichlormethan/n-Hexan 2:1).

Man erhält 3-Methoxy-2-[6-(2-Phenyl-thiazol-4-yl)-indol-1-yl)-acrylsäuremethylester als Z-Isomeres vom Schmelzpunkt Fp: 145-146 °C, Ausbeute 2.20 g (32.1% der Theorie) und ein Z/E-Isomerengemisch (1:3), Ausbeute 1.50 g (21.9% der Theorie) vom Schmelzpunkt Fp: 150-154 °C.

Beispiel 2

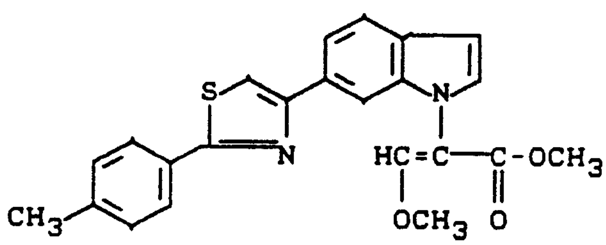

Auf analoge Weise zu Beispiel 1 erhält man 3-Methoxy-2-[6-[(4-Methyl-phenyl)-thiazol-4-yl]-indol-1-yl]-acrylsäuremethylester als Z-Isomeres: Ausbeute 2.90 g (42.6% der Theorie) vom Schmelzpunkt Fp: 145-146 °C und ein Z/E-Isomerengemisch (7:93): Ausbeute 0.90 g (13.2% der Theorie) vom Schmelzpunkt Fp: 154-155 °C.

Beispiel 3

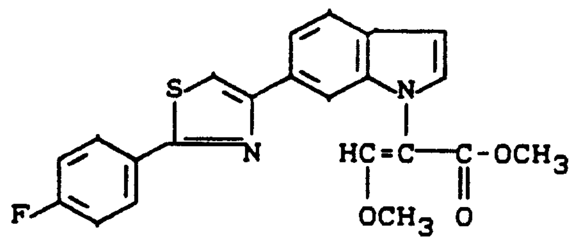

Auf analoge Weise zu Beispiel 1 und 2 erhält man 3-Methoxy-2-[6-[(4-Fluor-phenyl)-thiazol-4-yl]-indol-1-yl]-acrylsäuremethylester vom Schmelzpunkt Fp: 130-131 °C.

Analog zu den Herstellungsbeispielen 1 bis 3 und gemäß den allgemeinen Angaben zu den erfindungsgemäßen Verfahren erhält man die in Tabelle 2 aufgeführten heterocyclisch substituierten Acrylsäureester der allgemeinen Formel (I)

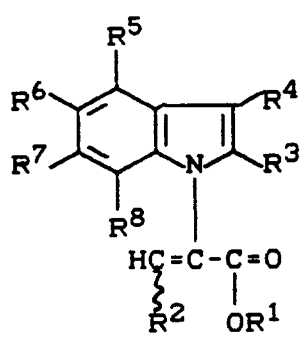

(I)

Tabelle 2

| Bsp. Nr. | $R^1$ | $R^2$ | $R^3$ | $R^4$ | $R^5$ | $R^6$ | $R^7$ | $R^8$ | physik. Konst. |
|---|---|---|---|---|---|---|---|---|---|
| 4 | $CH_3$ | $OCH_3$ | H | H | H | H | 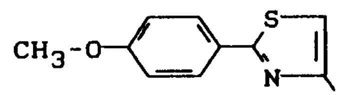 | H | F. 130° C<br>E/Z-Gemisch |
| 5 | $CH_3$ | $N(CH_3)_2$ | H | H | H | H | 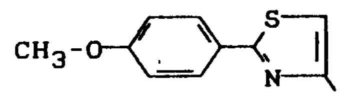 | H | F. 42-43° C |
| 6 | $CH_3$ | $OCH_3$ | H | H | H | H | 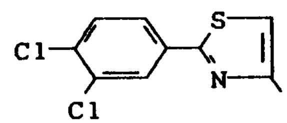 | H | $n_D^{20}$ 1,5937<br>E/Z= 85/15 |
| 7 | $CH_3$ | $OCH_3$ | H | H | H | H | 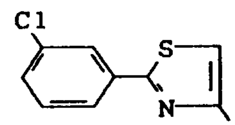 | H | F. 44-45° C<br>E/Z= 50/50 |
| 8 | $CH_3$ | $OCH_3$ | H | H | H | H | 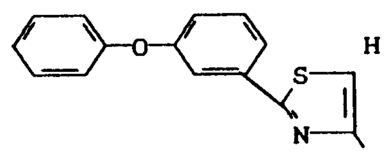 | H | $n_D^{20}$ 1,5863<br>E/Z= 60/40 |

Herstellung der Ausgangsverbindungen

Beispiel (IV-1)

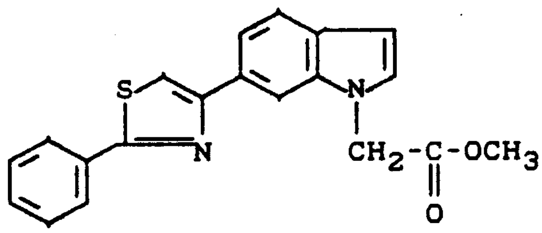

Ein Gemisch aus 15.00 g (54.28 mmol) 6-(2-Phenyl-thiazol-4-yl)indol, 15.30 g (100.02 mmol) Bromessigsäuremethylester, 20 g feingemörsertem Kaliumcarbonat und 120 ml Acetonitril wird 8 Stunden lang unter Rühren und Rückfluß erhitzt. Das Reaktionsende wird dünnschichtchromatographisch ermittelt. Zur Aufarbeitung läßt man abkühlen, verteilt den Inhalt des Reaktionsgefäßes zwischen Wasser und Essigsäureethylester, trocknet die organische Phase und engt ein. Der kristallisierende Rückstand wird mit Diisopropylether verrieben, abgesaugt und im Hochvakuum bei 50 °C getrocknet.

Man erhält 18.10 g (95.7% der Theorie) [6-(2-Phenylthiazol-4-yl)-indol-1-yl-essigsäuremethylester vom Schmelzpunkt 83 °C.

Beispiel (IV-2)

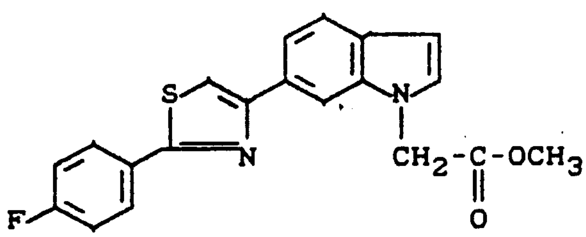

Auf analoge Weise zu Beispiel (IV-1) erhält man [6-[2-(4-Fluor-phenyl)-thiazol-4-yl]-indol-1-yl]-essigsäuremethylester.

Beispiel (IV-3)

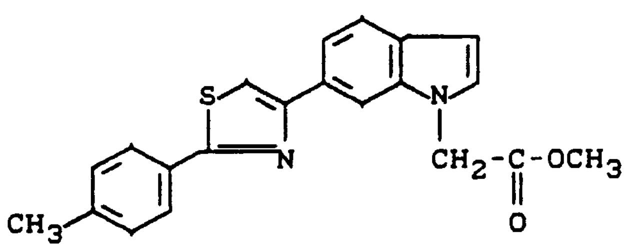

Auf analoge Weise zu Beispiel (IV-1) und (IV-2) erhält man [6-[2-(4-Methyl-phenyl)-thiazol-4-yl]-indol-1-yl]-essigsäuremethylester vom Schmelzpunkt Fp: 100-102 °C.

Analog zu den Herstellungsbeispielen (IV-1) bis (IV-3) und gemäß den allgemeinen Angaben zu den erfindungsgemäßen Verfahren erhält man die in Tabelle 3 aufgeführten substituierten Essigsäureester der Formel (IV)

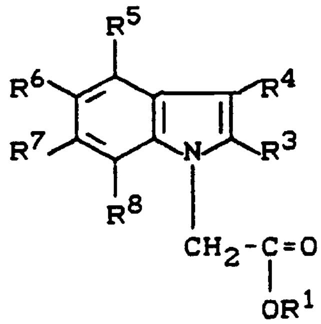

(IV)

<u>Tabelle 3</u>

| Bsp. Nr. | $R^1$ | $R^3$ | $R^4$ | $R^5$ | $R^6$ | $R^7$ | $R^8$ | physik. Konst. |
|---|---|---|---|---|---|---|---|---|
| (IV-4) | $CH_3$ | H | H | H | H | 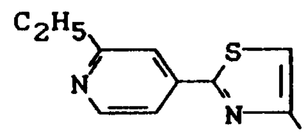 | H | F. 57-58° C |
| (IV-5) | $CH_3$ | H | H | H | H | 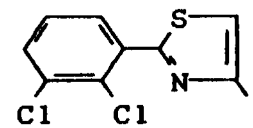 | H | $n_D^{20}$ 1,5955 |
| (IV-6) | $CH_3$ | H | H | H | H | 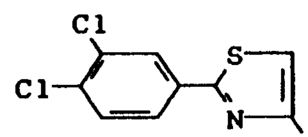 | H | F. 108-109° C |
| (IV-7) | $CH_3$ | H | H | H | H | 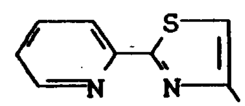 | H | $n_D^{20}$ 1,5978 |
| (IV-8) | $CH_3$ | H | H | H | H | 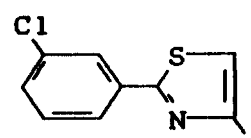 | H | F. 121-122° C |

**Tabelle 3** - Fortsetzung -

| Bsp. Nr. | $R^1$ | $R^3$ | $R^4$ | $R^5$ | $R^6$ | $R^7$ | $R^8$ | physik. Konst. |
|---|---|---|---|---|---|---|---|---|
| (IV-9) | $CH_3$ | H | H | H | H | 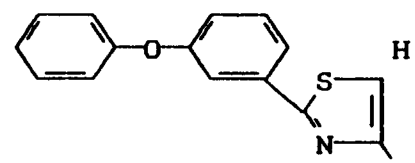 | H | F. 98-99° C |
| (IV-10) | $CH_3$ | H | H | H | H | 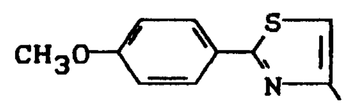 | H | F.106-107° C |
| (IV-11) | $CH_3$ | H | H | H | H | 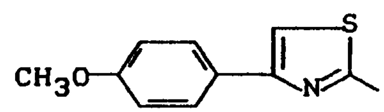 | H | F.108-109° C |

Beispiel (XI-1)

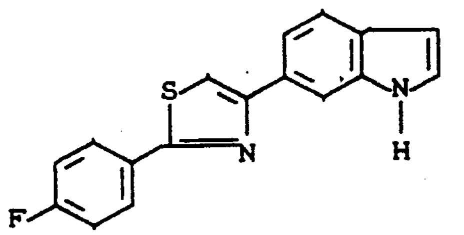

Eine Lösung des rohen β-Dimethylamino-4-[2-(4-fluor-phenyl)-thiazol-4-yl]-2-nitro-styrols in 200 ml Tetrahydrofuran wird unter Zusatz von 5 g Raney-Nickel bei einem Wasserstoffdruck von 50-60 bar bei 70 °C 4.5 Stunden hydriert. Zur Aufarbeitung filtriert man, engt ein und chromatographiert den Rückstand an Silicagel (Elutionsmittel Dichlormethan).

Man erhält 10.33 g (43.5% der Theorie) 6-[2-(4-Fluorphenyl)-thiazol-4-yl]-indol vom Schmelzpunkt Fp: 139-140 °C.

Beispiel (XI-2)

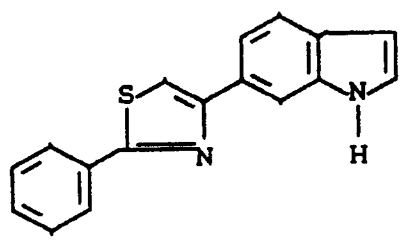

Auf analoge Weise zu Beispiel (XI-1) erhält man 6-(2-Phenyl-thiazol-4-yl)-indol vom Schmelzpunkt Fp: 159-161 °C.

Beispiel (XI-3)

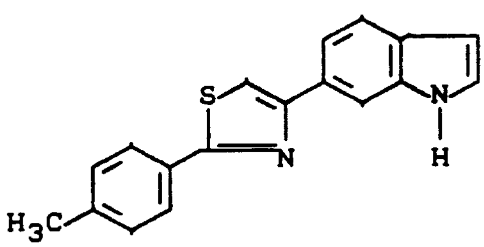

Auf analoge Weise zu Beispiel (XI-1) und (XI-2) erhält man 6-[2-(4-Methyl-phenyl)-thiazol-4-yl]-indol vom Schmelzpunkt Fp: 177 °C.

Analog zu den Herstellungbeispielen (XI-1) bis (XI-3) und gemäß den allgemeinen Angaben zu den erfindungsgemäßen Verfahren erhält man die in Tabelle 4 aufgeführten Indolderivate der allgemeinen Formel (XI)

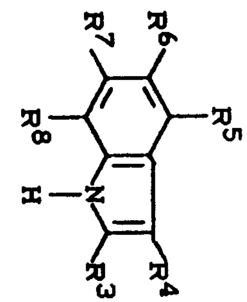

(XI)

Tabelle 4

| Bsp. Nr. | $R^3$ | $R^4$ | $R^5$ | $R^6$ | $R^7$ | $R^8$ | physik. Konst. |
|---|---|---|---|---|---|---|---|
| (XI-4) | H | H | H | H | 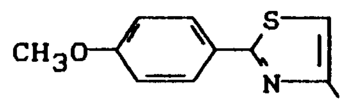 | H | F. 110-111° C |
| (XI-5) | H | H | H | H | 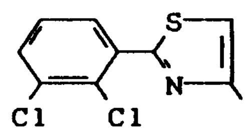 | H | |
| (XI-6) | H | H | H | H | 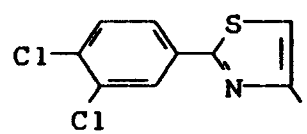 | H | F. 98-99° C |
| (XI-7) | H | H | H | H | 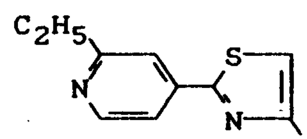 | H | F. 109-110° C |
| (XI-8) | H | H | H | H | 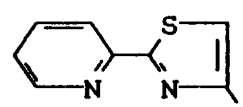 | H | F. 156° C |

**Tabelle 4** - Fortsetzung -

| Bsp. Nr. | $R^3$ | $R^4$ | $R^5$ | $R^6$ | $R^7$ | $R^8$ | physik. Konst. |
|---|---|---|---|---|---|---|---|
| (XI-9) | H | H | H | H | 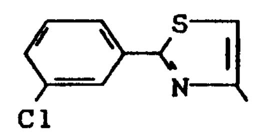 | H | F. 136° C |
| (XI-10) | H | H | H | H | 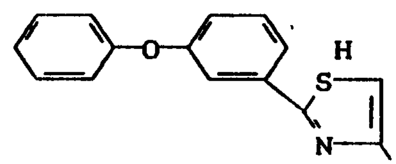 | H | F. 108-109° C |
| (XI-11) | H | H | H | H | 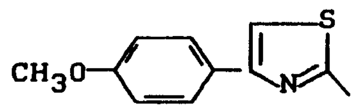 | H | F. 164-165° C |
| (XI-12) | H | H | H | H | 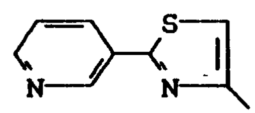 | H | MS: m/e 277 (M+) |

Beispiel (XV-1)

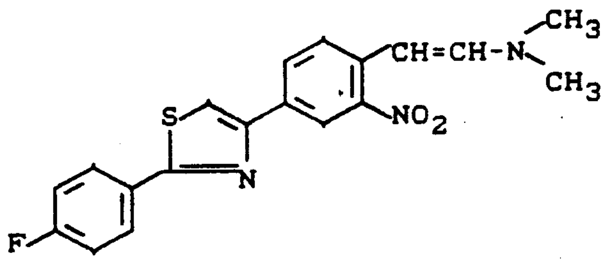

Ein Gemisch aus 25.38 g (50.76 mmol) 2-Methyl-5-[2-(4-fluor-phenyl)-thiazol-4-yl]-nitrobenzol, 84.00 g (704.87 mmol) Dimethylformamiddimethylacetal und 120 ml Dimethylformamid wird ca. 18 Stunden bis zur vollständigen Umsetzung am Rückfluß erhitzt. Das Reaktionsende wird dünnschichtchromatographisch ermittelt. Nach beendeter Reaktion trennt man die flüchtigen Anteile zunächst im Wasserstrahl-, dann im Ölpumpenvakuum ab. Das verbleibende schwarzrote Öl wird ohne weitere Reinigung in die nächste Reaktionsstufe eingesetzt.

Man erhält bei längerem Stehenlassen β-Dimethylamino-4-[2-(4-fluor-phenyl)-thiazol-4-yl]-2-nitrostyrol als kristallines Produkt vom Schmelzpunkt Fp: 137-138 °C.

Beispiel (XV-2)

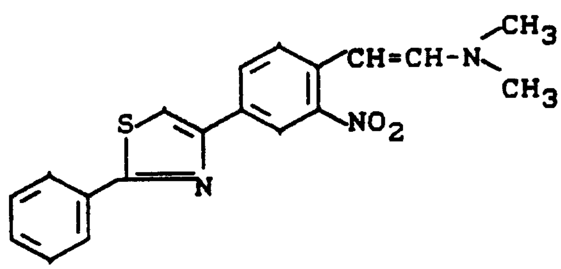

Auf analoge Weise zu Beispiel (XV-1) erhält man β-Dimethylamino-4-(2-phenyl-thiazol-4-yl)-2-nitro-styrol.

Beispiel (XV-3)

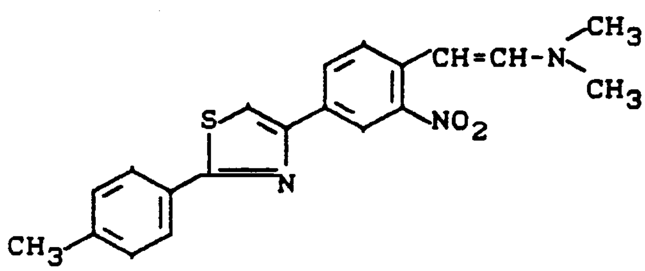

Auf analoge Weise zu Beispiel (XV-1) und Beispiel (XV-2) erhält man β-Dimethylamino-4-[2-(4-methylphenyl)-thiazol-4-yl]-2-nitro-styrol als kristallines Produkt vom Schmelzpunkt Fp: 124-125 °C.

Analog zu den Herstellungsbeispielen (XV-1) bis (XV-3) und gemäß den allgemeinen Angaben zu den erfindungsgemäßen Verfahren erhält man die in Tabelle 5 aufgeführten Verbindungen der allgemeinen Formel

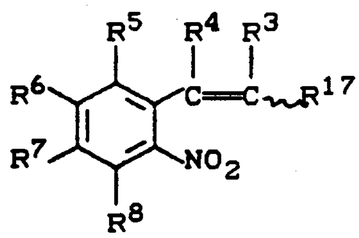

(XV)

Tabelle 5

| Bsp. Nr. | $R^3$ | $R^4$ | $R^5$ | $R^6$ | $R^7$ | $R^8$ | $R^{17}$ | physik. Konst. |
|---|---|---|---|---|---|---|---|---|
| (XV-4) | H | H | H | H | 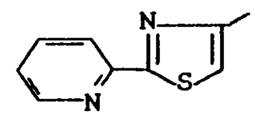 | H | $N(CH_3)_2$ | F. 130° C |
| (XV-5) | H | H | H | H | 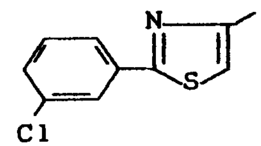 | H | $N(CH_3)_2$ | F. 114° C |
| (XV-6) | H | H | H | H | 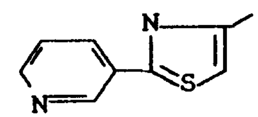 | H | $N(CH_3)_2$ | F. 147° C |
| (XV-7) | H | H | H | H | 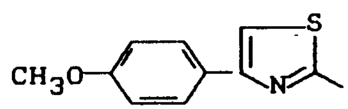 | H | $N(CH_3)_2$ | MS: m/e 381 ($M^+$) |
| (XV-8) | H | H | H | H | 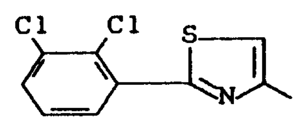 | H | $N(CH_3)_2$ | MS: m/e 419 ($M^+$-H) |

Tabelle 5 - Fortsetzung -

| Bsp. Nr. | $R^3$ | $R^4$ | $R^5$ | $R^6$ | $R^7$ | $R^8$ | $R^{17}$ | physik. Konst. |
|---|---|---|---|---|---|---|---|---|
| (XV-9) | H | H | H | H | 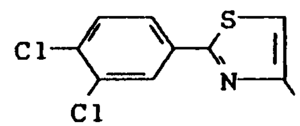 | H | $N(CH_3)_2$ | MS: m/e 419 ($M^+$-H) |
| (XV-10) | H | H | H | H | 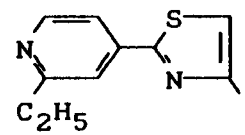 | H | $N(CH_3)_2$ | MS: m/e 380 ($M^+$) |
| (XV-11) | H | H | H | H | 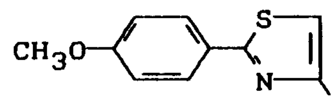 | H | $N(CH_3)_2$ | MS: m/e 381 ($M^+$) |
| (XV-12) | H | H | H | H | 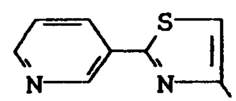 | H | $N(CH_3)_2$ | |
| (XV-13) | H | H | H | H | 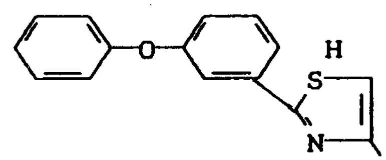 | H | $N(CH_3)_2$ | MS: m/e 443 ($M^+$) |

Beispiel (XIII-1)

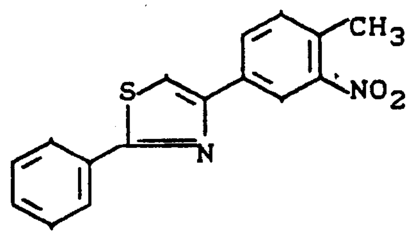

Ein Gemisch aus 23.79 g (92.2 mmol) 4-Methyl-3-nitrophenacylbromid, 18.61 g (135.6 mmol) Thiobenzamid und 350 $cm^3$ Ethanol wird ca. eine Stunde lang unter Rühren und Rückfluß erhitzt. Man läßt abkühlen und saugt die ausfallenden Kristalle ab.

Man erhält 26.46 g (96.8% der Theorie) 2-Methyl-5-(2-phenyl-thiazol-4-yl)-nitrobenzol vom Schmelzpunkt Fp: 97-98 °C.

Beispiel (XIII-2)

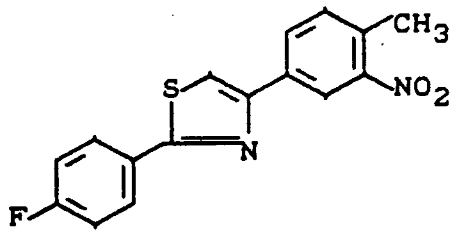

Auf analoge Weise zu Beispiel (XIII-1) erhält man 2-Methyl-5-[2-(4-fluor-phenyl)-thiazol-4-yl]-nitrobenzol vom Schmelzpunkt Fp: 124 °C.

Beispiel (XIII-3)

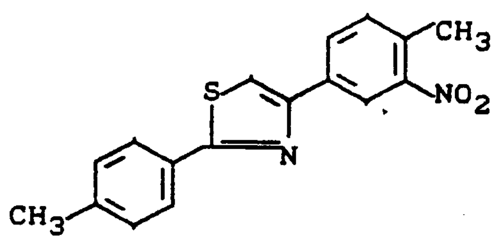

In analoger Weise zu Beispiel (XIII-1) und (XIII-2) erhält man 2-Methyl-5-[2-(4-methyl-phenyl)-thiazol-4-yl]-nitrobenzol vom Schmelzpunkt Fp: 104 °C.

Analog zu den Herstellungsbeispielen (XIII-1) - XIII-3) und gemäß den allgemeinen Angaben zu den erfindungsgemäßen Verfahren erhält man die in Tabelle 6 aufgeführten Verbindungen der allgemeinen Formel

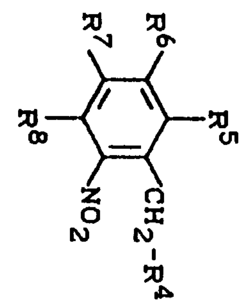

(XIII)

Tabelle 6

| Bsp. Nr. | $R^4$ | $R^5$ | $R^6$ | $R^7$ | $R^8$ | physik. Konst. |
|---|---|---|---|---|---|---|
| (XIII-4) | H | H | H | 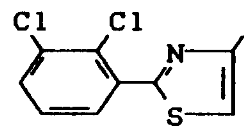 | H | F. 164° C |
| (XIII-5) | H | H | H | 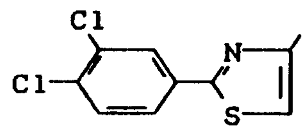 | H | F. 164° C |
| (XIII-6) | H | H | H | 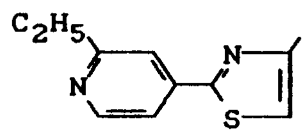 | H | F.>240° C |
| (XIII-7) | H | H | H | 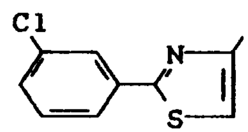 | H | F.>240° C |
| (XIII-8) | H | H | H | 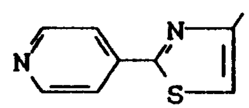 | H | F.>240° C |

Tabelle 6 - Fortsetzung -

| Bsp. Nr. | $R^4$ | $R^5$ | $R^6$ | $R^7$ | $R^8$ | physik. Konst. |
|---|---|---|---|---|---|---|
| (XIII-9) | H | H | H | 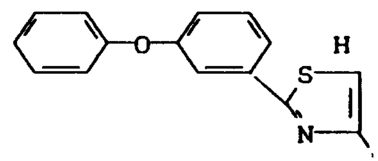 | H | F.106-107° C |
| (XIII-10) | H | H | H | 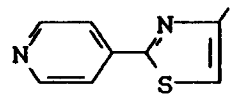 | H | F. 135° C |
| (XIII-11) | H | H | H | 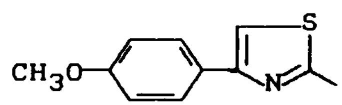 | H | F.108-109° C |
| (XIII-12) | H | H | H | 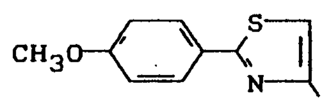 | H | MS: m/e 326 ($M^+$) |
| (XIII-13) | H | H | H | 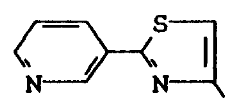 | H | $^1$H-NMR:* δ=2,56(S,$CH_3$) 7,63-9,45 (m, 8H) |

* Die $^1$H-NMR-Spektren wurden in Deuterochloroform ($CDCl_3$) mit Tetramethylsilan (TMS) als inneren Standard aufgenommen. Angegeben ist die chemische Verschiebung als δ-Wert in ppm.

## Anwendungsbeispiele

In den folgenden Anwendungsbeispielen wurde die nachstehend aufgeführte Verbindung als Vergleichssubstanz eingesetzt:

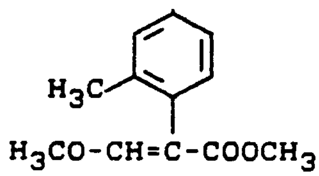

(A)

3-Methoxy-2-(2-methylphenyl)-acrylsäuremethylester (bekannt aus EP 178 816).

Beispiel A

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanezn mit einer wäßrigen Sporensuspension von Pyricularia oryzae inokuliert. Anschließend werden die Pflanzen in einem Gewächshaus bei 100% relativer Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen der Herstellungsbeispiele (1) (Z-Isomeres), (1) (Z/E-Isomerengemisch), (2) (Z-Isomeres), (2) (Z/E-Isomerengemisch), (IV-3), (IV-4), (IV-5), (IV-6) und (IV-10).

Beispiel B

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100% relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen (2) (Z-Isomeres) und (2) (Z/E-Isomerengemisch) gemäß den Herstellungsbeispielen.

Beispiel C

Pyrenophora teres-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100% relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen der Herstellungsbeispiele (2) (Z-Isomeres), (2) (Z/E-Isomerengemisch), (IV-1) und (IV-3).

Beispiel D

Phytophthora-Test (Tomate)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen der Herstellungsbeispiele (1) (Z/E-Isomerengemisch), (2) (Z-Isomeres) und (2) (Z/E-Isomerengemisch).

Beispiel E

Venturia-Test (Apfel)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100% relativer Luftfeuchtigkeit in einer Inkubationskabine.

Die Pflanzen werden dann im Gewächshaus bei 20 °C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen der Herstellungsbeispiele (1) (Z-Isomeres), (1) (Z/E-Isomerengemisch), (2) (Z-Isomeres) und (2) (Z/E-Isomerengemisch).

Beispiel F

Spodoptera-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat

mit emulgatorhaltigem Wasser auf die gewünschten Konzentrationen.

Sojabohnenpflanzen (Glycine soja) werden mit der Wirkstoffzubereitung der gewünschten Konzentration behandelt. In zehnfacher Wiederholung wird je ein Blatt der behandelten Pflanze in eine Plastikdose gelegt und mit je einer Larve ($L_2$) des Heerwurms (Spodoptera frugiperda) besetzt. Nach 3 Tagen füttert man je Dose mit einem weiteren Blatt der entsprechenden Pflanze nach. Am 7. Tag werden die Larven auf unbehandeltes Kunstfutter umgesetzt.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei diesem Test zeigen z.B. die folgenden Verbindungen der Herstellungsbeispiele überlegene Wirksamkeit gegenüber dem Stand der Technik: (1) (Z-Isomeres), (1) (Z/E-Isomerengemisch), (2) (Z-Isomeres) und (3).

Beispiel G

Plutella-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven ($L_2$) der Kohlschabe (Plutella xylostella) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei diesem Test zeigen z.B. die folgenden Verbindungen der Herstellungsbeispiele überlegene Wirksamkeit gegenüber dem Stand der Technik: (1) (Z-Isomeres), (1) (Z/E-Isomerengemisch), (2) (Z-Isomeres), (2) (Z/E-Isomerengemisch) und (3).

Beispiel H

Phaedon-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven ($L_2$) des Meerrettichkäfers (Phaedon cochleariae) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei diesem Test zeigen z.B. die folgenden Verbindungen der Herstellungsbeispiele überlegene Wirksamkeit gegenüber dem Stand der Technik: (1) (Z-Isomeres), (1) (Z/E-Isomerengemisch), (2) (Z-Isomeres), (2) (Z/E-Isomerengemisch) und (3).

Beispiel I

Myzus-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat

mit emulgatorhaltigem Wasser auf die gewünschten Konzentrationen.

Mit der gewünschten Konzentration der Wirkstoffzubereitung werden Kohlpflanzen (Brassica oleracea), welche mit der Grünen Pfirsichblattlaus (Myzus persicae) befallen sind, tropfnaß gespritzt.

Nach der gewünschten Zeit wird die Wirkung in % bestimmt. Dabei bedeutet 100%, daß alle Tiere abgetötet wurden; 0% bedeutet, daß keine Tiere abgetötet wurden.

Bei diesem Test zeigen z.B. die folgenden Verbindungen der Herstellungsbeispiele überlegene Wirksamkeit gegenüber dem Stand der Technik: (1) (Z/E-Isomerengemisch), (2) (Z-Isomeres) und (3).

**Patentansprüche**

**1.** Acrylsäureester der allgemeinen Formel (I)

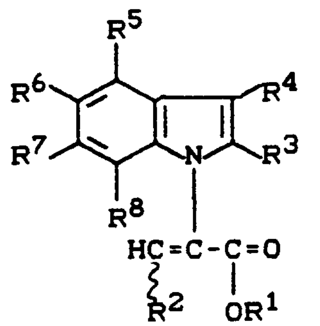

(I)

in welcher

| | |
|---|---|
| $R^1$ | für Alkyl oder für unsubstituiertes oder substituiertes Aralkyl steht, |
| $R^2$ | für Dialkylamino, Alkoxy, Alkylthio oder für jeweils unsubstituiertes oder substituiertes Aralkyloxy oder Arylalkylthio steht, |
| $R^3$ und $R^4$ | jeweils unabhängig voneinander für Wasserstoff, Cyano, Halogen oder Alkyl stehen, |
| $R^5$, $R^6$ und $R^8$ | unabhängig voneinander jeweils für Wasserstoff, Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils unsubstituiertes oder im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, -insbesondere Stickstoff, Sauerstoff und/oder Schwefel - im Heteroarylteil und gegebenenfalls 1 bis 4 Kohlenstoffatomen im geradkettigen oder verzweigten Alkylteil stehen und |
| $R^7$ | für eine der folgenden Gruppierungen steht |

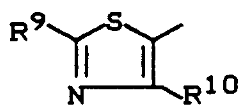 ; 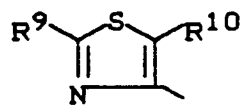 ; 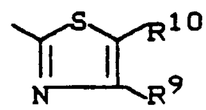

oder 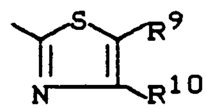

wobei

$R^9$ und $R^{10}$ jeweils unabhängig voneinander für Wasserstoff, Halogen, Cyano, Nitro, Alkyl, Alkoxy, Alkylthio, Halogenalkyl, Alkoxycarbonyl, Dialkylaminocarbonyl oder für jeweils unsubstituiertes oder substituiertes Aryl, Aralkyl, Aryloxy, Arylthio, Aralkyloxy, Aralkylthio, Hetaryl, Hetaryloxy oder Hetarylthio stehen.

**2.** Acrylsäureester der Formel (I) gemäß Anspruch 1, in welcher

$R^1$ für geradkettiges oder verzweigtes Alkyl mit 1 bis 6 Kohlenstoffatomen oder für Benzyl steht,

$R^2$ für Dialkylamino mit jeweils 1 bis 6 Kohlenstoffatomen in den einzelnen geradkettigen oder verzweigten Alkylteilen, jeweils geradkettiges oder verzweigtes Alkoxy oder Alkylthio mit 1 bis 6 Kohlenstoffatomen oder für jeweils unsubstituiertes oder einfach oder mehrfach, gleich oder verschieden substituiertes Benzyloxy oder Benzylthio steht, wobei als Phenylsubstituenten infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Cycloalkyl mit 3 bis 7 Kohlenstoffatomen,

$R^3$ und $R^4$ jeweils unabhängig voneinander für Wasserstoff, Cyano, Fluor, Chlor, Brom oder geradkettiges oder verzweigtes Alkyl mit 1 bis 6 Kohlenstoffatomen stehen,

$R^5$, $R^6$ und $R^8$ unabhängig voneinander für Wasserstoff, Fluor, Chlor, Brom, Methyl, Ethyl, n-oder i-Propyl, n-, i-, s- oder t-Butyl, Methoxy, Ethoxy, n-oder i-Propoxy, Methylendioxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, zweifach verknüpftes 1,3-Propandiyl oder 1,4-Butandiyl oder für jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Benzyloxy, Phenylthio oder Benzylthio stehen und

$R^7$ für eine der folgenden Gruppierungen steht

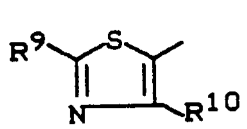 ; 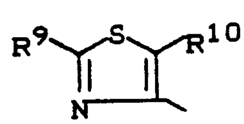 ; 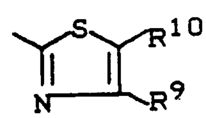

oder 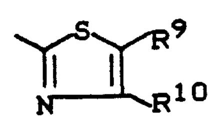

wobei

$R^9$ und $R^{10}$ jeweils unabhängig voneinander für Wasserstoff, Fluor, Chlor, Brom, Cyano, jeweils geradkettiges oder verzweigtes Alkyl, Alkoxy, Alkylthio oder Halogenalkyl

mit 1 bis 6 Kohlenstoffatomen und gegebenenfalls 1 bis 13 gleichen oder verschiedenen Halogenatomen steht, für geradkettiges oder verzweigtes Alkoxycarbonyl mit 1 bis 6 Kohlenstoffatomen im Alkoxyteil, jeweils in den einzelnen Alkylteilen geradkettiges oder verzweigtes, gleich oder verschieden substituiertes Dialkylaminocarbonyl mit 1 bis 4 Kohlenstoffatomen in jedem Alkylteil oder für jeweils unsubstituiertes oder einfach bis dreifach, gleich oder verschieden substituiertes Phenyl, Benzyl, Phenyloxy, Phenylthio, Benzyloxy oder Benzylthio steht oder für unsubstituiertes oder einfach oder zweifach, gleich oder verschieden substituiertes 5-oder 6-gliedriges Heteroaryl steht, das 1 bis 3 Heteroatome aus der Reihe Stickstoff, Sauerstoff oder Schwefel enthält, wobei als Substituenten jeweils Halogen, Alkyl, Alkoxy, Alkylthio, Halogenalkyl, Halogenalkyloxy, Halogenalkylthio mit jeweils 1 bis 4 Kohlenstoffatomen und gegebenenfalls 1 bis 9 gleichen oder verschiedenen Halogenatomen, Dialkylamino oder Dialkylaminocarbonyl mit jeweils 1 bis 4 Kohlenstoffatomen in den jeweils geradkettigen oder verzweigten Alkylteilen, 1,3-Propandiyl, 1,4-Butandiyl oder unsubstituiertes oder einfach bis zweifach, gleich oder verschieden substituiertes Phenyl oder Benzyl genannt seien, wobei als Substituenten jeweils Halogen oder Phenyloxy infrage kommen.

**3.** Acrylsäureester der Formel (I) gemäß Anspruch 1, in welcher

$R^1$ für geradkettiges oder verzweigtes Alkyl mit 1 bis 4 Kohlenstoffatomen steht,

$R^2$ für Dialkylamino mit jeweils 1 bis 4 Kohlenstoffatomen in den einzelnen geradkettigen oder verzweigten Alkylteilen, jeweils geradkettiges oder verzweigtes Alkoxy oder Alkylthio mit 1 bis 4 Kohlenstoffatomen oder für Benzyloxy oder Benzylthio steht,

$R^3$ und $R^4$ gleich oder verschieden sind und für Wasserstoff, Cyano, Fluor, Chlor, Brom, Methyl, Ethyl, n-oder i-Propyl, n-, i-, s- oder t-Butyl stehen,

$R^5$ und $R^6$ gleich oder verschieden sind und für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl oder Cyclohexyl stehen oder gemeinsam für eine Methylendioxy-, 1,3-Propandiyl- oder 1,4-Butandiylgruppierung stehen,

$R^7$ für eine der folgenden Gruppierungen steht,

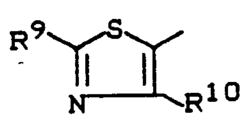 ; 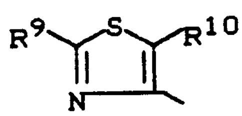 ; 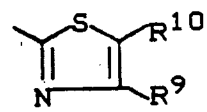

oder 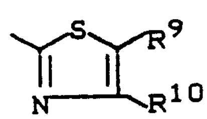

wobei

$R^9$ für Wasserstoff, geradkettiges oder verzweigtes Alkyl mit 1 bis 6 Kohlenstoffatomen, jeweils unsubstituiertes oder einfach bis dreifach, gleich oder verschieden substituiertes Phenyl, Benzyl oder für einfach oder zweifach, gleich oder verschieden substituiertes 5- oder 6-gliedriges Heteroaryl steht, das 1 bis 3 Heteroatome aus der Reihe Stickstoff, Sauerstoff oder Schwefel enthält, wobei als Substituenten jeweils Halogen, Methyl, Ethyl, n-oder i-Propyl, n-, i-, s- oder t-Butyl, Methoxy, Ethoxy, n- oder i-Propoxy, n-, i-, s-oder t-Butoxy, Methylthio, Ethylthio, n-oder i-Propylthio, n-, i-, s-oder t-Butylthio, Halogenalkyl, Halogenalkoxy oder Halogenalkylthio mit jeweils 1 oder 2 Kohlenstoffatomen und 1 bis 5 gleichen oder verschiedenen Fluor oder Chloratomen, Dialkylamino oder Dialkylaminocarbonyl mit 1 oder 2 Kohlenstoffatomen in den einzelnen Alkylteilen, unsubstituiertes oder einfach bis zweifach, gleich oder verschieden substituiertes Phenyl, Phenoxy oder Benzyl genannt seien, wobei als Substituenten jeweils Fluor, Chlor oder Phenoxy infrage kommen und

$R^{10}$ für Wasserstoff, geradkettiges oder verzweigtes Alkyl mit 1 bis 4 Kohlenstoffatomen, Chlor, Brom, Cyano, Methoxycarbonyl, Ethoxycarbonyl, Dimethylaminocarbonyl, Diethylaminocarbonyl, Methoxy, Ethoxy, Methylthio oder Ethylthio steht und

$R^8$ für Wasserstoff, Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl oder Ethoxycarbonyl steht.

**4.** Acrylsäureester der Formel (I) gemäß Anspruch 1, in welcher

$R^1$ für Methyl oder Ethyl steht,

$R^2$ für Dimethylamino, Diethylamino, Methoxy, Ethoxy, Methylthio, Ethylthio, Benzyloxy oder Benzylthio steht,

$R^3$ für Wasserstoff, Chlor oder Methyl steht,

$R^4$ für Wasserstoff, Chlor oder Methyl steht,

$R^5$ für Wasserstoff, Fluor, Chlor, Brom, Methyl, Ethyl, Trifluormethyl, Methoxycarbonyl oder Ethoxycarbonyl steht,

$R^6$ für Wasserstoff, Fluor, Chlor, Brom, Methyl, Ethyl, Methoxy, Ethoxy, Methylthio, Trifluormethyl, Methoxycarbonyl oder Ethoxycarbonyl steht oder

$R^5$ und $R^6$ gemeinsam für eine Methylendioxy-, 1,3-Propandiyl- oder 1,4-Butandiylgruppierung stehen,

$R^7$ für eine der folgenden Gruppierungen steht

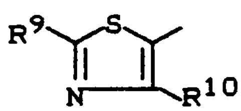 ; 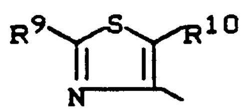 ; 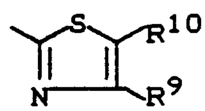

oder 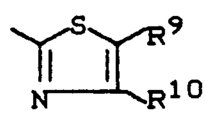

wobei

$R^9$ für Wasserstoff, Methyl, Ethyl, n- oder i-Propyl, n-, s-, i-Butyl, n- oder i-Pentyl, n-Hexyl, Benzyl, o-, m- oder p-Chlorbenzyl, o-, m- oder p-Methylbenzyl, Phenyl oder jeweils einfach bis dreifach, gleich oder verschieden substituiertes Phenyl oder für einfach oder zweifach, gleich oder verschieden substituiertes 5- oder 6-gliedriges Heteroaryl steht, das 1 oder 2 Heteroatome aus der Reihe Stickstoff, Sauerstoff oder Schwefel enthält, wobei

als Substituenten jeweils Fluor, Chlor, Methyl, Ethyl, t-Butyl, Methoxy, Ethoxy, Methylthio, Trifluormethyl, Trifluormethoxy, Trifluormethylthio, Phenyl, p-Chlorphenyl, m- oder p-Phenoxyphenyl oder Benzyl infrage kommen und

$R^{10}$ für Wasserstoff, Methyl, Ethyl, Chlor, Brom, Methoxycarbonyl oder Ethoxycarbonyl steht, oder

$R^8$ für Wasserstoff, Methyl oder Ethyl steht.

**5.** Verfahren zur Herstellung von Acrylsäureestern der allgemeinen Formel (I)

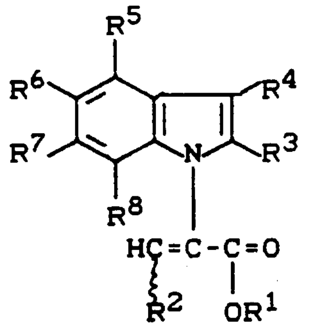

(I)

in welcher

| | |
|---|---|
| $R^1$ | für Alkyl oder für unsubstituiertes oder substituiertes Aralkyl steht, |
| $R^2$ | für Dialkylamino, Alkoxy, Alkylthio oder für jeweils unsubstituiertes oder substituiertes Aralkyloxy oder Arylalkylthio steht, |
| $R^3$ und $R^4$ | jeweils unabhängig voneinander für Wasserstoff, Cyano, Halogen oder Alkyl stehen, |
| $R^5$, $R^6$ und $R^8$ | unabhängig voneinander jeweils für Wasserstoff, Halogen, Cyano, Nitro, jeweils geradkettiges oder verzweigtes Alkyl, Alkoxy oder Alkylthio mit jeweils 1 bis 4 Kohlenstoffatomen, Alkylidendioxy mit 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils unsubstituiertes oder im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Arylthio, Aralkyloxy oder Aralkylthio mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl, Heteroaryloxy, Heteroarylthio oder Heteroaryl mit jeweils 2 bis 9 Kohlenstoffatomen und 1 bis 4 gleichen oder verschiedenen Heteroatomen, -insbesondere Stickstoff, Sauerstoff und/oder Schwefel - im Heteroarylteil und gegebenenfalls 1 bis 4 Kohlenstoffatomen im geradkettigen oder verzweigten Alkylteil stehen und |
| $R^7$ | für eine der folgenden Gruppierungen steht |

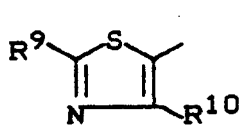 ; 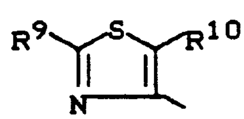 ; 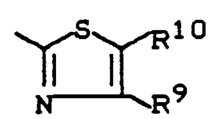

oder 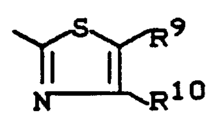

wobei

| | |
|---|---|
| $R^9$ und $R^{10}$ | jeweils unabhängig voneinander für Wasserstoff, Halogen, Cyano, Nitro, Alkyl, Alkoxy, Alkylthio, Halogenalkyl, Alkoxycarbonyl, Dialkylaminocarbonyl oder für jeweils onsubstituiertes oder substituiertes Aryl, Aralkyl, Aryloxy, Arylthio, Aralkyloxy, Aralkylthio, Hetaryl, Hetaryloxy oder Hetarylthio stehen, |

dadurch gekennzeichnet, daß man

a) substituierte Acrylsäureester der allgemeinen Formel (Ia)

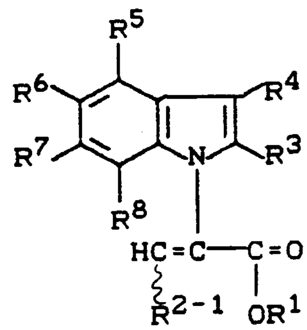

(Ia)

in welcher

| | |
|---|---|
| $R^1$, $R^3$, $R^4$, $R^5$, $R^6$, $R^7$ und $R^8$ | die oben angegebene Bedeutung haben und |
| $R^{2-1}$ | für Alkoxy oder unsubstituiertes oder substituiertes Aralkyloxy steht, |

erhält,

wenn man Hydroxyacrylsäureester oder deren Alkalimetallsalze der Formel (II)

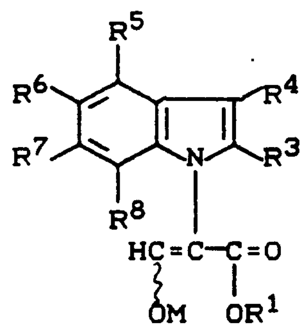

(II)

in welcher

| | |
|---|---|
| M | für Wasserstoff oder für ein Alkalimetallkation steht und |
| $R^1$, $R^3$, $R^4$, $R^5$, $R^6$, $R^7$ und $R^8$ | die oben angegebene Bedeutung haben, |

mit Alkylierungsmitteln der Formel (III)

$R^{11}$-$E^1$ (III)

in welcher

| | |
|---|---|
| $R^{11}$ | für Alkyl oder unsubstituiertes oder substituiertes Aralkyl steht und |
| $E^1$ | für eine elektronenanziehende Abgangsgruppe steht, |

gegebenenfalls in Gegenwart eines Verdünnungsmittels und gegebenenfalls in Gegenwart eines Reaktionshilfsmittels umsetzt;

b) substituierte Acrylsäureester der allgemeinen Formel (Ib)

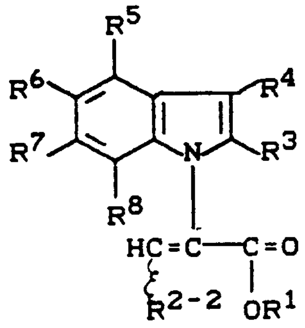

(Ib)

in welcher

$R^1$, $R^3$, $R^4$, $R^5$, $R^6$, $R^7$ und $R^8$ die oben angegebene Bedeutung haben und

$R^{2-2}$ für Dialkylamino steht, erhält,

wenn man substituierte Essigsäureester der Formel (IV)

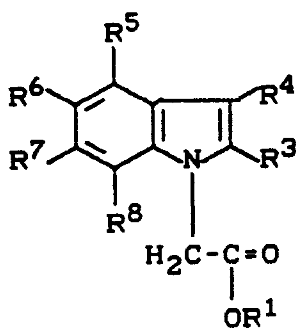

(IV)

in welcher

$R^1$, $R^3$, $R^4$, $R^5$, $R^6$, $R^7$ und $R^8$ die oben angegebene Bedeutung haben,

mit Formamiden der Formel (Va)

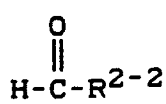

(Va)

in welcher

$R^{2-2}$ die oben angegebene Bedeutung hat,

oder mit Formamid-Derivaten der Formel (Vb)

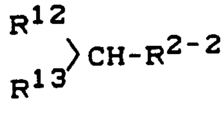

(Vb)

in welcher

$R^{12}$ und $R^{13}$ unabhängig voneinander für Alkoxy oder Dialkylamino stehen und

$R^{2-2}$ die oben angegebene Bedeutung hat,

gegebenenfalls in Gegenwart eines Verdünnungsmittels umsetzt;

c) substituierte Acrylsäureester der Formel (Ic)

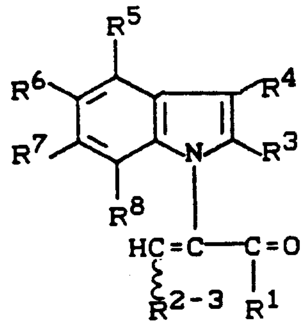

(Ic)

in welcher

$R^1$, $R^3$, $R^4$, $R^5$, $R^6$, $R^7$ und $R^8$ die oben angegebene Bedeutung haben und

$R^{2-3}$ für Alkylthio oder unsubstituiertes oder substituiertes Aralkylthio steht,

erhält,

wenn man Ketocarbonsäurederivate der Formel (VI)

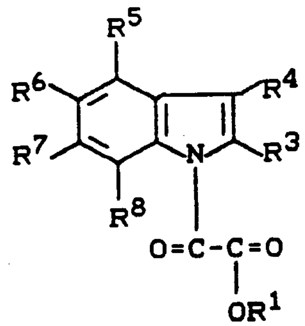

(VI)

in welcher

$R^1$, $R^3$, $R^4$, $R^5$, $R^6$, $R^7$ und $R^8$ die oben angegebene Bedeutung haben,

mit metallorganischen Verbindungen der Formel (VII)

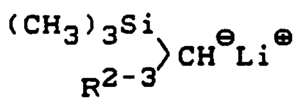

(VII)

in welcher

$R^{2-3}$ die oben angegebene Bedeutung hat,

gegebenenfalls in Gegenwart eines Verdünnungsmittels umsetzt;

d) substituierte Acrylsäureester der Formel (Ic) erhält,

wenn man substituierte Acrylsäureester der Formel (VIII)

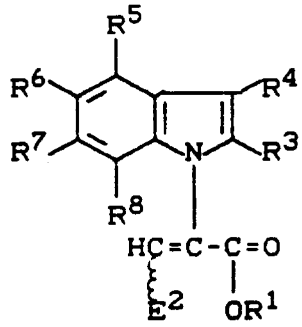

(VIII)

in welcher

$R^1$, $R^3$, $R^4$, $R^5$, $R^6$, $R^7$ und $R^8$ die oben angegebene Bedeutung haben und
$E^2$ für eine elektronenanziehende Abgangsgruppe steht,

mit Thiolen der Formel (IX)

$R^{2-3}$-H (IX)

in welcher

$R^{2-3}$ die oben angegebene Bedeutung hat,

gegebenenfalls in Gegenwart eines Verdünnungsmittels und gegebenenfalls in Gegenwart eines Reaktionshilfsmittels umsetzt.

**6.** Schädlingsbekämpfungsmittel, gekennzeichnet durch einen Gehalt an mindestens einem Acrylsäureester der Formel (I) nach den Ansprüchen 1 bis 5.

**7.** Verwendung von Acrylsäureester der Formel (I) nach den Ansprüchen 1 bis 5 zur Bekämpfung von Schädlingen.

**8.** Verfahren zur Bekämpfung von Schädlingen, dadurch gekennzeichnet, daß man Acrylsäureester der Formel (I) nach den Ansprüchen 1 bis 5 auf Schädlinge und/oder ihren Lebensraum einwirken läßt.

**9.** Verfahren zur Herstellung von Schädlingsbekämpfungsmitteln, dadurch gekennzeichnet, daß man Acrylsäureester der Formel (I) nach den Ansprüchen 1 bis 5 mit Streckmitteln und/oder oberflächenaktiven Mitteln vermischt.

**10.** Hydroxyacrylsäureester oder deren Alkalimetallsalze der Formel (II)

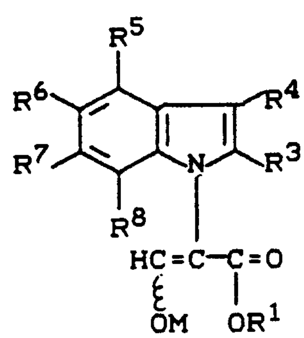

(II)

in welcher

M für Wasserstoff oder für ein Alkalimetallkation steht und
$R^1$, $R^3$, $R^4$, $R^5$, $R^6$, $R^7$ und $R^8$ die in Anspruch 1 angegebene Bedeutung haben.

**11.** Verfahren zur Herstellung von Hydroxyacrylsäureestern der Formel (II)

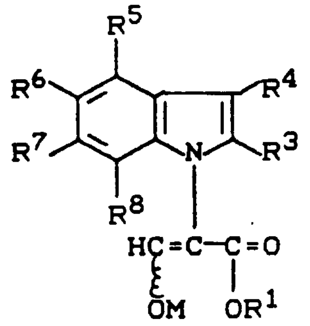

(II)

in welcher
M, $R^1$ und $R^3$ bis $R^8$ die in Anspruch 10 angegebene Bedeutung haben, dadurch gekennzeichnet, daß man
substituierte Essigsäureester der Formel (IV),

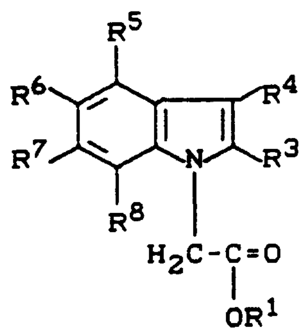

(IV)

in welcher
$R^1$, $R^3$, $R^4$, $R^5$, $R^6$, $R^7$ und $R^8$ die in Anspruch 10 angegebene Bedeutung haben,
mit Ameisensäureestern der Formel (X),

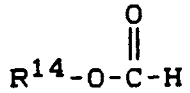

(X)

in welcher
$R^{14}$ für Alkyl steht
gegebenenfalls in Gegenwart eines Verdünnungsmittels und gegebenenfalls in Gegenwart eines basischen Reaktionshilfsmittels bei Temperaturen von -20 °C bis +50 °C umsetzt.

**12.** Essigsäureester der Formel (IV)

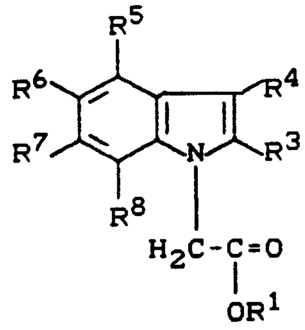

(IV)

in welcher
$R^1$, $R^3$, $R^4$, $R^5$, $R^6$, $R^7$ und $R^8$ die in Anspruch 1 angegebene Bedeutung haben.

**13.** Verfahren zur Herstellung von Essigsäureestern der Formel (IV),

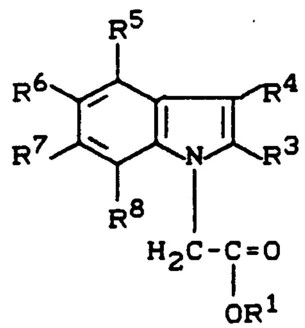

(IV)

in welcher $R^1$ bis $R^8$ die in Anspruch 1 angegebene Bedeutung haben, dadurch gekennzeichnet, daß man Indolderivate der allgemeinen Formel (XI)

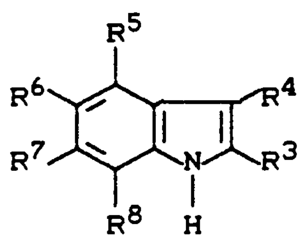

(XI)

in welcher
$R^3$, $R^4$, $R^5$, $R^6$, $R^7$ und $R^8$ die in Anspruch 1 angegebene Bedeutung haben,
mit Essigsäurederivaten der allgemeinen Formel (XII)

$E^3$-$CH_2$-$COOR^1$ (XII)

in welcher
$R^1$ die oben angegebene Bedeutung hat und
$E^3$ für eine elektronenanziehende Abgangsgruppe, bevorzugt für Halogen, insbesondere für Chlor oder Brom, steht,
gegebenenfalls in Gegenwart eines Verdünnungsmittels und gegebenenfalls in Gegenwart eines basischen Hilfsmittels bei Temperaturen zwischen -20 °C und +100 °C umsetzt.

**14.** Indolderivate der Formel (XI)

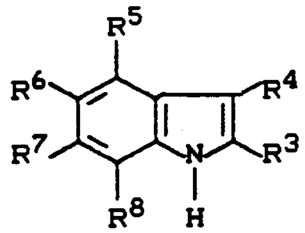

(XI),

in welcher
$R^3$ bis $R^8$ die in Anspruch 1 angegebene Bedeutung haben.

**15.** Verfahren zur Herstellung von Indolderivaten der Formel (XI)

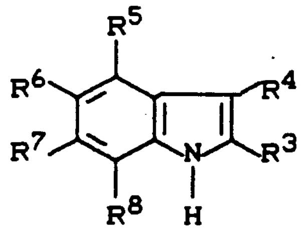

(XI),

in welcher
$R^3$ bis $R^8$ die in Anspruch 1 angegebene Bedeutung haben,
dadurch gekennzeichnet, daß man Nitrobenzol-Derivate der Formel (XIII)

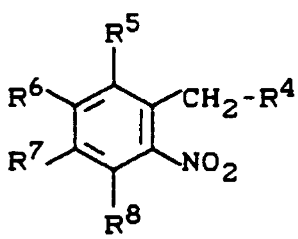

(XIII),

in welcher
$R^4$, $R^5$, $R^6$, $R^7$ und $R^8$ die in Anspruch 1 angegebene Bedeutung haben,
mit Verbindungen der Formel (XIV)

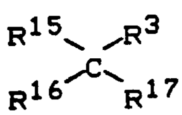

(XIV),

in welcher

$R^3$ die in Anspruch 1 angegebene Bedeutung hat,
$R^{15}$ für Alkoxy oder Dialkylamino steht,
$R^{16}$ für Alkoxy oder Dialkylamino steht und
$R^{17}$ für Dialkylamino steht,

gegebenenfalls in Gegenwart eines Verdünnungsmittels bei Temperaturen zwischen 25 °C und 200 °C

und gegebenenfalls unter einem Druck von 1 bis 100 bar zu den Verbindungen der allgemeinen Formel (XV)

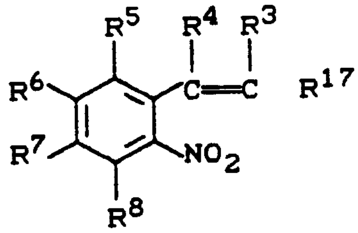

(XV),

in welcher
$R^3$, $R^4$, $R^5$, $R^6$, $R^7$, $R^8$ und $R^{17}$ die in Anspruch 1 angegebene Bedeutung haben,
umsetzt und die so erhaltenen Verbindungen der Formel (XV), gegebenenfalls nach ihrer Isolierung und/oder Reinigung, gegebenenfalls in Gegenwart eines Verdünnungsmittels, gegebenenfalls in Gegenwart eines Inertgases mit üblichen Reduktionsmitteln, in Gegenwart eines geeigneten Katalysators und einem Druck zwischen 1 und 200 bar, bei Temperaturen zwischen -20 °C und +200 °C cyclisiert.

**16.** Ketocarbonsäurederivate der Formel (VI)

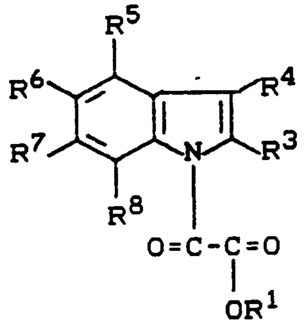

(VI),

in welcher
$R^1$, $R^3$, $R^4$, $R^5$, $R^6$, $R^7$ und $R^8$ die in Anspruch 1 angegebene Bedeutung haben.

**17.** Verfahren zur Herstellung von Ketocarbonsäurederivaten der Formel (VI)

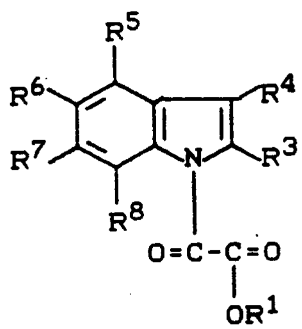

(VI),

in welcher
$R^1$ bis $R^8$ die in Anspruch 1 angegebene Bedeutung haben,
dadurch gekennzeichnet, daß man Oxalester der Formel (XXVI)

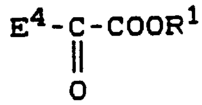 (XXVI),

in welcher

$R^1$ die oben angegebene Bedeutung hat und

$E^4$ für Alkoxy oder Halogen, insbesondere für Methoxy, Ethoxy oder Chlor steht,

mit Indolderivaten der Formel (XI)

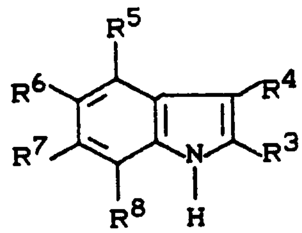 (XI),

in welcher

$R^3$, $R^4$, $R^5$, $R^6$, $R^7$ und $R^8$ die oben angegebene Bedeutung haben,

gegebenenfalls in Gegenwart eines Verdünnungsmittels und gegebenenfalls in Gegenwart einer Base bei Temperaturen zwischen -80 °C und +80 °C umsetzt.

**18.** Schädlingsbekämpfungsmittel, gekennzeichnet durch einen Gehalt an mindestens einem Essigsäureester der Formel (IV) nach den Ansprüchen 12 und 13.

**19.** Verwendung von Essigsäureestern der Formel (IV) nach den Ansprüchen 12 und 13 zur Bekämpfung von Schädlingen.

**20.** Verfahren zur Bekämpfung von Schädlingen, dadurch gekennzeichnet, daß man Essigsäurester der Formel (IV) nach den Ansprüchen 12 und 13 auf Schädlinge und/oder ihren Lebensraum einwirken läßt.

**21.** Verfahren zur Herstellung von Schädlingsbekämpfungsmitteln, dadurch gekennzeichnet, daß man Essigsäurester der Formel (IV) nach den Ansprüchen 12 und 13 mit Streckmitteln und/oder oberflächenaktiven Mitteln vermischt.

**22.** Verwendung der Verbindungen der Formeln (II), (IV), (XI) und (VI) nach den Ansprüchen 10 bis 17 als Zwischenprodukte für die Herstellung von Verbindungen der Formel (I) nach den Ansprüchen 1 bis 5.

## Claims

**1.** Acrylic esters of the general formula (I)

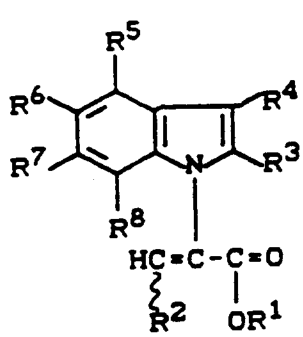 (I)

in which

$R^1$ represents alkyl, or represents unsubstituted or substituted aralkyl,

$R^2$ represents dialkylamino, alkoxy or alkylthio, or represents in each case unsubstituted or substituted aralkyloxy or arylalkylthio,

$R^3$ and $R^4$ in each case independently of one another represent hydrogen, cyano, halogen or alkyl,

$R^5$, $R^6$ and $R^8$ independently of one another in each case represent hydrogen, halogen, cyano, nitro, in each case straight-chain or branched alkyl, alkoxy or alkylthio, each of which has 1 to 4 carbon atoms, or represent alkylidenedioxy having 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, cycloalkyl having 3 to 7 carbon atoms, divalent alkanediyl having 3 to 5 carbon atoms, or aryl, aralkyl, aryloxy, arylthio, aralkyloxy or aralkylthio, each of which has 6 to 10 carbon atoms in the aryl moiety and where appropriate 1 to 4 carbon atoms in the straight-chain or branched alkyl moiety and each of which is unsubstituted or monosubstituted or polysubstituted in the aryl moiety by identical or different substitutents from the series comprising halogen, alkyl, alkoxy, alkylthio, halogenoalkyl, halogenoalkoxy or halogenoalkylthio, each of which has 1 to 4 carbon atoms and where appropriate 1 to 9 identical or different halogen atoms, or represent heteroarylalkyl, heteroaryloxy, heteroarylthio or heteroaryl, each of which has 2 to 9 carbon atoms and 1 to 4 identical or different hetero atoms - in particular nitrogen, oxygen and/or sulphur - in the heteroaryl moiety and where appropriate 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and where appropriate 1 to 9 identical or different halogen atoms, and

$R^7$ represents one of the following groups

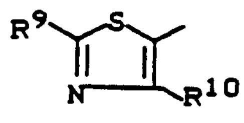 ; 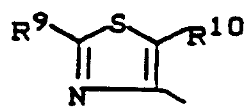 ; 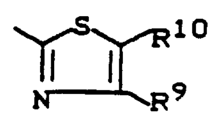

or

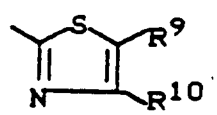

where

$R^9$ and $R^{10}$ in each case independently of one another represent hydrogen, halogen, cyano, nitro, alkyl, alkoxy, alkylthio, halogenoalkyl, alkoxycarbonyl or dialkylaminocarbonyl, or represent in each case unsubstituted or substituted aryl, aralkyl, aryloxy, arylthio, aralkyloxy, aralkylthio,
hetaryl, hetaryloxy or hetarylthio.

**2.** Acrylic esters of the formula (I) according to Claim 1, in which

$R^1$ represents straight-chain or branched alkyl having 1 to 6 carbon atoms, or represents benzyl,

$R^2$ represents dialkylamino having in each case 1 to 6 carbon atoms in the individual straight-chain or branched alkyl moieties, in each case straight-chain or branched alkoxy or alkylthio having 1 to 6 carbon atoms, or represents benzyloxy or benzylthio, each of which is unsubstituted or monosubstituted or polysubstituted by identical or different substitutents, suitable phenyl substituents being: halogen,

in each case straight-chain or branched alkyl, alkoxy or alkylthio each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, or cycloalkyl having 3 to 7 carbon atoms,

$R^3$ and $R^4$ in each case independently of one another represent hydrogen, cyano, fluorine, chlorine, bromine or straight-chain or branched alkyl having 1 to 6 carbon atoms,

$R^5$, $R^6$ and $R^8$ independently of one another represent hydrogen, fluorine, chlorine, bromine, methyl, ethyl, n- or i-propyl, n-, i-, s- or t-butyl, methoxy, ethoxy, n- or i-propoxy, methylenedioxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoxyiminoethyl, cyclopentyl, cyclohexyl, divalent 1,3-propanediyl or 1,4-butanediyl, or represents phenyl, benzyl, phenoxy, benzyloxy, phenylthio or benzylthio, each of which is optionally monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and/or trifluoromethylthio, and

$R^7$ represents one of the following groups

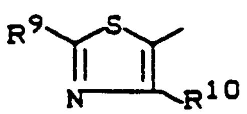 ; 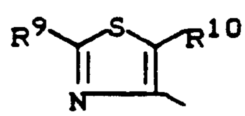 ; 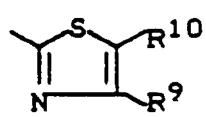

or 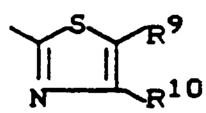

where

$R^9$ and $R^{10}$ in each case independently of one another represent hydrogen, fluorine, chlorine, bromine, cyano, in each case straight-chain or branched alkyl, alkoxy, alkylthio or halogenoalkyl having 1 to 6 carbon atoms and where appropriate 1 to 13 identical or different halogen atoms, or represent straight-chain or branched alkoxycarbonyl having 1 to 6 carbon atoms in the alkoxy moiety, or dialkylaminocarbonyl which has 1 to 4 carbon atoms in each alkyl moiety, each of the individual alkyl moieties being straight-chain or branched and substituted by identical or different substituents, or represents phenyl, benzyl, phenyloxy, phenylthio, benzyloxy or benzylthio, each of which is unsubstituted or monosubstituted to trisubstituted by identical or different substituents, or represent 5- or 6-membered heteroaryl which contains 1 to 3 hetero atoms from the series comprising nitrogen, oxygen or sulphur and which is unsubstituted or monosubstituted or disubstituted by identical or different substituents, suitable substituents in each case being halogen, alkyl, alkoxy, alkylthio, halogenoalkyl, halogenoalkyloxy, halogenoalkylthio, each of which has 1 to 4 carbon atoms and where appropriate 1 to 9 identical or different halogen atoms, dialkylamino or dialkylaminocarbonyl, each of which has 1 to 4 carbon atoms in each of the straight-chain or branched alkyl moieties, 1,3-propanediyl, 1,4-butanediyl, or phenyl or benzyl which are unsubstituted or monosubstituted to disubstituted by identical or different substituents, suitable substituents in each case being halogen or phenyloxy.

**3.** Acrylic esters of the formula (I) according to Claim 1, in which

$R^1$ represents straight-chain or branched alkyl having 1 to 4 carbon atoms,

$R^2$ represents dialkylamino having 1 to 4 carbon atoms in each of the individual straight-chain or branched alkyl moieties, in each case straight-chain or branched alkoxy or alkylthio having 1 to 4 carbon atoms, or represents benzyloxy or benzylthio,

$R^3$ and $R^4$ are identical or different and represent hydrogen, cyano, fluorine, chlorine, bromine,

methyl, ethyl, n- or i-propyl or n-, i-, s- or t-butyl,

$R^5$ and $R^6$ are identical or different and represent hydrogen, fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximininomethyl, ethoximinomethyl, methoximinoethyl, ethoximininoethyl, cyclopentyl or cyclohexyl, or together represent a methylenedioxy, 1,3-propanediyl or 1,4-butanediyl group,

$R^7$ represents one of the following groups

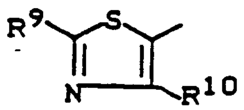 ; 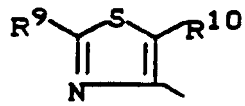 ; 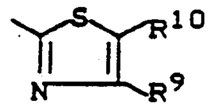

or

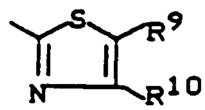

where

$R^9$ represents hydrogen, straight-chain or branched alkyl having 1 to 6 carbon atoms, or phenyl or benzyl, each of which is unsubstituted or mono-substituted to trisubstituted by identical or different substituents, or represents 5- or 6-membered heteroaryl which contains 1 to 3 hetero atoms from the series comprising nitrogen, oxygen or sulphur, and which is monosubstituted or disubstituted by identical or different substituents, suitable substituents in each case being halogen, methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, halogenoalkyl, halogenoalkoxy or halogenoalkylthio, each of which has 1 or 2 carbon atoms and 1 to 5 identical or different fluorine or chlorine atoms, or dialkylamino or dialkylaminocarbonyl having 1 or 2 carbon atoms in the individual alkyl moieties, or phenyl, phenoxy or benzyl which are unsubstituted or monosubstituted to disubstituted by identical or different substituents, suitable substituents in each case being fluorine, chlorine or phenoxy, and

$R^{10}$ represents hydrogen, straight-chain or branched alkyl having 1 to 4 carbon atoms, chlorine, bromine, cyano, methoxycarbonyl, ethoxycarbonyl, dimethylaminocarbonyl, diethylamino

$R^8$ represents hydrogen, fluorine, chlorine, bromine, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl or ethoxycarbonyl.

**4.** Acrylic esters of the formula (I) according to Claim 1, in which

$R^1$ represents methyl or ethyl,

$R^2$ represents dimethylamino, diethylamino, methoxy, ethoxy, methylthio, ethylthio, benzyloxy or benzylthio,

$R^3$ represents hydrogen, chlorine or methyl,

$R^4$ represents hydrogen, chlorine or methyl,

$R^5$ represents hydrogen, fluorine, chlorine, bromine, methyl, ethyl, trifluoromethyl, methoxycarbonyl or ethoxycarbonyl,

$R^6$ represents hydrogen, fluorine, chlorine, bromine, methyl, ethyl, methoxy, ethoxy, methylthio, trifluoromethyl, methoxycarbonyl or ethoxycarbonyl, or

$R^5$ and $R^6$ together represent a methylenedioxy, 1,3 -propanediyl or 1,4-butanediyl group,

$R^7$ represents one of the following groups

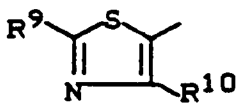 ; 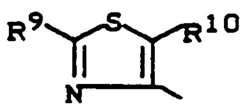 ; 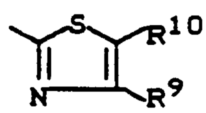

or 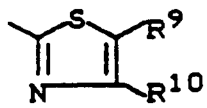

where

$R^9$ represents hydrogen, methyl, ethyl, n- or i-propyl, n-, s-, i-butyl, n- or i-pentyl, n-hexyl, benzyl, o-, m- or p-chlorobenzyl, o-, m- or p-methylbenzyl, phenyl or phenyl which is in each case monosubstituted to trisubstituted by identical or different substituents, or represents 5- or 6-membered heteroaryl which contains 1 or 2 hetero atoms from the series comprising nitrogen, oxygen or sulphur and which is monosubstituted or disubstituted by identical or different substituents, suitable substituents in each case being fluorine, chlorine, methyl, ethyl, t-butyl, methoxy, ethoxy, methylthio, trifluoromethyl, trifluoromethoxy, trifluoromethylthio, phenyl, p-chlorophenyl, m- or p-phenoxyphenyl or benzyl, and

$R^{10}$ represents hydrogen, methyl, ethyl, chlorine, bromine, methoxycarbonyl or ethoxycarbonyl, and

$R^8$ represents hydrogen, methyl or ethyl.

**5.** Process for the preparation of acrylic esters of the general formula (I)

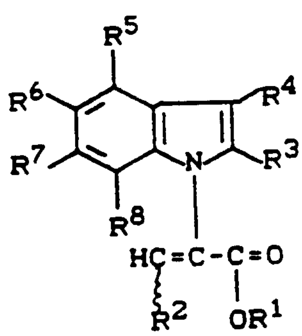 (I)

in which

$R^1$ represents alkyl, or represents unsubstituted or substituted aralkyl,

$R^2$ represents dialkylamino, alkoxy or alkylthio, or represents in each case unsubstituted or substituted aralkyloxy or arylalkylthio,

$R^3$ and $R^4$ in each case independently of one another represent hydrogen, cyano, halogen or alkyl,

$R^5$, $R^6$ and $R^8$ independently of one another in each case represent hydrogen, halogen, cyano, nitro, in each case straight-chain or branched alkyl, alkoxy or alkylthio, each of which has 1 to 4 carbon atoms, or represent alkylidenedioxy having 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, cycloalkyl having 3 to 7 carbon atoms, divalent alkanediyl having 3 to 5 carbon atoms, or aryl, aralkyl, aryloxy, arylthio, aralkyloxy or aralkylthio, each of which has 6 to 10 carbon atoms in the aryl moiety and where appropriate 1 to 4 carbon atoms in the straight-chain or branched alkyl moiety and each of which is unsubstituted or monosubstituted or polysubstituted in the aryl moiety by identical

or different substitutents from the series comprising halogen, alkyl, alkoxy, alkylthio, halogenoalkyl, halogenoalkoxy or halogenoalkylthio, each of which has 1 to 4 carbon atoms and where appropriate 1 to 9 identical or different halogen atoms, or represent heteroarylalkyl, heteroaryloxy, heteroarylthio or heteroaryl, each of which has 2 to 9 carbon atoms and 1 to 4 identical or different hetero atoms - in particular nitrogen, oxygen and/or sulphur - in the heteroaryl moiety and where appropriate 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and where appropriate 1 to 9 identical or different halogen atoms, and

$R^7$ represents one of the following groups

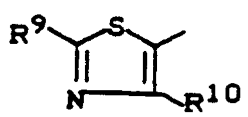 ; 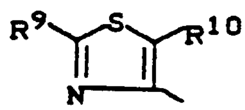 ; 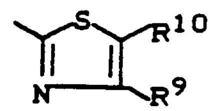

or

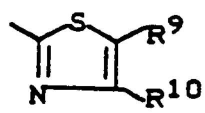

where

$R^9$ and $R^{10}$ in each case independently of one another represent hydrogen, halogen, cyano, nitro, alkyl, alkoxy, alkylthio, halogenoalkyl, alkoxycarbonyl or dialkylaminocarbonyl, or represent in each case unsubstituted or substituted aryl, aralkyl, aryloxy, arylthio, aralkyloxy, aralkylthio, hetaryl, hetaryloxy or hetarylthio,

characterized in that

a) substituted acrylic esters of the general formula (Ia)

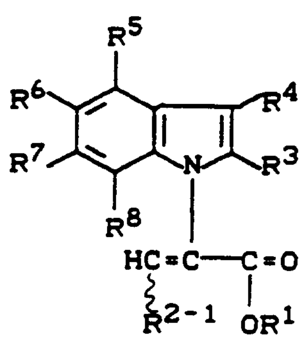

(Ia)

in which

$R^1$, $R^3$, $R^4$, $R^5$, $R^6$, $R^7$ and $R^8$ have the abovementioned meanings and

$R^{2-1}$ represents alkoxy or unsubstituted or substituted aralkyloxy,

are obtained when hydroxyacrylic esters or alkali metal salts thereof of the formula (II)

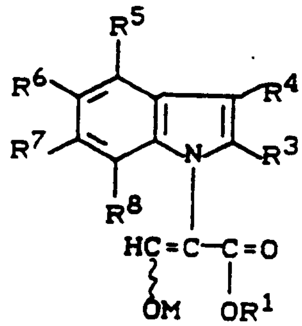

(II)

in which

| | |
|---|---|
| M | represents hydrogen or an alkali metal cation and |
| $R^1$, $R^3$, $R^4$, $R^5$, $R^6$, $R^7$ and $R^8$ | have the abovementioned meanings, |

are reacted with alkylating agents of the formula (III)

$R^{11}$-$E^1$ (III)

in which

| | |
|---|---|
| $R^{11}$ | represents alkyl or unsubstituted or substituted aralkyl and |
| $E^1$ | represents an electron-attracting leaving group, |

if appropriate in the presence of a diluent and if appropriate in the presence of a reaction auxiliary;

b) substituted acrylic esters of the general formula (Ib)

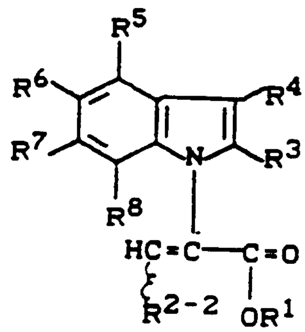

(Ib)

in which

| | |
|---|---|
| $R^1$, $R^3$, $R^4$, $R^5$, $R^6$, $R^7$ and $R^8$ | have the abovementioned meanings and |
| $R^{2-2}$ | represents dialkylamino, |

are obtained when substituted acetic esters of the formula (IV)

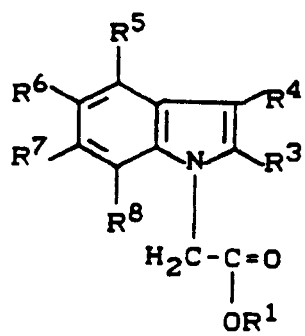

(IV)

in which

| | |
|---|---|
| $R^1$, $R^3$, $R^4$, $R^5$, $R^6$, $R^7$ and $R^8$ | have the abovementioned meanings, |

are reacted with formamides of the formula (Va)

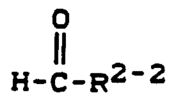

(Va)

in which

$R^{2-2}$ has the abovementioned meaning,

or with formamide derivatives of the formula (Vb)

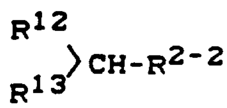

(Vb)

in which

$R^{12}$ and $R^{13}$ independently of one another represent alkoxy or dialkylamino and

$R^{2-2}$ has the abovementioned meaning,

if appropriate in the presence of a diluent;

c) substituted acrylic esters of the formula (Ic)

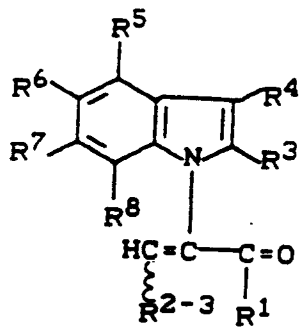

(Ic)

in which

$R^1$, $R^3$, $R^4$, $R^5$, $R^6$, $R^7$ and $R^8$ have the abovementioned meanings and

$R^{2-3}$ represents alkylthio or unsubstituted or substituted aralkylthio,

are obtained when ketocarboxylic acid derivatives of the formula (VI)

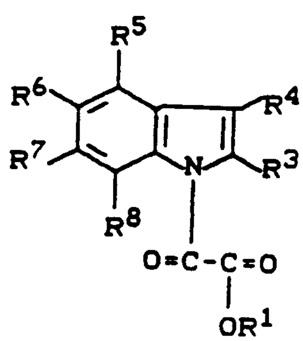

(VI)

in which

$R^1$, $R^3$, $R^4$, $R^5$, $R^6$, $R^7$ and $R^8$ have the abovementioned meanings,

are reacted with organometal compounds of the formula (VII)

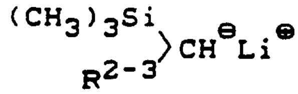

(VII)

in which

$R^{2-3}$ has the abovementioned meaning,

if appropriate in the presence of a diluent;

d) substituted acrylic esters of the formula (Ic) are obtained when substituted acrylic esters of the formula (VIII)

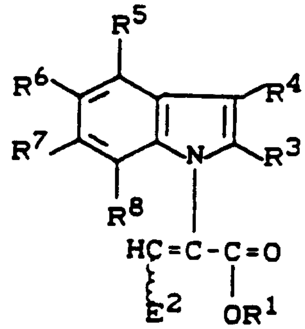

(VIII)

in which

$R^1$, $R^3$, $R^4$, $R^5$, $R^6$, $R^7$ and $R^8$ have the abovementioned meanings and

$E^2$ represents an electron-attracting leaving group,

are reacted with thiols of the formula (IX)

$R^{2-3}$-H (IX)

in which

$R^{2-3}$ has the abovementioned meaning,

if appropriate in the presence of a diluent and if appropriate in the presence of a reaction auxiliary.

**6.** Pesticides, characterized in that they contain at least one acrylic ester of the formula (I) according to Claims 1 to 5.

**7.** Use of acrylic esters of the formula (I) according to Claims 1 to 5 for combating pests.

**8.** Method of combating pests, characterized in that acrylic esters of the formula (I) according to Claims 1 to 5 are allowed to act on pests and/or their habitat.

**9.** Process for the preparation of pesticides, characterized in that acrylic esters of the formula (I) according to Claims 1 to 5 are mixed with extenders and/or surface-active agents.

**10.** Hydroxyacrylic esters or their alkali metal salts of the formula (II)

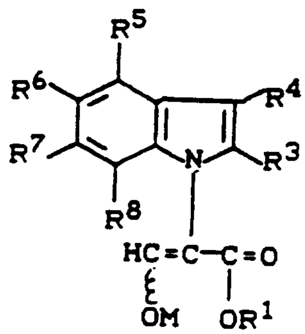

(II)

in which

| | |
|---|---|
| M | represents hydrogen, or an alkali metal cation, and |
| $R^1$, $R^3$, $R^4$, $R^5$, $R^6$, $R^7$ and $R^8$ | have the meanings given in Claim 1. |

**11.** Process for the preparation of hydroxyacrylic esters of the formula (II)

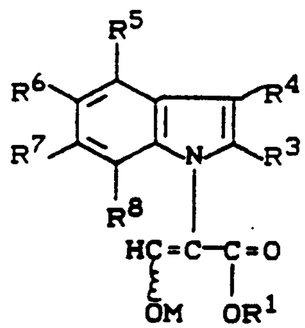

(II)

in which
M, $R^1$ and $R^3$ to $R^8$ have the meanings given in Claim 10, characterized in that substituted acetic esters of the formula (IV)

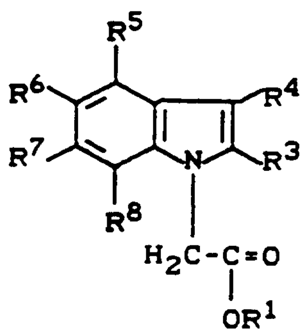

(IV)

in which
$R^1$, $R^3$, $R^4$, $R^5$, $R^6$, $R^7$ and $R^8$ have the meanings given in Claim 10
are reacted with formic esters of the formula (X)

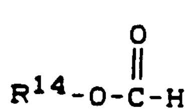

(X)

in which

$R^{14}$ represents alkyl,

if appropriate in the presence of a diluent and if appropriate in the presence of a basic reaction auxiliary, at temperatures of from -20 °C to +50 °C.

**12.** Acetic esters of the formula (IV)

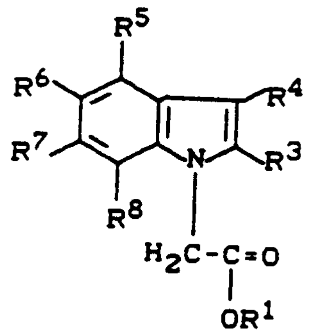

(IV)

in which

$R^1$, $R^3$, $R^4$, $R^5$, $R^6$, $R^7$ and $R^8$ have the meanings given in Claim 1.

**13.** Process for the preparation of acetic esters of the formula (IV)

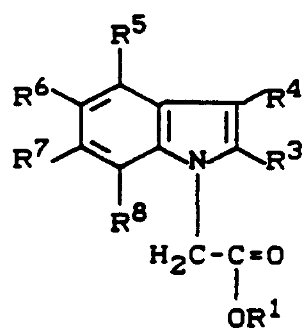

(IV)

in which $R^1$ to $R^8$ have the meanings given in Claim 1, characterized in that indole derivatives of the general formula (XI)

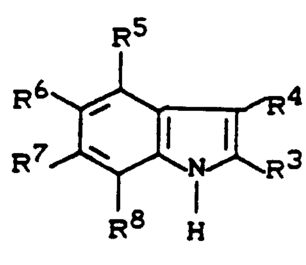

(XI)

in which

$R^3$, $R^4$, $R^5$, $R^6$, $R^7$ and $R^8$ have the meanings given in Claim 1

are reacted with acetic acid derivatives of the general formula (XII)

$E^3\text{-}CH_2\text{-}COOR^1$ (XII)

in which

$R^1$ has the abovementioned meaning and

$E^3$ represents an electron-attracting leaving group, preferably halogen, in particular chlorine or bromine,

if appropriate in the presence of a diluent, and if appropriate in the presence of a basic auxiliary, at temperatures between -20 °C and +100 °C.

**14.** Indole derivatives of the formula (XI)

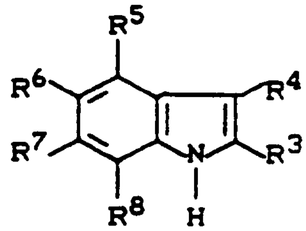

(XI),

in which

$R^3$ to $R^8$ have the meanings given in Claim 1.

**15.** Process for the preparation of indole derivatives of the formula (XI)

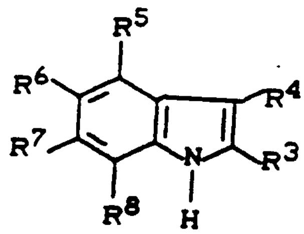

(XI),

in which

$R^3$ to $R^8$ have the meanings given in Claim 1, characterized in that nitrobenzene derivatives of the formula (XIII)

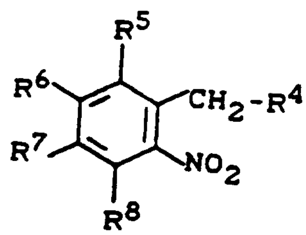

(XIII)

in which

$R^4$, $R^5$, $R^6$, $R^7$ and $R^8$ have the meanings given in Claim 1

are reacted with compounds of the formula (XIV)

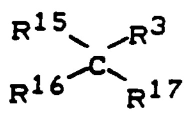

(XIV),

in which

$R^3$ has the meaning given in Claim 1,

$R^{15}$ represents alkoxy or dialkylamino,

$R^{16}$ represents alkoxy or dialkylamino and

$R^{17}$ represents dialkylamino,

if appropriate in the presence of a diluent, at temperatures between 25 °C and 200 °C and if appropriate under a pressure of from 1 to 100 bar, to give the compounds of the general formula (XV)

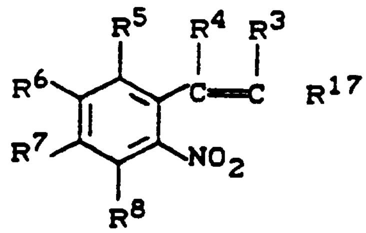

(XV),

in which

$R^3$, $R^4$, $R^5$, $R^6$, $R^7$, $R^8$ and $R^{17}$ have the meanings given in Claim 1,

and the resulting compounds of the formula (XV), if appropriate after they have been isolated and/or purified, if appropriate in the presence of a diluent, if appropriate in the presence of an inert gas, are cyclized using customary reducing agents at temperatures between -20 °C and +200 °C in the presence of a suitable catalyst under a pressure between 1 and 200 bar.

**16.** Ketocarboxylic acid derivatives of the formula (VI)

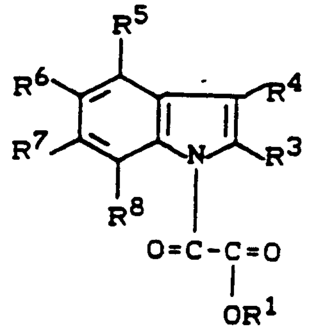

(VI),

in which

$R^1$, $R^3$, $R^4$, $R^5$, $R^6$, $R^7$ and $R^8$ have the meanings given in Claim 1.

**17.** Process for the preparation of ketocarboxylic acid derivatives of the formula (VI)

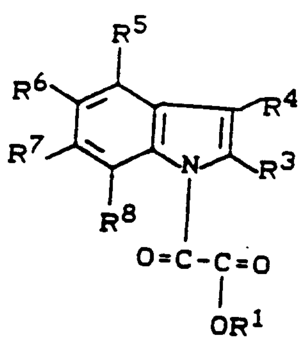

(VI),

in which

$R^1$ to $R^8$ have the meanings given in Claim 1,

characterized in that oxalic esters of the formula (XXVI)

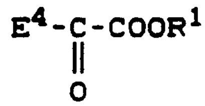

(XXVI),

in which

$R^1$ has the abovementioned meaning and

$E^4$ represents alkoxy or halogen, in particular methoxy, ethoxy or chlorine,

are reacted with indole derivatives of the formula (XI)

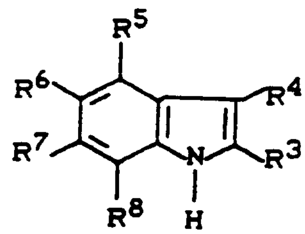

(XI),

in which

$R^3$, $R^4$, $R^5$, $R^6$, $R^7$ and $R^8$ have the abovementioned meanings,

if appropriate in the presence of a diluent and if appropriate in the presence of a base, at temperatures between -80 °C and +80 °C.

**18.** Pesticides, characterized in that they contain at least one acetic ester of the formula (IV) according to Claims 12 and 13.

**19.** Use of acetic esters of the formula (IV) according to Claims 12 and 13 for combating pests.

**20.** Method of combating pests, characterized in that acetic esters of the formula (IV) according to Claims 12 and 13 are allowed to act on pests and/or their habitat.

**21.** Process for the preparation of pesticides, characterized in that acetic esters of the formula (IV) according to Claims 12 and 13 are mixed with extenders and/or surface-active agents.

**22.** Use of the compounds of the formulae (II), (IV), (XI) and (VI) according to Claims 10 to 17 as intermediates for the preparation of compounds of the formula (I) according to Claims 1 to 5.

**Revendications**

**1.** Esters acryliques répondant à la formule générale (I)

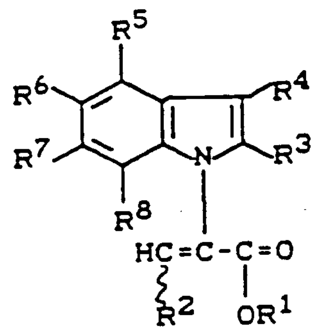

(I)

dans laquelle

$R^1$ représente un groupe alkyle; ou un groupe aralkyle non substitué ou substitué,

$R^2$ représente un groupe dialkylamino, un groupe alcoxy, un groupe alkylthio; ou encore un groupe aralkyloxy ou un groupe arylalkylthio, respectivement non substitué ou substitué,

$R^3$ et $R^4$ représentent, chacun indépendamment l'un de l'autre, un atome d'hydrogène, un groupe cyano, un atome d'halogène ou un groupe alkyle,

$R^5$, $R^6$ et $R^8$ représentent, indépendamment l'un de l'autre, respectivement un atome d'hydrogène, un atome d'halogène, un groupe cyano, un groupe nitro; un groupe alkyle, un groupe alcoxy ou un groupe alkylthio, respectivement à chaîne droite ou ramifiée, contenant respectivement de 1 à 4 atomes de carbone; un groupe alkylidènedioxy contenant de 1 à 6 atomes de carbone; un groupe halogénalkyle, un groupe halogénalcoxy ou un groupe halogénalkylthio, respectivement à chaîne droite ou ramifiée, contenant respectivement de 1 à 4 atomes de carbone et de 1 à 9 atomes d'halogène identiques ou différents; un groupe alcoxycarbonyle ou un groupe alcoximinoalkyle, respectivement à chaîne droite ou ramifiée et contenant respectivement de 1 à 4 atomes de carbone dans les fractions alkyle individuelles; un groupe cycloalkyle contenant de 3 à 7 atomes de carbone; un groupe alcanediyle deux fois lié contenant de 3 à 5 atomes de carbone; un groupe aryle, un groupe aralkyle, un groupe aryloxy, un groupe arylthio, un groupe aralkyloxy ou un groupe aralkylthio contenant respectivement de 6 à 10 atomes de carbone dans la fraction aryle et éventuellement de 1 à 4 atomes de carbone dans la fraction alkyle à chaîne droite ou ramifiée, respectivement non substitués ou substitués dans la fraction aryle, une ou plusieurs fois, de manière identique ou différente par un atome d'halogène, par un groupe alkyle, par un groupe alcoxy, par un groupe alkylthio, par un groupe halogénalkyle, par un groupe halogénalcoxy ou par un groupe halogénalkylthio contenant respectivement de 1 à 4 atomes de carbone et éventuellement de 1 à 9 atomes d'halogène identiques ou différents; ou encore un groupe hétéro-arylalkyle, un groupe hétéro-aryloxy, un groupe hétéro-arylthio ou un groupe hétéro-aryle contenant respectivement de 2 à 9 atomes de carbone et de 1 à 4 hétéro-atomes identiques ou différents - en particulier, un atome d'azote, un atome d'oxygène et un atome de soufre - dans la fraction hétéro-aryle et éventuellement de 1 à 4 atomes de carbone dans la fraction alkyle à chaîne droite ou ramifiée, respectivement éventuellement substitués dans la fraction hétéro-aryle, une ou plusieurs fois, de manière identique ou différente, par un atome d'halogène, par un groupe alkyle, par un groupe alcoxy, par un groupe alkylthio, par un groupe halogénalkyle; par un groupe halogénalcoxy ou par un groupe halogénalkylthio, contenant respectivement de 1 à 4 atomes de carbone et éventuellement de 1 à 9 atomes d'halogène identiques ou différents, et

$R^7$ représente un des groupements ci-après :

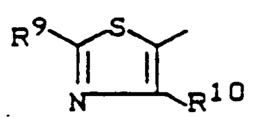 ; 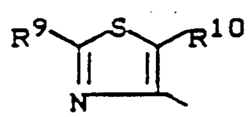 ; 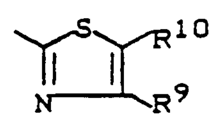

ou 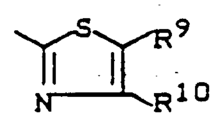

dans lesquels

$R^9$ et $R^{10}$ représentent, chacun indépendamment l'un de l'autre, un atome d'hydrogène, un atome d'halogène, un groupe cyano, un groupe nitro, un groupe alkyle, un groupe alcoxy, un groupe alkylthio, un groupe halogénalkyle, un groupe alcoxycarbonyle, un groupe dialkylaminocarbonyle; ou un groupe aryle, un groupe aralkyle, un groupe aryloxy, un groupe arylthio, un groupe aralkyloxy, un groupe aralkylthio, un

groupe hétaryle, un groupe hétaryloxy ou un groupe hétarylthio, respectivement non substitués ou substitués.

**2.** Esters acryliques de formule (I) selon la revendication 1, dans lesquels

$R^1$ représente un groupe alkyle à chaîne droite ou ramifiée contenant de 1 à 6 atomes de carbone ou encore un groupe benzyle,

$R^2$ représente un groupe dialkylamino contenant respectivement de 1 à 6 atomes de carbone dans les fractions alkyle individuelles à chaîne droite ou ramifiée; un groupe alcoxy ou un groupe alkylthio contenant de 1 à 6 atomes de carbone, respectivement à chaîne droite ou ramifiée; ou encore un groupe benzyloxy ou un groupe benzylthio respectivement non substitués ou substitués une ou plusieurs fois de manière identique ou différente, dans lesquels, comme substituants du groupe phényle, entrent en ligne de compte : un atome d'halogène; un groupe alkyle, un groupe alcoxy ou un groupe alkylthio respectivement à chaîne droite ou ramifiée contenant respectivement de 1 à 4 atomes de carbone; un groupe halogénalkyle, un groupe halogénalcoxy ou un groupe halogénalkylthio contenant respectivement de 1 à 4 atomes de carbone et de 1 à 9 atomes d'halogène identiques ou différents, respectivement à chaîne droite ou ramifiée; ou encore un groupe cycloalkyle contenant de 3 à 7 atomes de carbone,

$R^3$ et $R^4$ représentent, chacun indépendamment l'un de l'autre, un atome d'hydrogène, un groupe cyano, un atome de fluor, un atome de chlore, un atome de brome; ou encore un groupe alkyle contenant de 1 à 6 atomes de carbone, à chaîne droite ou ramifiée,

$R^5$, $R^6$ et $R^8$ représentent, indépendamment l'un de l'autre, un atome d'hydrogène, un atome de fluor, un atome de chlore, un atome de brome, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylènedioxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle, un groupe cyclohexyle; un groupe 1,3-propanediyle ou un groupe 1,4-butanediyle deux fois liés; ou encore un groupe phényle, un groupe benzyle, un groupe phénoxy, un groupe benzyloxy, un groupe phénylthio ou un groupe benzylthio respectivement éventuellement substitués dans la fraction phényle de 1 à 3 fois de manière identique ou différente par un atome de fluor, par un atome de chlore, par un atome de brome, par un groupe méthyle, par un groupe éthyle, par un groupe méthoxy, par un groupe éthoxy, par un groupe méthylthio, par un groupe trifluorométhyle, par un groupe difluorométhoxy, par un groupe trifluorométhoxy et/ou par un groupe trifluorométhylthio, et

$R^7$ représente un des groupements ci-après :

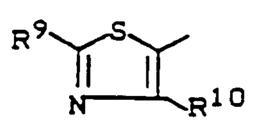 ; 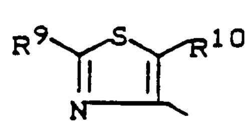 ; 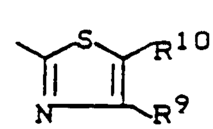

ou

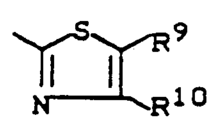

dans lesquels

$R^9$ et $R^{10}$ représentent, chacun indépendamment l'un de l'autre, un atome d'hydrogène, un atome de fluor, un atome de chlore, un atome de brome, un groupe cyano; un groupe alkyle, un groupe alcoxy, un groupe alkylthio ou un groupe halogénalkyle

contenant de 1 à 6 atomes de carbone et éventuellement de 1 à 13 atomes d'halogène identiques ou différents, respectivement à chaîne droite ou ramifiée; un groupe alcoxycarbonyle contenant de 1 à 6 atomes de carbone dans la fraction alcoxy, à chaîne droite ou ramifiée; un groupe dialkylaminocarbonyle contenant de 1 à 4 atomes de carbone dans chaque fraction alkyle, à chaîne droite ou ramifiée, respectivement substitué de manière identique ou différente dans les fractions alkyle individuelles; ou encore un groupe phényle, un groupe benzyle, un groupe phényloxy, un groupe phénylthio, un groupe benzyloxy ou un groupe benzylthio respectivement non substitués ou substitués de 1 à 3 fois de manière identique ou différente; ou encore un groupe hétéroaryle penta- ou hexagonal non substitué ou substitué 1 ou 2 fois de manière identique ou différente, qui contient de 1 à 3 hétéroatomes choisis parmi le groupe comprenant un atome d'azote, un atome d'oxygène ou un atome de soufre, dans lequel, comme substituants, on mentionnera respectivement : un atome d'halogène, un groupe alkyle, un groupe alcoxy, un groupe alkylthio, un groupe halogénalkyle, un groupe halogénalcoxy, un groupe halogénalkylthio contenant respectivement de 1 à 4 atomes de carbone et éventuellement de 1 à 9 atomes d'halogène identiques ou différents; un groupe dialkylamino ou un groupe dialkylaminocarbonyle contenant chacun de 1 à 4 atomes de carbone dans les fractions alkyle respectivement à chaîne droite ou ramifiée; un groupe 1,3-propanediyle; un groupe 1,4-butanediyle; ou encore un groupe phényle ou un groupe benzyle non substitués ou substitués 1 ou 2 fois de manière identique ou différente, dans lesquels, comme substituants, entrent en ligne de compte respectivement un atome d'halogène ou un groupe phényloxy.

**3.** Esters acryliques de formule (I) selon la revendication 1, dans lesquels

$R^1$ représente un groupe alkyle à chaîne droite ou ramifiée contenant de 1 à 4 atomes de carbone,

$R^2$ représente un groupe dialkylamino contenant respectivement de 1 à 4 atomes de carbone dans les fractions alkyle individuelles à chaîne droite ou ramifiée; un groupe alcoxy ou un groupe alkylthio contenant de 1 à 4 atomes de carbone, respectivement à chaîne droite ou ramifiée; ou encore un groupe benzyloxy ou un groupe benzylthio,

$R^3$ et $R^4$ sont identiques ou différents et représentent un atome d'hydrogène, un groupe cyano, un atome de fluor, un atome de chlore, un atome de brome, un groupe méthyle, un groupe éthyle, un groupe n- ou i-propyle, un groupe n-, i-, s- ou t-butyle,

$R^5$ et $R^6$ sont identiques ou différents et représentent un atome d'hydrogène, un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle, un groupe éthoxycarbonyle, un groupe méthoximinométhyle, un groupe éthoximinométhyle, un groupe méthoximinoéthyle, un groupe éthoximinoéthyle, un groupe cyclopentyle ou un groupe cyclohexyle; ou représentent ensemble un groupement méthylènedioxy, un groupement 1,3-propanediyle ou un groupement 1,4-butanediyle,

$R^7$ représente un des groupements ci-après :

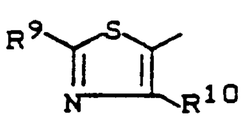 ; 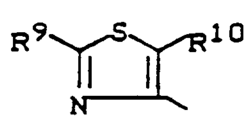 ; 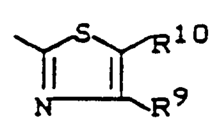

ou 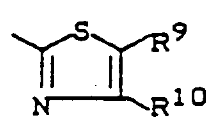

dans lesquels

$R^9$ représente un atome d'hydrogène; un groupe alkyle à chaîne droite ou ramifiée contenant de 1 à 6 atomes de carbone; un groupe phényle, un groupe benzyle respectivement non substitués ou substitués de 1 à 3 fois de manière identique ou différente; ou encore un groupe hétéroaryle pentaou hexagonal substitué 1 ou 2 fois de manière identique ou différente, qui contient de 1 à 3 hétéroatomes choisis parmi le groupe comprenant un atome d'azote, un atome d'oxygène ou un atome de soufre, dans lequel, comme substituants, on mentionnera respectivement : un atome d'halogène, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe n-, i-, s- ou t-butoxy, un groupe méthylthio, un groupe éthylthio, un groupe n- ou i-propylthio, un groupe n-, i-, s- ou t-butylthio, un groupe halogénalkyle, un groupe halogénalcoxy ou un groupe halogénalkylthio contenant respectivement 1 ou 2 atomes de carbone et de 1 à 5 atomes de fluor ou de chlore identiques ou différents; un groupe dialkylamino ou un groupe dialkylaminocarbonyle contenant 1 ou 2 atomes de carbone dans les fractions alkyle individuelles; un groupe phényle, un groupe phénoxy ou un groupe benzyle non substitués ou substitués 1 ou 2 fois de manière identique ou différente, dans lesquels, comme substituants, entrent respectivement en ligne de compte : un atome de fluor, un atome de chlore ou un groupe phénoxy, et

$R^{10}$ représente un atome d'hydrogène; un groupe alkyle à chaîne droite ou ramifiée contenant de 1 à 4 atomes de carbone; un atome de chlore, un atome de brome, un groupe cyano, un groupe méthoxycarbonyle, un groupe éthoxycarbonyle, un groupe diméthylaminocarbonyle, un groupe diéthylaminocarbonyle, un groupe méthoxy, un groupe éthoxy, un groupe méthylthio ou un groupe éthylthio, et

$R^8$ représente un atome d'hydrogène, un atome de fluor, un atome de chlore, un atome de brome, un groupe méthyle, un groupe éthyle, un groupe n- ou i-propyle, un groupe n-, i-, s- ou t-butyle, un groupe méthoxy, un groupe éthoxy, un groupe n- ou i-propoxy, un groupe méthylthio, un groupe trifluorométhyle, un groupe trifluorométhoxy, un groupe difluorométhoxy, un groupe trifluorométhylthio, un groupe méthoxycarbonyle ou un groupe éthoxycarbonyle.

**4.** Esters acryliques de formule (I) selon la revendication 1, dans lesquels

$R^1$ représente un groupe méthyle ou un groupe éthyle,

$R^2$ représente un groupe diméthylamino, un groupe diéthylamino, un groupe méthoxy, un groupe éthoxy, un groupe méthylthio, un groupe éthylthio, un groupe benzyloxy ou un groupe benzylthio,

$R^3$ représente un atome d'hydrogène, un atome de chlore ou un groupe méthyle,

$R^4$ représente un atome d'hydrogène, un atome de chlore ou un groupe méthyle,

$R^5$ représente un atome d'hydrogène, un atome de fluor, un atome de chlore, un atome de brome, un groupe méthyle, un groupe éthyle, un groupe trifluorométhyle, un groupe méthoxycarbonyle ou un groupe éthoxycarbonyle,

$R^6$ représente un atome d'hydrogène, un atome de fluor, un atome de chlore, un atome de brome, un groupe méthyle, un groupe éthyle, un groupe méthoxy, un groupe éthoxy, un groupe méthylthio, un groupe trifluorométhyle, un groupe méthoxycarbonyle ou un groupe éthoxycarbonyle, ou bien

$R^5$ et $R^6$ représentent, ensemble, un groupement méthylènedioxy, un groupement 1,3-propanediyle ou un groupement 1,4-butanediyle,

$R^7$ représente un des groupements ci-après :

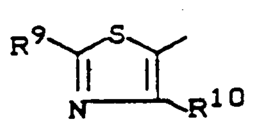 ; 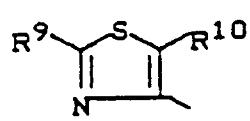 ; 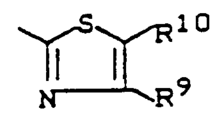

ou 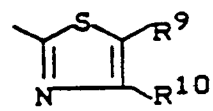

dans lesquels

| | |
|---|---|
| $R^9$ | représente un atome d'hydrogène, un groupe méthyle, un groupe éthyle, un groupe n- ou i-propyle, un groupe n-, s-, i-butyle, un groupe n- ou i-pentyle, un groupe n-hexyle, un groupe benzyle, un groupe o-, m- ou p-chlorobenzyle, un groupe o-, m- ou p-méthylbenzyle, un groupe phényle; ou encore un groupe phényle respectivement substitué de 1 à 3 fois de manière identique ou différente; ou encore un groupe hétéroaryle penta- ou hexagonal substitué 1 ou 2 fois de manière identique ou différente, qui contient 1 ou 2 hétéroatomes choisis parmi le groupe comprenant un atome d'azote, un atome d'oxygène ou un atome de soufre, dans lequel, comme substituants, entrent respectivement en ligne de compte : un atome de fluor, un atome de chlore, un groupe méthyle, un groupe éthyle, un groupe t-butyle, un groupe méthoxy, un groupe éthoxy, un groupe méthylthio, un groupe trifluorométhyle, un groupe trifluorométhoxy, un groupe trifluorométhylthio, un groupe phényle, un groupe p-chlorophényle, un groupe m- ou p-phénoxyphényle ou un groupe benzyle, et |
| $R^{10}$ | représente un atome d'hydrogène, un groupe méthyle, un groupe éthyle, un atome de chlore, un atome de brome, un groupe méthoxycarbonyle ou un groupe éthoxycarbonyle, ou |
| $R^8$ | représente un atome d'hydrogène, un groupe méthyle ou un groupe éthyle. |

**5.** Procédé pour la préparation d'esters acryliques répondant à la formule générale (I)

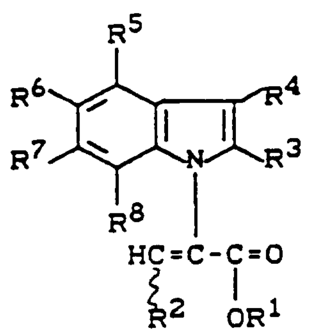 (I)

dans laquelle

| | |
|---|---|
| $R^1$ | représente un groupe alkyle; ou un groupe aralkyle non substitué ou substitué, |
| $R^2$ | représente un groupe dialkylamino, un groupe alcoxy, un groupe alkylthio; ou encore un groupe aralkyloxy ou un groupe arylalkylthio, respectivement non substitué ou substitué, |
| $R^3$ et $R^4$ | représentent, chacun indépendamment l'un de l'autre, un atome d'hydrogène, un groupe cyano, un atome d'halogène ou un groupe alkyle, |
| $R^5$, $R^6$ et $R^8$ | représentent, indépendamment l'un de l'autre, respectivement un atome d'hydrogène, un atome d'halogène, un groupe cyano, un groupe nitro; un groupe alkyle, un groupe alcoxy ou un groupe alkylthio, respectivement à chaîne droite ou ramifiée, contenant respectivement de 1 à 4 atomes de carbone; un groupe alkylidènedioxy contenant de 1 à 6 atomes de carbone; un groupe halogénalkyle, un groupe halogénalcoxy ou un groupe halogénalkylthio, respectivement à chaîne droite ou |

ramifiée, contenant respectivement de 1 à 4 atomes de carbone et de 1 à 9 atomes d'halogène identiques ou différents; un groupe alcoxycarbonyle ou un groupe alcoximinoalkyle, respectivement à chaîne droite ou ramifiée et contenant respectivement de 1 à 4 atomes de carbone dans les fractions alkyle individuelles; un groupe cycloalkyle contenant de 3 à 7 atomes de carbone; un groupe alcanediyle deux fois lié contenant de 3 à 5 atomes de carbone; un groupe aryle, un groupe aralkyle, un groupe aryloxy, un groupe arylthio, un groupe aralkyloxy ou un groupe aralkylthio contenant respectivement de 6 à 10 atomes de carbone dans la fraction aryle et éventuellement de 1 à 4 atomes de carbone dans la fraction alkyle à chaîne droite ou ramifiée, respectivement non substitués ou substitués dans la fraction aryle, une ou plusieurs fois, de manière identique ou différente par un atome d'halogène, par un groupe alkyle, par un groupe alcoxy, par un groupe alkylthio, par un groupe halogénalkyle, par un groupe halogénalcoxy ou par un groupe halogénalkylthio contenant respectivement de 1 à 4 atomes de carbone et éventuellement de 1 à 9 atomes d'halogène identiques ou différents; ou encore un groupe hétéro-arylalkyle, un groupe hétéro-aryloxy, un groupe hétéro-arylthio ou un groupe hétéro-aryle contenant respectivement de 2 à 9 atomes de carbone et de 1 à 4 hétéro-atomes identiques ou différents - en particulier, un atome d'azote, un atome d'oxygène et un atome de soufre - dans la fraction hétéro-aryle et éventuellement de 1 à 4 atomes de carbone dans la fraction alkyle à chaîne droite ou ramifiée, respectivement éventuellement substitués dans la fraction hétéro-aryle, une ou plusieurs fois, de manière identique ou différente, par un atome d'halogène, par un groupe alkyle, par un groupe alcoxy, par un groupe alkylthio, par un groupe halogénalkyle, par un groupe halogénalcoxy ou par un groupe halogénalkylthio, contenant respectivement de 1 à 4 atomes de carbone et éventuellement de 1 à 9 atomes d'halogène identiques ou différents, et

$R^7$ représente un des groupements ci-après :

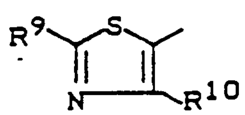 ; 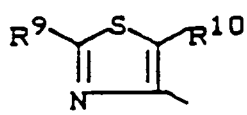 ; 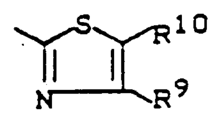

ou

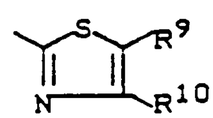

dans lesquels

$R^9$ et $R^{10}$ représentent, chacun indépendamment l'un de l'autre, un atome d'hydrogène, un atome d'halogène, un groupe cyano, un groupe nitro, un groupe alkyle, un groupe alcoxy, un groupe alkylthio, un groupe halogénalkyle, un groupe alcoxycarbonyle, un groupe dialkylaminocarbonyle; ou un groupe aryle, un groupe aralkyle, un groupe aryloxy, un groupe arylthio, un groupe aralkyloxy, un groupe aralkylthio, un groupe hétaryle, un groupe hétaryloxy ou un groupe hétarylthio, respectivement non substitués ou substitués,

caractérisé en ce qu'on obtient

a) des esters acryliques substitués répondant à la formule générale (Ia)

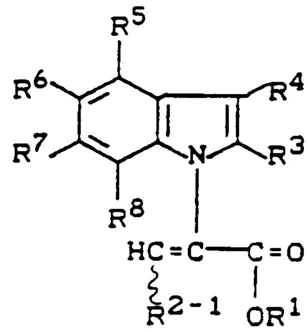

(Ia)

dans laquelle

$R^1$, $R^3$, $R^4$, $R^5$, $R^6$, $R^7$ et $R^8$ ont la signification indiquée ci-dessus, et

$R^{2-1}$ représente un groupe alcoxy; ou encore un groupe aralkyloxy non substitué ou substitué,

lorsqu'on fait réagir des esters hydroxyacryliques ou leurs sels de métaux alcalins de formule (II)

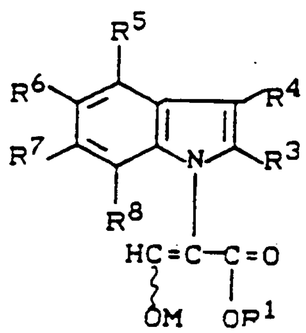

(II)

dans laquelle

M représente un atome d'hydrogène ou un cation de métal alcalin, et

$R^1$, $R^3$, $R^4$, $R^5$, $R^6$, $R^7$ et $R^8$ ont la signification indiquée ci-dessus,

avec des agents d'alkylation de formule (III)

$R^{11}$-$E^1$ (III)

dans laquelle

$R^{11}$ représente un groupe alkyle; ou un groupe aralkyle non substitué ou substitué, et

$E^1$ représente un groupe qui se sépare, attirant les électrons,

éventuellement en présence d'un diluant et éventuellement en présence d'un adjuvant réactionnel;

b) on obtient des esters acryliques substitués répondant à la formule générale (Ib)

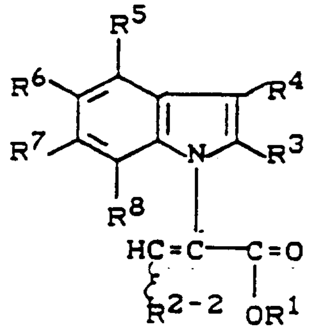

(Ib)

dans laquelle

$R^1$, $R^3$, $R^4$, $R^5$, $R^6$, $R^7$ et $R^8$ ont la signification indiquée ci-dessus, et

$R^{2-2}$ représente un groupe dialkylamino,

lorsqu'on fait réagir des esters acétiques substitués de formule (IV)

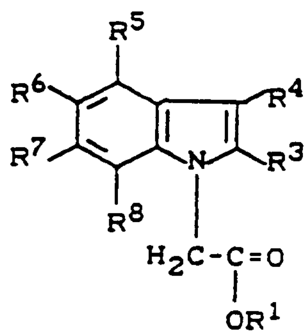

(IV)

dans laquelle

$R^1$, $R^3$, $R^4$, $R^5$, $R^6$, $R^7$ et $R^8$ ont la signification indiquée ci-dessus,

avec des formamides de formule (Va)

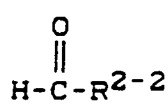

(Va)

dans laquelle

$R^{2-2}$ a la signification indiquée ci-dessus,

ou bien avec des dérivés de formamide de formule (Vb)

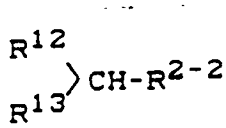

(Vb)

dans laquelle

$R^{12}$ et $R^{13}$ représentent, indépendamment l'un de l'autre, un groupe alcoxy ou un groupe dialkylamino, et

$R^{2-2}$ a la signification indiquée ci-dessus,

éventuellement en présence d'un diluant;

c) on obtient des esters acryliques substitués de formule (Ic)

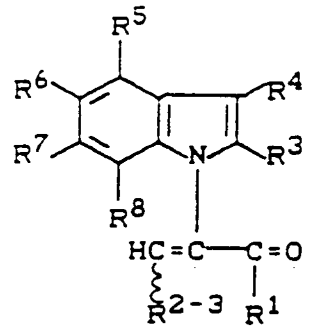

(Ic)

dans laquelle

$R^1$, $R^3$, $R^4$, $R^5$, $R^6$, $R^7$ et $R^8$ ont la signification indiquée ci-dessus, et

$R^{2-3}$ représente un groupe alkylthio; ou un groupe aralkylthio non substitué ou substitué,

lorsqu'on fait réagir des dérivés d'acides cétocarboxyliques de formule (VI)

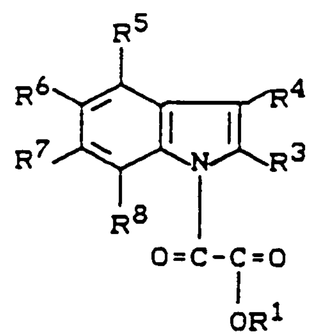

(VI)

dans laquelle

$R^1$, $R^3$, $R^4$, $R^5$, $R^6$, $R^7$ et $R^8$ ont la signification indiquée ci-dessus,

avec des composés organométalliques de formule (VII)

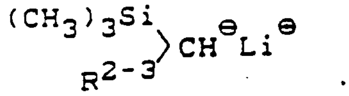

(VII)

dans laquelle

$R^{2-3}$ a la signification indiquée ci-dessus,

éventuellement en présence d'un diluant;

d) on obtient des esters acryliques substitués de formule (Ic)

lorsqu'on fait réagir des esters acryliques substitués de formule (VIII)

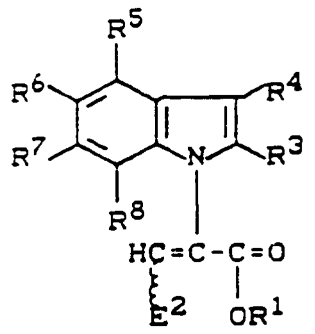

(VIII)

dans laquelle

$R^1$, $R^3$, $R^4$, $R^5$, $R^6$, $R^7$ et $R^8$ ont la signification indiquée ci-dessus, et

$E^2$ représente un groupe qui se sépare, attirant les électrons,

avec des thiols de formule (IX)

$R^{2-3}$-H (IX)

dans laquelle

$R^{2-3}$ a la signification indiquée ci-dessus,

éventuellement en présence d'un diluant et éventuellement en présence d'un adjuvant réactionnel.

**6.** Agent de lutte contre les parasites, caractérisé par une teneur en au moins un ester acrylique de formule (I) selon les revendications 1 à 5.

**7.** Utilisation d'esters acryliques de formule (I) selon les revendications 1 à 5, pour lutter contre les parasites.

**8.** Procédé pour lutter contre les parasites, caractérisé en ce qu'on laisse agir des esters acryliques de formule (I) selon les revendications 1 à 5, sur des parasites et/ou sur leur biotope.

**9.** Procédé pour la préparation d'agents de lutte contre les parasites, caractérisé en ce qu'on mélange des esters acryliques de formule (I) selon les revendications 1 à 5, avec des diluants et/ou avec des agents tensioactifs.

**10.** Esters hydroxyacryliques ou leurs sels de métaux alcalins de formule (II)

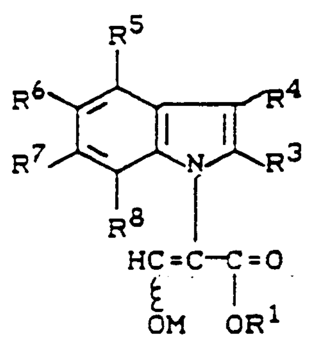

(II)

dans laquelle

M représente un atome d'hydrogène ou un cation de métal alcalin, et

$R^1$, $R^3$, $R^4$, $R^5$, $R^6$, $R^7$ et $R^8$ ont la signification indiquée à la revendication 1.

**11.** Procédé pour la préparation d'esters hydroxyacryliques de formule (II)

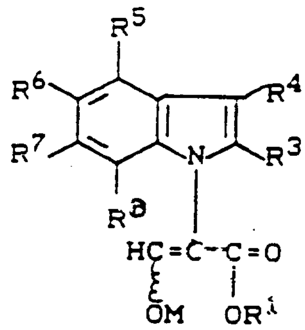

(II)

dans laquelle

M, $R^1$ et $R^3$ à $R^8$ ont la signification indiquée à la revendication 10,

caractérisé en ce qu'on fait réagir des esters acétiques substitués de formule (IV)

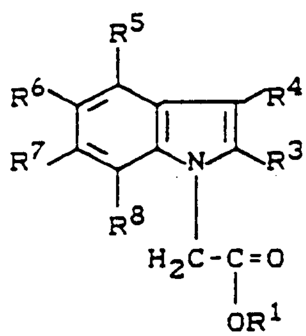

(IV)

dans laquelle

$R^1$, $R^3$, $R^4$, $R^5$, $R^6$, $R^7$ et $R^8$ ont la signification indiquée à la revendication 10,

avec des esters formiques de formule (X)

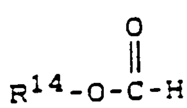

(X)

dans laquelle

$R^{14}$ représente un groupe alkyle

éventuellement en présence d'un diluant et éventuellement en présence d'un adjuvant réactionnel basique, à des températures de -20 °C à +50 °C.

**12.** Esters acétiques de formule (IV)

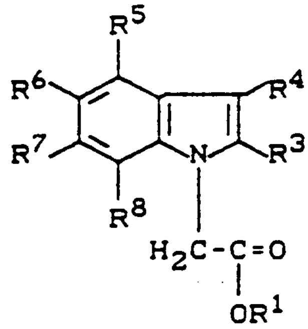

(IV)

dans laquelle

$R^1$, $R^3$, $R^4$, $R^5$, $R^6$, $R^7$ et $R^8$ ont la signification indiquée à la revendication 1.

**13.** Procédé pour la préparation d'esters acétiques de formule (IV)

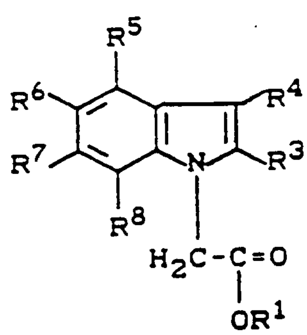

(IV)

dans laquelle $R^1$ à $R^8$ ont la signification indiquée à la revendication 1, caractérisé en ce qu'on fait réagir des dérivés d'indole répondant à la formule générale (XI)

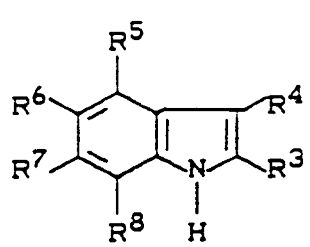

(XI),

dans laquelle

$R^3$, $R^4$, $R^5$, $R^6$, $R^7$ et $R^8$ ont la signification indiquée à la revendication 1,

avec des dérivés d'acide acétique répondant à la formule générale (XII)

$E^3$-$CH_2$-$COOR^1$ (XII)

dans laquelle

- $R^1$ a la signification indiquée ci-dessus et
- $E^3$ représente un groupe qui se sépare, attirant les électrons, de préférence un atome d'halogène, en particulier un atome de chlore ou un atome de brome,

éventuellement en présence d'un diluant et éventuellement en présence d'un adjuvant réactionnel basique, à des températures entre -20 °C et +100 °C.

**14.** Dérivés d'indole de formule (XI)

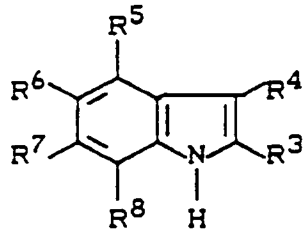

(XI)

dans laquelle

$R^3$ à $R^8$ ont la signification indiquée à la revendication 1.

**15.** Procédé pour la préparation de dérivés d'indole de formule (XI)

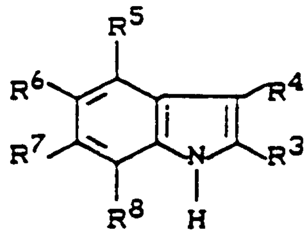

(XI),

dans laquelle

$R^3$ à $R^8$ ont la signification indiquée à la revendication 1,

caractérisé en ce qu'on fait réagir des dérivés de nitrobenzène de formule (XIII)

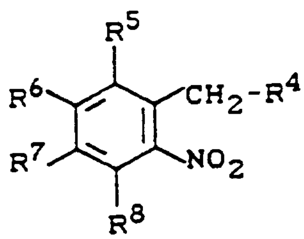

(XIII),

dans laquelle

$R^4$, $R^5$, $R^6$, $R^7$ et $R^8$ ont la signification indiquée à la revendication 1,

avec des composés de formule (XIV)

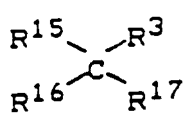

(XIV),

dans laquelle

$R^3$ a la signification indiquée dans la revendication 1,

$R^{15}$ représente un groupe alcoxy ou un groupe dialkylamino,

$R^{16}$ représente un groupe alcoxy ou un groupe dialkylamino,

$R^{17}$ représente un groupe dialkylamino,

éventuellement en présence d'un diluant, à des températures entre 25 ° C et 200 ° C et éventuellement

sous une pression de 1 à 100 bar pour obtenir les composés répondant à la formule générale (XV)

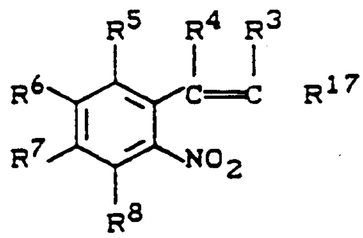

(XV),

dans laquelle

$R^3$, $R^4$, $R^5$, $R^6$, $R^7$, $R^8$ et $R^{17}$ ont la signification indiquée à la revendication 1,

et on cyclise les composés de formule (XV) ainsi obtenus, éventuellement après leur isolation et/ou leur purification, éventuellement en présence d'un diluant, éventuellement en présence d'un gaz inerte, avec des agents de réduction habituels, en présence d'un catalyseur approprié et sous une pression entre 1 et 200 bar, à des températures entre -20 °C et +200 °C.

**16.** Dérivés d'acides cétocarboxyliques de formule (VI)

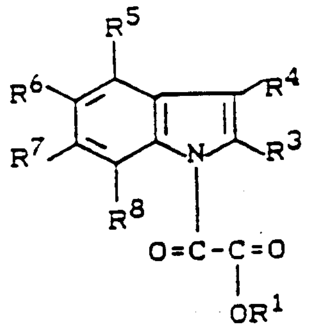

(VI),

dans laquelle

$R^1$, $R^3$, $R^4$, $R^5$, $R^6$, $R^7$ et $R^8$ ont la signification indiquée à la revendication 1.

**17.** Procédé pour la préparation de dérivés d'acides cétocarboxyliques de formule (VI)

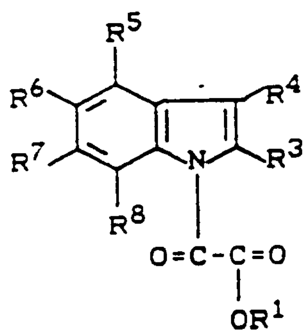

(VI),

dans laquelle

$R^1$ à $R^8$ ont la signification indiquée à la revendication 1,

caractérisé en ce qu'on fait réagir des esters oxaliques de formule (XXVI)

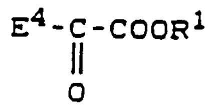

(XXVI),

dans laquelle

$R^1$ a la signification indiquée ci-dessus et

$E^4$ représente un groupe alcoxy ou un atome d'halogène, en particulier un groupe méthoxy, un groupe éthoxy ou un atome de chlore,

avec des dérivés d'indole de formule (XI)

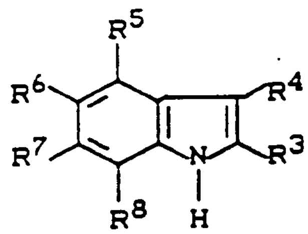

(XI),

dans laquelle

$R^3$, $R^4$, $R^5$, $R^6$, $R^7$ et $R^8$ ont la signification indiquée ci-dessus,

éventuellement en présence d'un diluant et éventuellement en présence d'une base, à des températures entre -80 °C et +80 °C.

**18.** Agent de lutte contre les parasites, caractérisé par une teneur en au moins un ester acétique de formule (IV) selon les revendications 12 et 13.

**19.** Utilisation d'esters acétiques de formule (IV) selon les revendications 12 et 13, pour lutter contre les parasites.

**20.** Procédé pour lutter contre les parasites, caractérisé en ce qu'on laisse agir des esters acétiques de formule (IV) selon les revendications 12 et 13, sur des parasites et/ou sur leur biotope.

**21.** Procédé pour la préparation d'agents de lutte contre les parasites, caractérisé en ce qu'on mélange des esters acétiques de formule (IV) selon les revendications 12 et 13, avec des diluants et/ou avec des agents tensioactifs.

**22.** Utilisation des composés des formules (II), (IV), (XI) et (VI) selon les revendications 10 à 17, comme produits intermédiaires pour la préparation des composés de formule (I) selon les revendications 1 à 5.